# EUROPEAN PATENT SPECIFICATION

(11) **EP 3 612 200 B1**
(45) Date of publication and mention of the grant of the patent: **11.11.2026**
(21) Application number: 18787737.8
(22) Date of filing: 20.04.2018
(51) Int. Cl.: A61K 35/76, A61K 35/741, A61K 47/64, C12N 7/00, A61P 17/10, A61K 31/327, A61K 31/60

(54) **COMPOSITIONS COMPRISING A PROPIONIBACTERIUM ACNES BACTERIOPHAGE AND SALICYLIC ACID FOR TREATING ACNE**
ZUSAMMENSETZUNGEN MIT EINEM PROPIONIBACTERIUM-ACNES-BAKTERIOPHAGE UND SALICYLSÄURE ZUR BEHANDLUNG VON AKNE
COMPOSITIONS COMPRENANT UN BACTÉRIOPHAGE DE PROPIONIBACTERIUM ACNES ET DE L'ACIDE SALICYLIQUE DESTINÉES AU TRAITEMENT DE L'ACNÉ

(30) Priority: 21.04.2017 US 201762488326 P
(43) Date of publication of application: 26.02.2020
(73) Proprietor: Phi Therapeutics, Inc., Alameda, CA 94502 (US)
(72) Inventor: VARMA, Yug, San Francisco CA 94117 (US); VAN PROOYEN, Nancy, San Francisco CA 94117 (US)
(74) Representative: HGF
(86) International application number: PCT/US2018/028556
(87) International publication number: WO 2018/195415

(56) References cited:
- EP-B1- 0 817 613
- WO-A1-2007/007055
- WO-A1-2013/142378
- WO-A1-2017/200873
- WO-A2-2015/118150
- WO-A2-2015/118150
- US-A1- 2016 338 979
- US-B2- 7 405 066
- US-B2- 9 125 919
- US-B2- 9 526 738
- EWA JONCZYK-MATYSIAK ET AL: "Prospects of Phage Application in the Treatment of Acne Caused by Propionibacterium acnes", FRONTIERS IN MICROBIOLOGY, vol. 8, 8 February 2017 (2017-02-08), XP055749906, DOI: 10.3389/fmicb.2017.00164

## Description

### CROSS-REFERENCE TO RELATED APPLICATIONS

This application claims the benefit of priority to U.S. Provisional Application No. 62/488,326, filed April 21, 2017.

### GOVERNMENT SUPPORT

This invention was made with government support under Grant No. 1R43AR068172 - 01 awarded by the National Institutes of Health. The government has certain rights in the invention.

### SEQUENCE LISTING

The content of the text file named "052004-503001WO_SequenceListing.TXT", was created on April 20, 2018, and is 101,782 bytes in size.

### BACKGROUND

Acne is a nearly universal condition that affects more than 80% of all people worldwide. This chronic skin condition is complex but the main etiological agent is *Propionibacterium acnes* whose overgrowth leads to inflammation that causes pimples. Despite a clear need for innovation, there has not been a novel acne drug in over 30 years. Current treatments including benzoyl peroxide and antibiotics are quite ineffective, and the most effective treatment - isotretinoin - is limited to a small set of patients due to dangerous side effects (including birth defects, liver damage, and suicide).

New methods and compositions for treating for acne are needed.

Ewa Jonczyk-Matysiak et al., FRONTIERS IN MICROBIOLOGY, vol. 8, 8 February 2017 is a review article discussing the prospects of Phage Application in the Treatment of Acne Caused by *Propionibacterium acnes.*

WO2017/200873A1 relates to *Propionibacterium acnes* bacteriophages with expanded host ranges for use in treating acne.

WO2013/142378A1 relates to methods of diagnosis and personalized treatment in patients afflicted with acne.

WO2015/118150A2 relates to the treatment of topical bacterial infections and systemic bacterial infections in mammals and humans.

US9526738B2 relates to topical gels that may controllably release nitric oxide and their use as an alternative to conventional antibiotics for controlling drug resistant bacteria.

US7405066 B2 relates to the use of a bacteriophage capable of lysing a *P*. *acnes* bacterium and incapable of lysing any bacterium which is not *P. acnes,* and which is incapable of sustaining lysogeny in a bacterium, for treating acne.

### BRIEF SUMMARY

The invention is defined by the claims. Any subject matter falling outside the scope of the claims is provided for information purposes only. Any references in the description to methods for treatment of the human or animal body by therapy refer to the compounds and pharmaceutical compositions of the present invention for use in the methods for treatment of the human or animal body by therapy.

Provided herein are, *inter alia,* compositions, combinations, systems, and methods for preventing or treating acne.

In an aspect of the invention, provided herein is a composition comprising, consisting essentially of, or consisting of at least one *Propionibacterium acnes* bacteriophage, at least one anti-acne compound comprising salicylic acid, and a pharmaceutically acceptable carrier, wherein the genome of the *P. acnes* bacteriophage comprises a nucleotide sequence that is at least about 98%, or 99% identical to the nucleotide sequence of SEQ ID NO: 1.

Also disclosed but not claimed herein is a composition that includes at least one *Propionibacterium acnes* bacteriophage, no more than one anti-acne compound, and a pharmaceutically acceptable carrier.

Also disclosed but not claimed herein is a composition that includes active ingredients consisting of at least one *Propionibacterium acnes* bacteriophage and no more than one anti-acne compound, and a pharmaceutically acceptable carrier.

Also disclosed but not claimed herein is a composition that includes at least one *Propionibacterium acnes* bacteriophage, at least one anti-acne compound, and a pharmaceutically acceptable carrier, wherein the composition does not comprise a probiotic bacterium.

Also disclosed but not claimed herein is a composition that includes a *Propionibacterium acnes* bacteriophage and an enzyme.

In a further aspect of the invention, provided herein is a combination comprising, consisting essentially of, or consisting of: (a)at least one *Propionibacterium acnes* bacteriophage; (b) at least one anti-acne compound comprising salicylic acid; (c) a pharmaceutically acceptable carrier, wherein (i) each of the at least one *Propionibacterium acnes* bacteriophage, the at least one anti-acne compound, and the pharmaceutically acceptable carrier are in a single composition in a container, and (ii) the genome of the *P. acnes* bacteriophage comprises a nucleotide sequence that is at least about 98% or 99% identical to the nucleotide sequence of SEQ ID NO: 1; and (d) a separate container comprising an additional anti-acne compound comprising benzoyl peroxide.

Also disclosed but not claimed herein is a combination that includes a *Propionibacterium acnes* bacteriophage and an enzyme.

In a further aspect of the invention, provided herein is a composition or combination according to the invention in a method of preventing or treating acne in a subject in need thereof.

### BRIEF DESCRIPTION OF THE DRAWINGS

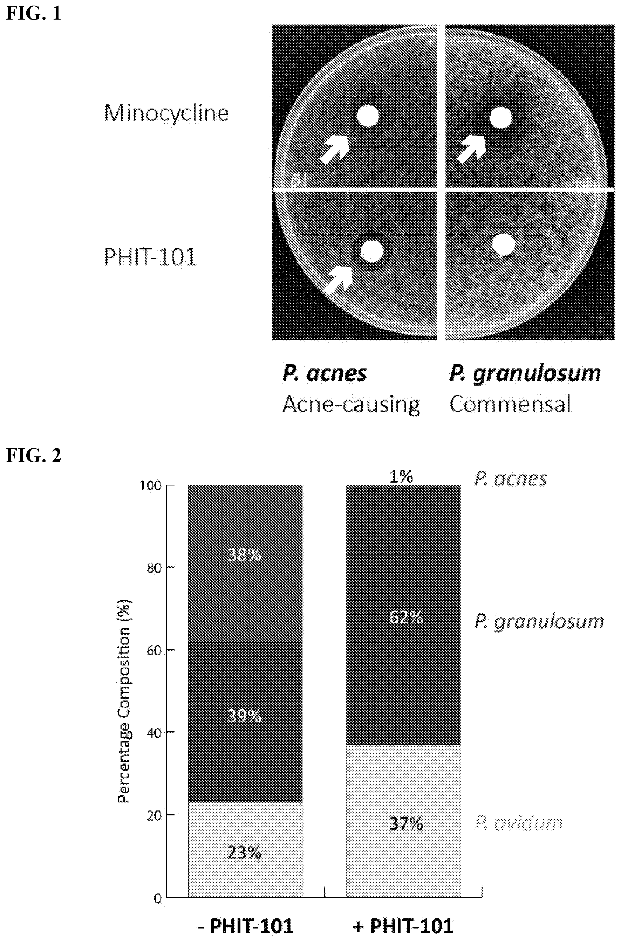

FIG. 1. *P. acnes* (acne-causing, left half plate) or *P. granulosum* (commensal, right half plate) bacteria was plated on RCM-agar petri dishes. Sterile half-pads soaked in either minocycline or PHIT-101 (10⁷ pfu/mL) were placed on each plate. After anaerobic incubation at 37°C for 3 days, zones of killing (arrows) appear, indicating that minocycline kills both pathogenic and commensal bacteria while PHIT-101 kills the acne-causing bacteria without disturbing commensal *P. granulosum.*
FIG. 2. A synthetic skin microbiome that includes *P. acnes, P. granulosum,* and *P*. *avidum* was grown to confluence in a test tube. It was then incubated in the presence or absence of PHIT-101 for 48 hours. The relative proportions of the three species were quantified by NGS sequencing of the 16S amplicon of the washed bacterial pellets using the Illumina MiSeq platform. PHIT-101 was able to almost completely wipe out acne-causing *P. acnes,* without affecting the growth of the other two commensal species.

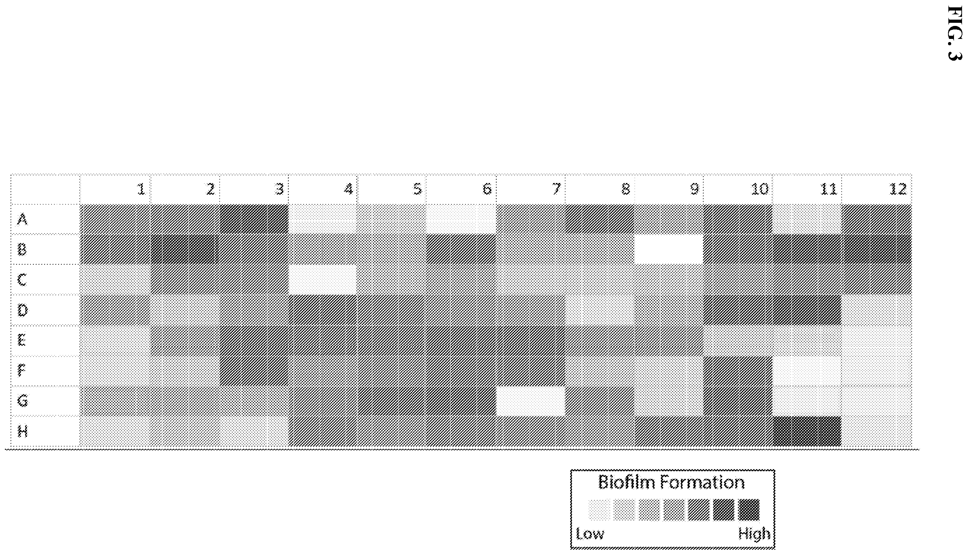

FIG. 3. Biofilm production amongst *P. acnes* strains is highly variable. 96 strains of *P. acnes* were grown in a 96-well polystyrene microtiter plate to stimulate biofilm production, and the biofilm produced by each strain was quantified. The variability demonstrated within this set of strains demonstrates the need to quantify biofilm formation under growth conditions more similar to those found in the human pore.

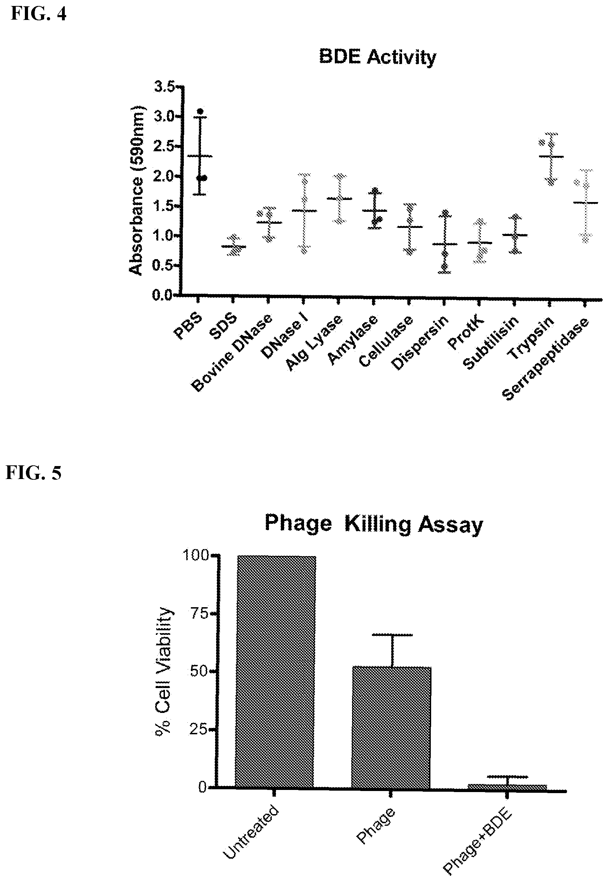

FIG. 4. A screen to select enzymes that can degrade *P. acnes* biofilms. *P. acnes* was grown in polystyrene microtiter plates to stimulate biofilm production. Enzymes were added at 0.01mg/mL to the wells and incubated at 30°C for 30 mins. The degraded biofilm was washed away with phosphate buffered saline (PBS), and the residual biofilm in each well was quantified by staining with crystal violet and recording absorbance at 590nm. Proteases like proteinase K and subtilisin showed good activity, and dispersin was the best glycoside depolymerase amongst those tested.
FIG. 5. Enhancement of phage with biofilm degrading enzyme (BDE) greatly increases bacterial killing. Sessile *P. acnes* cells were incubated with PBS (untreated), PHIT-101, or PHIT-101 and Dispersin. Cell survival was measured using the CellTiter-Blue reagent, and fluorescence was recorded at 560_{Ex}/590_{Em}. PHIT-101 was unable to kill *P. acnes* as effectively as in liquid culture, but addition of the biofilm degrading enzyme Dispersin greatly increased the bacterial killing to levels similar to liquid culture.

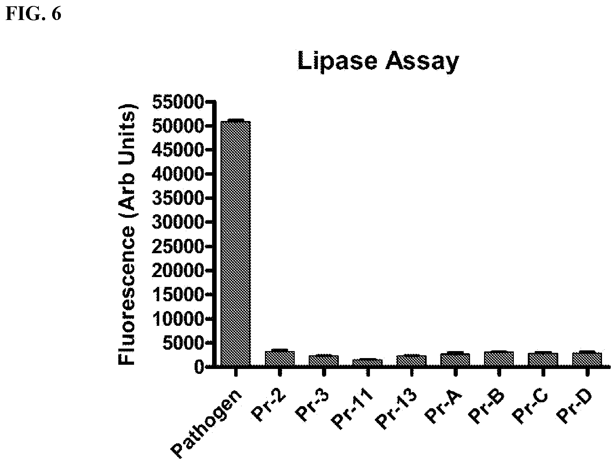

FIG. 6. Probiotic strains produce low levels of lipase in adherent culture. Probiotic *P*. *acnes* strains with known genotypes were grown under biofilm conditions in a microtiter plate. After 72 hrs of growth, the culture supernatant was filter-sterilized and incubated with 4-MU palmitate at 37C for 4 hours to determine extracellular lipase production. The lipase production of the probiotic strains (Pr#X) was very low in comparison to pathogen, indicating a lower inflammatory potential.

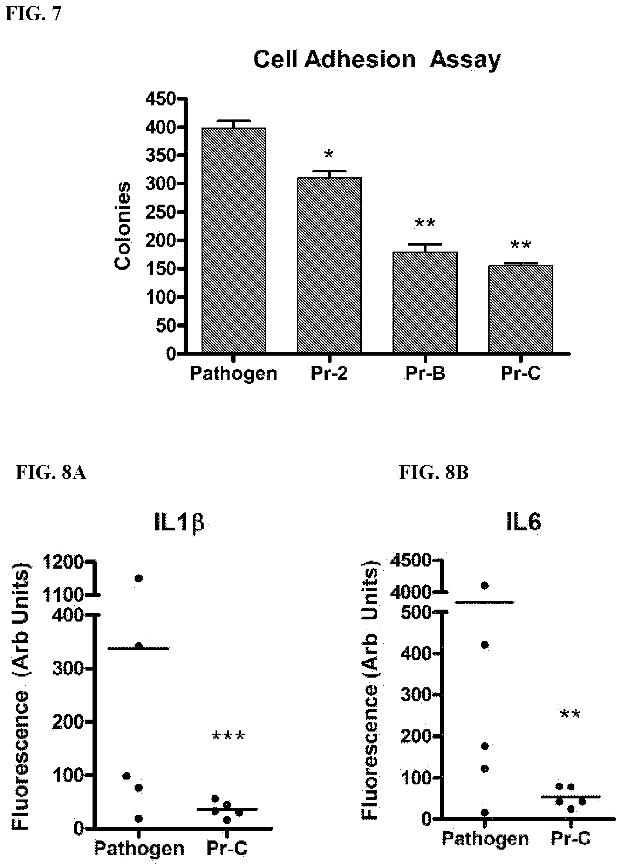

FIG. 7. Probiotic strains adhere significantly less to epithelial cells than pathogenic *P*. *acnes.* Select probiotic strains were incubated with confluent A-431 epithelial cells (MOI 10). After washing the wells, cells were lifted using 0.1% Tween 80 solution and plated on BHI plates. After anaerobic incubation for 72 hours, colonies were counted. The data show that probiotic strains showed significantly lower binding to epithelial cells (* p<0.05, ** p<0.005).

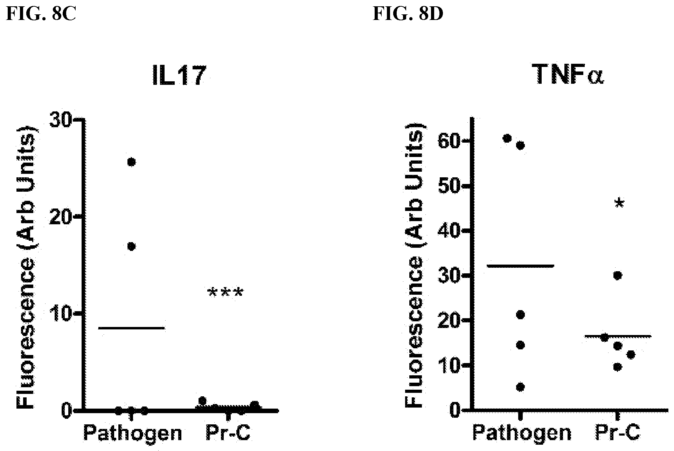

FIGS. 8A-8D. Lower inflammatory potential of probiotic strain in mouse ear inflammation model. CBA/J mice (5 mice per cohort) were injected with *P. acnes* strains, and cytokine analysis was performed at day 5. The probiotic strain Pr#C showed significantly lower levels (* p<0.05, ** p<0.01, *** p<0.0001) of inflammatory cytokines IL-1β (FIG. 8A), IL-6 (FIG. 8B), IL-17 (FIG. 8C), and TNFα (FIG. 8D ) than the pathogenic strain. Pr-C has the ProII 16S sequence.

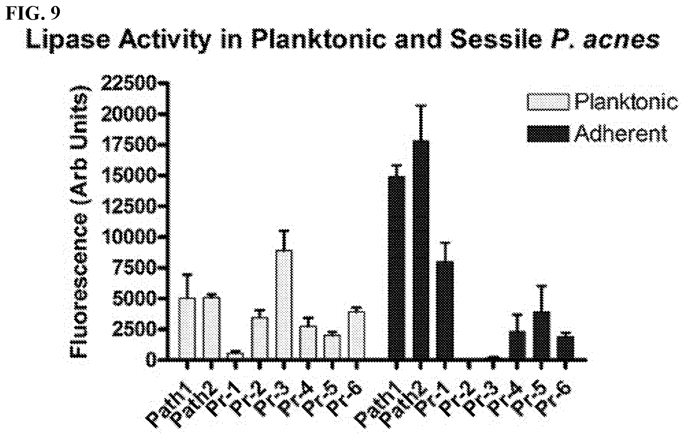

FIG. 9. *P. acnes* strains have different lipase profiles in planktonic and sessile cultures. A set of two pathogenic (Path-1, Path-2) and two probiotic (Pr-1 to Pr-6) *P. acnes* strains were evaluated for lipase production in planktonic (gray bars) and sessile (black bars) cultures. While the lipase production of probiotic strains was not significantly different from the pathogenic strains in liquid (planktonic) culture, their lipase output in adherent culture was consistently lower than corresponding pathogenic cultures. Interestingly, variability in lipase production amongst probiotic strains was observed. The strains with lowest lipase activity were selected.

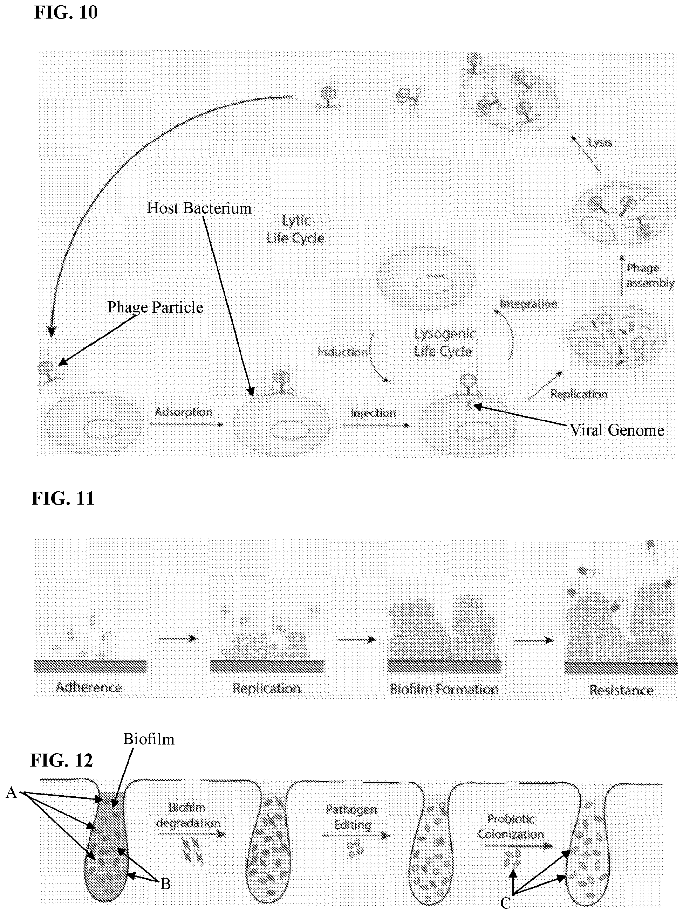

FIG. 10. illustrates life-cycles of exemplary bacteriophages. Anticlockwise from bottom left: A phage particle recognizes and adsorbs onto the surface of the host bacterium. The phage genome is injected into the bacteria. In the lysogenic life cycle, this DNA gets integrated into the bacterial genome and replicates with it for several cycles. In the lytic life cycle, the genome does not integrate and proceeds to hijack the host machinery to replicate its genome and phage structural components. The fully assembled phage then lyses the cell, typically by producing endolysins and holins at the late stage of infection. The liberated phages are now free to seek out and infect a new host bacterium, initiating another lytic cycle.
FIG. 11 illustrates the formation of exemplary bacterial biofilms. Bacterial cells land and adhere to a surface with favorable conditions for growth. They replicate to form a colony, until a certain threshold of cell density (quorum) triggers biofilm formation. The biofilm includes a mixture of polysaccharides, proteins, DNA and lipids in varying proportions. The biofilm is a physical barrier that protects the bacterial colony from harsh external conditions and grants resistance to antibiotics, toxins and immune cells.
FIG. 12 illustrates an embodiment of three components act in concert; their effects are described sequentially for exposition. An inflamed comedone is typically clogged with the biofilm produced by overgrown *P. acnes* (A), along with commensal skin bacteria (B). The biofilm-degrading enzyme (bolts) breaks down the *P. acnes* biofilm to provide better access for the other components. The bacteriophage (hexagons) then edits or specifically kills the pathogenic *P. acnes* and clears the infection. Finally, the probiotic bacteria (C) colonize the pore and occupy the niche of the pathogen, preventing it from growing back and recalibrating the microbiome to a healthy state.

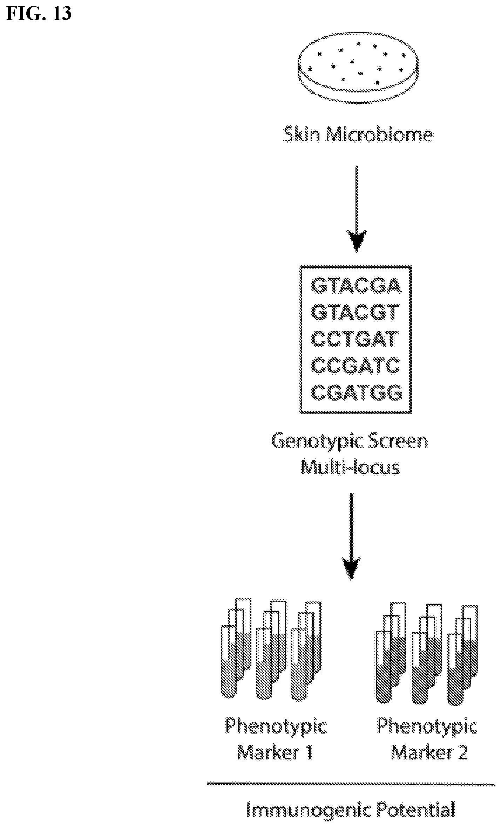

FIG. 13 is a cartoon of a non-limiting probiotic bacterium screening process.

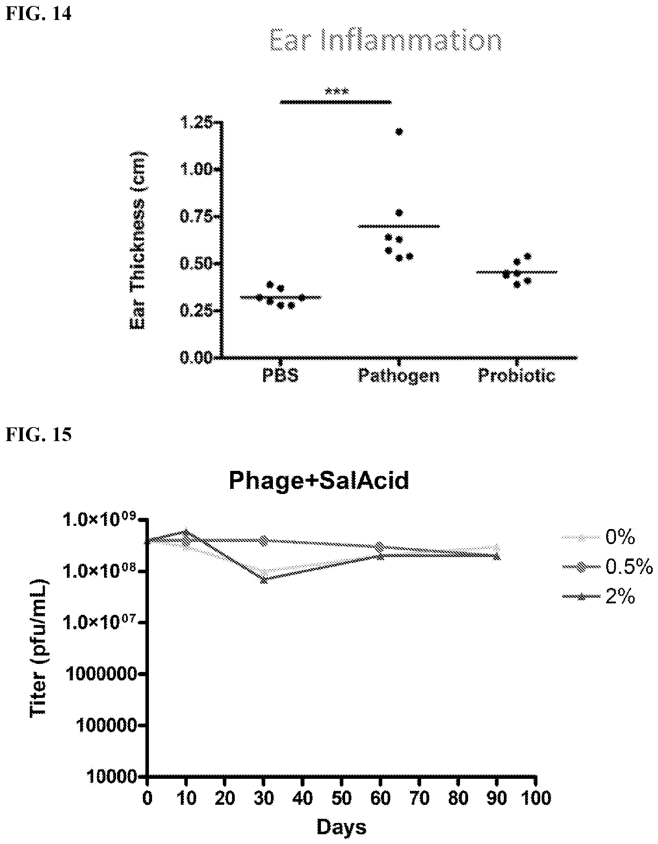

FIG. 14 is a graph showing that the pathogenic strain produces significantly higher ear inflammation than PBS control, while the lead probiotic strain Pr-C induces ear inflammation not significantly different from PBS control.
FIG. 15 is a graph showing that a phage remains stable in the presence of low (0.5% w/v) and high (2% w/v) concentrations of salicylic acid.

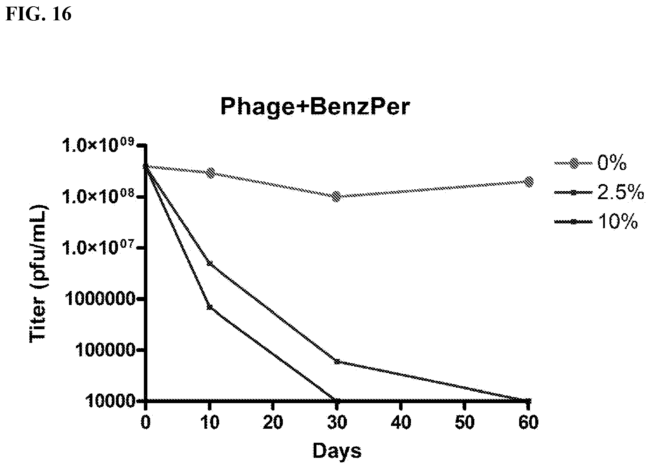

FIG. 16 is a graph showing that a phage loses its viability in the presence of benzoyl peroxide over 60 days. The rate of loss of phage viability is greater at the higher concentration (10% w/v) compared to the lower concentration (2.5% w/v).

### DETAILED DESCRIPTION

Provided herein, are, *inter alia,* compositions, combinations, methods, and systems for treating and preventing acne.

Salicylic acid and benzoyl peroxide are the most commonly used anti-acne agents in over-the-counter (OTC) products. The stability of phages in combination with these anti-acne agents is unknown, especially since phages diverge widely in their stability and response to external physical and chemical factors. The redox properties of benzoyl peroxide and sulfur can potentially cause the degradation of the protein coat of the phage. Previous studies have shown that exposure to peroxide increases the rate of protein degradation by destabilizing the protein and increasing its susceptibility to proteolysis (Fligiel et al. Protein degradation following treatment with hydrogen peroxide. Am J Pathol 1984, 115 (3), 418-25; Kocha et al. Hydrogen peroxide-mediated degradation of protein: different oxidation modes of copper- and iron-dependent hydroxyl radicals on the degradation of albumin. Biochim Biophys Acta 1997, 1337 (2), 319-26). Salicylic acid is noted for its protein-binding ability (Lee et al. Protein binding of acetylsalicylic acid and salicylic acid in porcine and human serum. Vet Hum Toxicol 1995, 37 (3), 224-5; Verbeeck and Cardinal, Plasma protein binding of salicylic acid, phenytoin, chlorpromazine, propranolol and pethidine using equilibrium dialysis and ultracentrifugation. Arzneimittelforschung 1985, 35 (6), 903-6), and a high affinity for the protein coat of the capsid or the tail fibers would render the phage unviable.

Surprisingly, a *Propionibacterium acnes* bacteriophage was found to be stable in compositions that include salicylic acid. See, for example, FIG. 15. Thus, salicylic acid is shown to be well tolerated by the phage and is a suitable anti-acne agent for co-formulation. In embodiments, the anti-keratolytic activity of the salicylic acid complements phage activity by enabling deeper penetration of the phage, thereby increasing its killing efficiency. In embodiments, phages as described herein may be combined with salicylic acid in compositions for preventing and treating acne.

While benzoyl peroxide is not suitable for co-formulation with the phage tested (see FIG. 16) for fomulations that wll be stored for more than, *e.g.,* a few days, benzoyl peroxide can be used along with a phage product as part of an anti-acne combination (e.g., a kit). In embodiments, the benzoyl peroxide is an active ingredient in a cleanser, which is applied to the skin and washed off prior to the application of a comprising the phage composition/formulation. In embodiments, the anti-keratolytic and transient antibacterial action of the benzoyl peroxide complements the specific deeper and targeted killing of *P. acnes* by the bacteriophage.

In embodiments, a *Propionibacterium acnes* bacteriophage and an anti-acne compound comprising salicylic acid (such as salicylic acid optionally in combination with sulfur) are in a single composition that is topically administered to the skin of a subject. In embodiments, a kit that includes a *Propionibacterium acnes* bacteriophage and an anti-acne compound comprising salicylic acid *(e.g.* in separate containers, such as bottles) is provided. In embodiments, a *Propionibacterium acnes* bacteriophage is in one composition and an anti-acne compound comprising salicylic acid (such as salicylic acid optionally in combination with benzoyl peroxide, and/or sulfur) is in another composition, and each composition is topically administered to the skin of a subject. In embodiments, the *Propionibacterium acnes* bacteriophage is administered to the subject, and then the anti-acne compound is administered to the subject. In embodiments, the anti-acne compound is administered to the subject, and then the *Propionibacterium acnes* bacteriophage is administered to the subject. In embodiments, the subject's face is washed between when the anti-acne compound and the *Propionibacterium acnes* bacteriophage (in either order) are topically administered to the face of the subject.

In embodiments, the effective dose of the anti-acne compound comprising salicylic acid (such as salicylic acid optionally in combination with benzoyl peroxide, and/or sulfur ) when used in combination with the *Propionibacterium acnes* bacteriophage is less than would be required if the anti-acne compound was used alone. In embodiments, the effective dose of the anti-acne compound comprising salicylic acid when used in combination with the *Propionibacterium acnes* bacteriophage is less than 90%, 80%, 70%, 60%, 50%, 40%, 30%, 20%, or 10% less than the dose that would be required if the anti-acne compound was used alone.

### DEFINITIONS

The following definitions are included for the purpose of understanding the present subject matter and for constructing the appended patent claims. The abbreviations used herein have their conventional meanings within the chemical and biological arts.

While various embodiments and aspects of the present invention are shown and described herein, it will be obvious to those skilled in the art that such embodiments and aspects are provided by way of example only. It should be understood that various alternatives to the embodiments of the invention described herein may be employed in practicing the invention.

The section headings used herein are for organizational purposes only and are not to be construed as limiting the subject matter described.

Unless defined otherwise, technical and scientific terms used herein have the same meaning as commonly understood by a person of ordinary skill in the art. *See, e.g.,* Singleton et al., DICTIONARY OF MICROBIOLOGY AND MOLECULAR BIOLOGY 2nd ed., J. Wiley & Sons (New York, NY 1994); Sambrook et al., MOLECULAR CLONING, A LABORATORY MANUAL, Cold Springs Harbor Press (Cold Springs Harbor, NY 1989). Any methods, devices and materials similar or equivalent to those described herein can be used in the practice of this invention. The following definitions are provided to facilitate understanding of certain terms used frequently herein and are not meant to limit the scope of the present disclosure

As used herein a *"Propionibacterium acnes* bacteriophage" is a bacteriophage that infects, replicates within, and kills *P. acnes* cells. The *P. acnes* bacteriophage according to the invention has a genome which comprises a nucleotide sequence that is at least about 98%, or 99% identical to the nucleotide sequence of SEQ ID NO: 1. In embodiments, a *P. acnes* bacteriophage is a lytic *P. acnes* bacteriophage. In embodiments, a *P. acnes* bacteriophage is capable of lysing a *P. acnes* bacterium and incapable of lysing any bacterium which is not *P*. *acnes.* In embodiments, a *P. acnes* bacteriophage is incapable of sustaining lysogeny in a bacterium. In embodiments, the use of a bacteriophage that can lyse *P. acnes* but is incapable of sustaining lysogeny has the advantage that the bacteriophage cannot lie dormant within a bacterium, but must lyse the bacterium and hence kill it. In embodiments, a *P. acnes* bacteriophage lacks the ability to express at least one gene necessary for sustaining lysogeny. The term "lacks the ability to express at least one gene necessary for sustaining lysogeny" is intended to indicate that the *P. acnes* bacteriophage lacks the ability to produce a fully functional protein product necessary to sustain lysogeny, for example, as the result of one or more point mutations or full or partial deletions of the genome. In embodiments, the *P. acnes* bacteriophage has a genome that lacks all or part of at least one gene necessary for sustaining lysogeny (*e.g*., artificially or naturally, *e.g*., the strain is or is derived from a strain that lacks all or part of at least one gene necessary for sustaining lysogeny). In embodiments, the *P. acnes* bacteriophage may comprise defects (*e.g*. mutations, insertions or deletions) in the genome in non-coding regions that may, nonetheless, affect the ability of the phage to sustain lysogeny, for example defects in the genome integration site(s) *(e.g.* a /att/ site) or in a repressor binding site. In embodiments, a *P. acnes* bacteriophage is naturally occurring and isolated, with the added advantage that artificial mutations need not be introduced into the bacteriophage. In embodiments, a *P. acnes* bacteriophage is capable of lysing a plurality of strains of the *P. acnes* bacterium. In embodiments, a *P. acnes* bacteriophage is capable of lysing at least about 5, 10, 15, 20, 25, 30 or more strains of the *P. acnes* bacterium. Non-limiting examples of *P. acnes* bacteriophages are disclosed herein. In embodiments, a *P. acnes* bacteriophage has a genome having the sequence of SEQ ID NO: 1, or includes the sequence of SEQ ID NO: 1. In embodiments, the genome of the *P. acnes* bacteriophage has no insertions or deletions compared to SEQ ID NO: 1. In embodiments, the genome of the *P. acnes* bacteriophage has no insertions or deletions, and only conservative substitutions compared to SEQ ID NO: 1. In embodiments, the *P. acnes* bacteriophage is one of the following exemplary isolates of *P. acnes* bacteriophages that have been deposited under the terms of the Budapest Treaty at The National Collection of Industrial, Marine and Food Bacteria (NCIMB), Ferguson Building, Craibstone Estate, Bucksburn, Aberdeen, AB21 9YA, United Kingdom, under the following accession numbers: Accession no. NCIMB 41332 (isolate PA6); Accession no. NCIMB 41334 (isolate 1874); Accession no. NCIMB 41333 (isolate 1878); Accession no. NCIMB 41335 (isolate 1905); Accession no. NCIMB 41349 (isolate 1894); Accession no. NCIMB 41350 (isolate 103609); Accession no. NCIMB 41351 (isolate 103672). In embodiments, a non-limiting example of a host bacterium, *P. acnes,* AT1 has been deposited as NCIMB 41336. In embodiments, a*P*. *acnes* bacteriophage has a genome having sequence identity of at least about 80%, 81%, 82%, 83%, 84%, 85%, 86%, 87%, 88%, 89%, 90%, 95%, or 99% with the genome of the bacteriophage deposited under Accession No. NCIMB 41349. In embodiments, a *P*. *acnes* bacteriophage has a genome having sequence identity of at least 87% with the genome of the bacteriophage deposited under Accession No. NCIMB 41350. In embodiments, a *P*. *acnes* bacteriophage has a genome having sequence identity of at least 88% with the genome of the bacteriophage deposited under Accession No. NCIMB 41351. Additional non-limiting descriptions relating to *P. acnes* bacteriophages are provided in U.S. Patent No. 9,068,159 B2, issued June 30, 2015. The terms "phage" and "bacteriophage" are used interchangeably herein.

As used herein, "degrading" a biofilm means cleaving a covalent bond of at least one compound that forms part of a biofilm *(e.g.,* by enzymatic activity). Non-limiting examples of compounds that may form a part of a biofilm include polymers, glycosides, proteins, polysaccharides, and nucleic acids. As used herein, a *"P. acnes* biofilm degrading enzyme" is an enzyme that degrades at least one compound that forms part of a *P. acnes* biofilm.

The enzymes as provided herein include any naturally occurring forms, homologs, isoforms or variants that maintain the enzymatic activity (e.g., within at least 50%, 80%, 90%, 95%, 96%, 97%, 98%, 99% or 100% activity compared to the native protein). In embodiments, variants have at least 90%, 95%, 96%, 97%, 98%, 99% or 100% amino acid sequence identity across the whole sequence or a portion of the sequence *(e.g.* a 50, 100, 150 or 200 continuous amino acid portion) compared to a naturally occurring form.

The term "isolated," when applied to a bacterium or bacteriophage, refers to a bacterium or bacteriophage that has been (1) separated from at least some of the components with which it was associated when initially produced (whether in nature or in an experimental setting), and/or (2) produced, prepared, purified, and/or manufactured by the hand of man, *e.g.* using artificial culture conditions such as (but not limited to) growing on a plate and/or in a fermenter. Isolated bacteria include those bacteria that are cultured, even if such cultures are not monocultures. In embodiments, the isolated bacteria are bacteria that are cultured as a monoculture (e.g., on a plate or in liquid culture such as in a fermenter). Isolated bacteria and bacteriophages may be separated from at least about 10%, about 20%, about 30%, about 40%, about 50%, about 60%, about 70%, about 80%, about 90%, about 95%, about 96%, about 97%, about 99% or more of the other components with which they were initially associated (e.g., by weight). In embodiments, isolated bacteria are more than about 80%, about 85%, about 90%, about 91%, about 92%, about 93%, about 94%, about 95%, about 96%, about 97%, about 98%, about 99%, or more than about 99% pure *(e.g.,* by weight). In embodiments, isolated bacteriophages are more than about 80%, about 85%, about 90%, about 91%, about 92%, about 93%, about 94%, about 95%, about 96%, about 97%, about 98%, about 99%, or more than about 99% pure *(e.g.,* by weight). In embodiments, a composition provided herein includes one or more isolated bacteriophages. In embodiments, a composition provided herein includes an isolated bacteriophage. In embodiments, a bacteriophage that is administered is an isolated bacteriophage. In embodiments, a composition provided herein includes one or more isolated bacteria. In embodiments, a composition provided herein includes an isolated bacterium. In embodiments, a bacterium that is administered is an isolated bacterium.

A "control" sample or value refers to a sample that serves as a reference, usually a known reference, for comparison to a test sample. For example, a test sample can be taken from a test condition, *e.g.,* in the presence of a test compound *(e.g.,* enzyme) or phage, and compared to samples from known conditions, *e.g.,* in the absence of the test compound, phage, or bacterium (negative control), or in the presence of a known compound, phage, or bacterium (positive control). A control can also represent an average value gathered from a number of tests or results. One of skill in the art will recognize that controls can be designed for assessment of any number of parameters. For example, a control can be devised to compare therapeutic benefit based on pharmacological data *(e.g.,* half-life, the degradation of a biofilm or a component thereof, or bacterial cell lysis) or therapeutic measures (*e.g*., comparison of side effects). One of skill in the art will understand which controls are valuable in a given situation and be able to analyze data based on comparisons to control values. Controls are also valuable for determining the significance of data. For example, if values for a given parameter are widely variant in controls, variation in test samples will not be considered as significant.

"Nucleic acid" refers to nucleotides (e.g., deoxyribonucleotides or ribonucleotides) and polymers thereof in either single-, double- or multiple-stranded form, or complements thereof. The terms "polynucleotide," "oligonucleotide," "oligo" or the like refer, in the usual and customary sense, to a linear sequence of nucleotides. Oligonucleotides are typically from about 5, 6, 7, 8, 9, 10, 12, 15, 25, 30, 40, 50 or more nucleotides in length, up to about 100 nucleotides in length. Polynucleotides are polymers of any length, including longer lengths, *e.g.,* 200, 300, 500, 1000, 2000, 3000, 5000, 7000, 10000, 20000, 30000, 40000 *etc.* Polynucleotides and oligonucleotides will generally contain phosphodiester bonds, although in some cases, nucleic acid analogs are included that may have alternate backbones, that include, *e.g*., phosphoramidate, phosphorothioate, phosphorodithioate, or O-methylphophoroamidite linkages (see Eckstein, Oligonucleotides and Analogues: A Practical Approach, Oxford University Press); and peptide nucleic acid backbones and linkages. Other analog nucleic acids include those with positive backbones; non-ionic backbones, and non-ribose backbones, including those described in U.S. Patent Nos. 5,235,033 and 5,034,506, and Chapters 6 and 7, ASC Symposium Series 580, *Carbohydrate Modifications in Antisense Research,* Sanghui & Cook, eds. Nucleic acids containing one or more carbocyclic sugars are also included within one definition of nucleic acids. Modifications of the ribose-phosphate backbone may be done for a variety of reasons, *e.g*., to increase the stability and half-life of such molecules in physiological environments or as probes on a biochip. Mixtures of naturally occurring nucleic acids and analogs can be made; alternatively, mixtures of different nucleic acid analogs, and mixtures of naturally occurring nucleic acids and analogs may be made.

The term "bp" and the like refer, in the usual and customary sense, to the indicated number of base pairs.

"Percentage of sequence identity" is determined by comparing two optimally aligned sequences over a comparison window, wherein the portion of the polynucleotide or polypeptide sequence in the comparison window may comprise additions or deletions (*i.e.,* gaps) as compared to the reference sequence (which does not comprise additions or deletions) for optimal alignment of the two sequences. In embodiments, the percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity.

The terms "identical" or percent "identity," in the context of two or more nucleic acids or polypeptide sequences, refer to two or more sequences or subsequences that are the same or have a specified percentage of amino acid residues or nucleotides that are the same (*i.e.,* 50%, 55%, 60%, 65%, 70%, 75%, 80%, 85%, 90%, 91%, 92%, 93%, 94%, 95%, 96%, 97%, 98%, 99%, or more identity over a specified region, *e.g.,* of an entire nucleic acid or polypeptide sequence or individual portions or domains of a nucleic acid or polypeptide), when compared and aligned for maximum correspondence over a comparison window, or designated region as measured using one of the following sequence comparison algorithms or by manual alignment and visual inspection. Such sequences are then said to be "substantially identical." This definition also refers to the complement of a test sequence. In embodiments, the identify exists over a region that is about or at least about 20, 50, 100, 1000, 2500, 5000, 7500, 10000, 15000, 20000, 25000, or 30000 amino acids or nucleotides in length to about, less than about, or at least about 31000, 32000, 33000, 34000 or 35000 amino acids or nucleotides in length. Optionally, the identity exists over a region that is at least about 10 to about 100, about 20 to about 75, about 30 to about 50 amino acids or nucleotides in length. Optionally, the identity exists over a region that is at least about 50 amino acids in length, or more preferably over a region that is 100 to 500 or 1000 or more amino acids in length. Included herein are phages comprising nucleic acids *(e.g.,* a genome or a portion thereof) having sequences that are substantially identical to any of SEQ ID NOs: 1, 11, 13, 15, 17, 19, 21, 23, 25, or 27. Non-limiting examples of phages provided herein comprise genomes having sequences that are substantially identical to SEQ ID NO: 1.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Preferably, default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of any one of the number of contiguous positions in which a sequence may be compared to a reference sequence of the same number of contiguous positions after the two sequences are optimally aligned. In embodiments, a comparison window includes about or at least about 20, 50, 100, 1000, 2500, 5000, 7500, 10000, 15000, 20000, 25000, or 30000 to about, less than about, or at least about 31000, 32000, 33000, 34000 or 35000 contiguous positions. In embodiments, a comparison window includes about or at least about 20 to about, less than about, or at least about 31000 contiguous positions. In embodiments, a comparison window includes about or at least about 25000 to about, less than about, or at least about 31000 contiguous positions. In embodiments, a comparison window includes about or at least about 26000 to about, less than about, or at least about 31000 contiguous positions. In embodiments, a comparison window includes about or at least about 27000 to about, less than about, or at least about 31000 contiguous positions. In embodiments, a comparison window includes about or at least about 28000 to about, less than about, or at least about 31000 contiguous positions. In embodiments, a comparison window includes about or at least about 29000 to about, less than about, or at least about 31000 contiguous positions. In embodiments, a comparison window includes about or at least about 30000 to about, less than about, or at least about 31000 contiguous positions. In embodiments, a comparison includes about 20 to about 600, about 50 to about 200, or about 100 to about 150 contiguous positions. In embodiments, the comparison window is the entire length of a reference sequence, such as the sequence of a bacteriophage genome. Methods of alignment of sequences for comparison are well-known in the art. In embodiments, optimal alignment of sequences for comparison can be conducted, *e.g*., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, Wis.), or by manual alignment and visual inspection (*see, e.g.,* Current Protocols in Molecular Biology (Ausubel et al., eds. 1995 supplement)).

An example of algorithms suitable for determining percent sequence identity and sequence similarity are the BLAST and BLAST 2.0 algorithms, which are described in Altschul et al., Nuc. Acids Res. 25:3389-3402 (1977) and Altschul et al., J. Mol. Biol. 215:403-410 (1990), respectively. As will be appreciated by one of skill in the art, the software for performing BLAST analyses is publicly available through the website of the National Center for Biotechnology Information (NCBI). In embodiments, BLAST and BLAST 2.0 are used, with the parameters described herein, to determine percent sequence identity for the nucleic acids and proteins. In embodiments, a BLAST algorithm involves first identifying high scoring sequence pairs (HSPs) by identifying short words of length W in the query sequence, which either match or satisfy some positive-valued threshold score T when aligned with a word of the same length in a database sequence. In embodiments, T is referred to as the neighborhood word score threshold (Altschul et al., supra). In embodiments, these initial neighborhood word hits act as seeds for initiating searches to find longer HSPs containing them. In embodiments, the word hits are extended in both directions along each sequence for as far as the cumulative alignment score can be increased. In embodiments, cumulative scores are calculated using, for nucleotide sequences, the parameters M (reward score for a pair of matching residues; always >0) and N (penalty score for mismatching residues; always <0). In embodiments, for amino acid sequences, a scoring matrix is used to calculate the cumulative score. In embodiments, extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. In embodiments, the BLAST algorithm parameters W, T, and X determine the sensitivity and speed of the alignment. In embodiments, the NCBI BLASTN or BLASTP program is used to align sequences. In embodiments, the BLASTN or BLASTP program uses the defaults used by the NCBI. In embodiments, the BLASTN program (for nucleotide sequences) uses as defaults: a word size (W) of 28; an expectation threshold (E) of 10; max matches in a query range set to 0; match/mismatch scores of 1, -2; linear gap costs; the filter for low complexity regions used; and mask for lookup table only used. In embodiments, the BLASTP program (for amino acid sequences) uses as defaults: a word size (W) of 3; an expectation threshold (E) of 10; max matches in a query range set to 0; the BLOSUM62 matrix (*see* Henikoff & Henikoff, Proc. Natl. Acad. Sci. USA 89:10915 (1992)); gap costs of existence: 11 and extension: 1; and conditional compositional score matrix adjustment.

The terms "polypeptide," "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, *e.g*., hydroxyproline, γ-carboxyglutamate, and O-phosphoserine. Amino acid analogs refers to compounds that have the same basic chemical structure as a naturally occurring amino acid, *i.e.,* an α carbon that is bound to a hydrogen, a carboxyl group, an amino group, and an R group, *e.g*., homoserine, norleucine, methionine sulfoxide, methionine methyl sulfonium. Such analogs have modified R groups *(e.g.,* norleucine) or modified peptide backbones, but retain the same basic chemical structure as a naturally occurring amino acid. Amino acid mimetics refers to chemical compounds that have a structure that is different from the general chemical structure of an amino acid, but that functions in a manner similar to a naturally occurring amino acid.

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

"Conservatively modified variants" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid which encodes a polypeptide is implicit in each described sequence with respect to the expression product, but not with respect to actual probe sequences.

As to amino acid sequences, one of skill will recognize that individual substitutions to a peptide, polypeptide, or protein sequence which alters a single amino acid is a "conservatively modified variant" where the alteration results in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles.

The following eight groups each contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (see, e.g., Creighton, Proteins (1984)).

The term "disease" refers to any deviation from the normal health of a mammal and includes a state when disease symptoms are present, as well as conditions in which a deviation *(e.g.,* dysbiosis, infection, gene mutation, genetic defect, *etc.*) has occurred, but symptoms are not yet manifested. In embodiments, the disease is acne. In embodiments, the disease includes dermal dysbiosis. In embodiments, methods, compositions, systems, phages, and probiotic bacteria provided herein are suitable for use in a subject that is a member of the Vertebrate class, Mammalia, including, without limitation, primates (such as humans), livestock, work animals, and domestic pets *(e.g.,* a companion animal). In embodiments, a subject is a human subject. As used herein, a "symptom" of a disease includes and clinical or laboratory manifestation associated with the disease, and is not limited to what a subject can feel or observe.

As used herein, the term "dermal dysbiosis" means a difference in the skin microbiota compared to a healthy or general population. In embodiments, the dysbiosis is on the surface of the skin, within skin (*e*.*g*., within a skin region or layer of skin cells), within a gland, and/or within a pore of the skin. In embodiments, the dysbiosis is within sweat and/or sebum. In embodiments, the skin is on the face *(e.g.,* the forehead, one or more cheeks, the nose, or the chin of a subject). In embodiments, the skin is on the shoulders, chest, or back. In embodiments, dermal dysbiosis includes a change in microbiota commensal species diversity as compared to a healthy or general population and may include decrease of beneficial microorganisms and/or increase of pathobionts (pathogenic or potentially pathogenic microorganisms) and/or decrease of overall microbiota species diversity. Many factors can lead to dysbiosis, including hormonal changes (*e.g*., during adolescence), infrequent washing, cosmetic use, antibiotic use, psychological and physical stress, radiation, and dietary changes.

In embodiments, compositions are administered to a subject suffering from acne in a "therapeutically effective dose." Amounts effective for this use may depend upon the severity of the disease and the general state of the patient's health. Single or multiple administrations of the compositions may be administered depending on the dosage and frequency as required and tolerated by the patient. A "patient" or "subject" includes both humans and other animals, particularly mammals. Thus the methods are applicable to both human therapy and veterinary applications.

"Pharmaceutically acceptable excipient" and "pharmaceutically acceptable carrier" refer to a substance that aids the administration of an active agent to and absorption by a subject and can be included in the compositions of the present invention without causing a significant adverse toxicological effect on the patient. Non-limiting examples of pharmaceutically acceptable excipients include water, NaCl, normal saline solutions, lactated Ringer's, normal sucrose, normal glucose, binders, fillers, disintegrants, lubricants, coatings, sweeteners, flavors, salt solutions (such as Ringer's solution), alcohols, oils, gelatins, carbohydrates such as lactose, amylose or starch, fatty acid esters, hydroxymethycellulose, polyvinyl pyrrolidine, and colors, and the like. Such preparations can be sterilized and, if desired, mixed with auxiliary agents such as lubricants, preservatives, stabilizers, wetting agents, emulsifiers, salts for influencing osmotic pressure, buffers, coloring, and/or aromatic substances and the like that do not deleteriously react with the bacteriophages, probiotic bacteria, and/or compounds of the invention. One of skill in the art will recognize that other pharmaceutical excipients are useful in the present invention.

The term "contacting" may include allowing two species to react, interact, or physically touch, wherein the two species may be, for example, an enzyme as described herein and a biofilm that includes a substrate of the enzyme. In another example, the two species may be a bacteriophage and a cell of a species that the bacteriophage infects. In embodiments contacting includes, for example, allowing a bacteriophage as described herein to interact with a *P. acnes* cell. In embodiments contacting includes, for example, allowing an enzyme as described herein to interact with a *P. acnes* biofilm.

"Patient" or "subject in need thereof" refers to a living member of the animal kingdom suffering from or who may suffer from the indicated disorder. In embodiments, the subject is a member of a species that includes individuals who naturally suffer from the disease. In embodiments, the subject is a mammal. Non-limiting examples of mammals include rodents *(e.g.,* mice and rats), primates *(e.g.,* lemurs, bushbabies, monkeys, apes, and humans), rabbits, dogs (e.g., companion dogs, service dogs, or work dogs such as police dogs, military dogs, race dogs, or show dogs), horses (such as race horses and work horses), cats (e.g., domesticated cats), livestock (such as pigs, bovines, donkeys, mules, bison, goats, camels, and sheep), and deer. In embodiments, the subject is a human.

The terms "subject," "patient," "individual," *etc.* are not intended to be limiting and can be generally interchanged. That is, an individual described as a "patient" does not necessarily have a given disease, but may be merely seeking medical advice.

As used herein the abbreviation "sp." for species means at least one species (e.g., 1, 2, 3, 4, 5, or more species) of the indicated genus. The abbreviation "spp." for species means 2 or more species *(e.g.,* 2, 3, 4, 5, 6, 7, 8, 9, 10 or more) of the indicated genus. In embodiments, methods and compositions provided herein comprise a single species within an indicated genus or indicated genera, or 2 or more *(e.g.,* a plurality that includes more than 2) species within an indicated genus or indicated genera. In embodiments, 1, 2, 3, 4, 5, or more or all or the indicated species is or are isolated. In embodiments, the indicated species are administered together. In embodiments, each of the indicated species is present in a single composition that includes each of the species. In embodiments, each of the species is administered concurrently, *e.g*., within about 1, 2, 3, 4, 5, 6, 7, 8, 9, 10, 30, or 60, 1-5, 1-10, 1-30, 1-60, or 5-15 seconds or minutes of each other.

In this disclosure, "comprises," "comprising," "containing," and "having" and the like can have the meaning ascribed to them in U.S. Patent law and can mean "includes," "including," and the like. Thus, the transitional term "comprising," which is synonymous with "including," "containing," or "characterized by," is inclusive or open-ended and does not exclude additional, unrecited features, integers, steps, operations, elements, and/or components. "Consisting essentially of" or "consists essentially" likewise has the meaning ascribed in U.S. Patent law and the term is open-ended, allowing for the presence of more than that which is recited so long as basic or novel characteristics of that which is recited is not changed by the presence of more than that which is recited, but excludes prior art embodiments. By contrast, the transitional phrase "consisting of" excludes any feature, integer, element, step, operation, component, and/or ingredient not specified.

As used herein, the term "about" in the context of a numerical value or range means ±10% of the numerical value or range recited or claimed, unless the context requires a more limited range.

In the descriptions herein and in the claims, phrases such as "at least one of" or "one or more of" may occur followed by a conjunctive list of elements or features. The term "and/or" may also occur in a list of two or more elements or features. Unless otherwise implicitly or explicitly contradicted by the context in which it is used, such a phrase is intended to mean any of the listed elements or features individually or any of the recited elements or features in combination with any of the other recited elements or features. For example, the phrases "at least one of A and B;" "one or more of A and B;" and "A and/or B" are each intended to mean "A alone, B alone, or A and B together." A similar interpretation is also intended for lists including three or more items. For example, the phrases "at least one of A, B, and C;" "one or more of A, B, and C;" and "A, B, and/or C" are each intended to mean "A alone, B alone, C alone, A and B together, A and C together, B and C together, or A and B and C together." In addition, use of the term "based on," herein and in the claims is intended to mean, "based at least in part on," such that an unrecited feature or element is also permissible.

It is understood that where a parameter range is provided, all integers within that range, and tenths thereof, are also provided by the invention. For example, "0.2-5 mg" is a disclosure of 0.2 mg, 0.3 mg, 0.4 mg, 0.5 mg, 0.6 mg etc. up to and including 5.0 mg.

As used in the description herein and throughout the claims that follow, the meaning of "a," "an," and "the" includes plural reference unless the context clearly dictates otherwise.

As used herein, "treating" or "treatment" of a condition, disease or disorder or symptoms associated with a condition, disease or disorder refers to an approach for obtaining beneficial or desired results, including clinical results. Beneficial or desired clinical results can include, but are not limited to, alleviation or amelioration of one or more symptoms or conditions, diminishment of extent of condition, disorder or disease, stabilization of the state of condition, disorder or disease, prevention of development of condition, disorder or disease, prevention of spread of condition, disorder or disease, delay or slowing of condition, disorder or disease progression, delay or slowing of condition, disorder or disease onset, amelioration or palliation of the condition, disorder or disease state, and remission, whether partial or total. "Treating" can also mean inhibiting the progression of the condition, disorder or disease, slowing the progression of the condition, disorder or disease temporarily, although in some instances, it involves halting the progression of the condition, disorder or disease permanently. In the case of treating acne, the terms can refer to reducing, *e.g*., dermal dysbiosis and/or the number or size of cystic lesions, whiteheads (closed plugged pores), blackheads (open plugged pores - in which oil exposed to the air has a dark color, *e.g*., brown or black), mall red, tender bumps (papules), pimples (pustules; papules with pus at their tips), large, solid, painful lumps beneath the surface of the skin (nodules).

As used herein, the terms "treat" and "prevent" are not intended to be absolute terms. In embodiments, treatment can refer to a 10%, 20%, 30%, 40%, 50%, 60%, 70%, 80%, 90%, or 100% reduction in the severity of an established disease, condition, or symptom of the disease or condition. In embodiments, a method for treating a disease is considered to be a treatment if there is a 10% reduction in one or more symptoms of the disease in a subject as compared to a control. Thus the reduction can be a 10%, 20%, 30%, 40%, 50%, 60%, 70%, 80%, 90%, 100%, or any percent reduction in between 10% and 100% as compared to native or control levels. It is understood that treatment does not necessarily refer to a cure or complete ablation of the disease, condition, or symptoms of the disease or condition. In embodiments, references to decreasing, reducing, or inhibiting include a change of 10%, 20%, 30%, 40%, 50%, 60%, 70%, 80%, 90% or greater as compared to a control level and such terms can include but do not necessarily include complete elimination. Treatment can refer to any delay in onset, amelioration of symptoms, improvement in patient skin appearance, *etc.* The effect of treatment can be compared to an individual or pool of individuals not receiving the treatment, or to the same patient prior to treatment or at a different time during treatment. In embodiments, the severity of disease is reduced by at least 10%, as compared, *e.g.,* to the individual before administration or to a control individual not undergoing treatment. In some aspects the severity of disease is reduced by at least 25%, 50%, 75%, 80%, or 90%, or in some cases, no longer detectable using standard diagnostic techniques. In embodiments, treatment is effective to reduce at least one symptom of acne. In embodiments, treatment is effective to reduce the level of pimples (pustules) on the face, forehead, chest, back, and/or shoulders of the subject. In embodiments, treatment is effective to reduce the level of whiteheads (closed plugged pores) on the face, forehead, chest, back, and/or shoulders of the subject. In embodiments, treatment is effective to reduce the level of blackheads (open plugged pores) on the face, forehead, chest, back, and/or shoulders of the subject. In embodiments, treatment is effective to reduce the level of papules on the face, forehead, chest, back, and/or shoulders of the subject. In embodiments, treatment is effective to reduce the level of solid, painful lumps beneath the surface of the skin (nodules) on the face, forehead, chest, back, and/or shoulders of the subject. In embodiments, treatment is effective to reduce the level of cystic lesions on the face, forehead, chest, back, and/or shoulders of the subject. In embodiments, the level (e.g., number) is reduced compared to before treatment has begun. In embodiments, the level (e.g., number) is reduced compared to a corresponding subject who is afflicted with acnes but who has not received treatment. In embodiments, the level *(e.g.,* number) is reduced compared to a corresponding subject who is afflicted with acnes but who has not received treatment comprising a bacteriophage.

The terms "effective amount," "effective dose," "therapeutically effective amount," *etc.* refer to the amount of an agent that is sufficient to ameliorate a disorder, as described herein. For example, for the given parameter, a therapeutically effective amount will show an increase or decrease of at least 5%, 10%, 15%, 20%, 25%, 40%, 50%, 60%, 75%, 80%, 90%, or at least 100%. Therapeutic efficacy can also be expressed as "-fold" increase or decrease. For example, a therapeutically effective amount can have at least a 1.2-fold, 1.5-fold, 2-fold, 5-fold, or more effect over a control.

The term "diagnosis" refers to a relative probability a subject has a given metabolic disorder. Symptoms and diagnostic criteria are summarized herein. Similarly, the term "prognosis" refers to a relative probability that a certain future outcome may occur in the subject. For example, in the context of the present invention, prognosis can refer to the likelihood that an individual will develop acne. Prognosis can also refer to the likely severity of the disease (*e.g*., severity of symptoms, rate of functional decline, *etc*.). The terms are not intended to be absolute, as will be appreciated by any one of skill in the field of medical diagnostics.

### COMPOSITIONS AND COMBINATIONS COMPRISING BACTERIOPHAGES

In an aspect of the invention, provided herein is a composition comprising, consisting essentially of, or consisting of at least one *P. acnes* bacteriophage, at least one anti-acne compound comprising salicylic acid, and a pharmaceutically acceptable carrier wherein the genome of the *P. acnes* bacteriophage comprises a nucleotide sequence that is at least about 98%, or 99% identical to the nucleotide sequence of SEQ ID NO: 1.

Also disclosed but not claimed herein is a composition that includes at least one *Propionibacterium acnes* bacteriophage, no more than one anti-acne compound, and a pharmaceutically acceptable carrier.

Also disclosed but not claimed herein is a composition that includes active ingredients consisting of at least one *Propionibacterium acnes* bacteriophage and no more than one anti-acne compound, and a pharmaceutically acceptable carrier.

Also disclosed but not claimed herein is a composition that includes at least one *P*. *acnes* bacteriophage, at least one anti-acne compound, and a pharmaceutically acceptable carrier, wherein the composition does not comprise a probiotic bacterium.

In non-claimed embodiments, the at least one anti-acne compound is benzoyl peroxide. In embodiments, the benzoyl peroxide is present at a concentration of 2.5% to 10% (weight/volume). In embodiments, the benzoyl peroxide is present at a concentration of less than 2.5% but greater than about 0.1%, 0.5%, 1%, 1.5%, or 2% (weight/volume). In embodiments, the benzoyl peroxide is present at a concentration of 2.5% to 10%, *e.g.,* about 2.5%, 3%, 3.5%, 4%, 4.5%, 5%, 5.5%, 6%, 6.5%, 7%, 7.5%, 8%, 8.5%, 9%, 9.5%, or 10% (weight/volume). In embodiments, the benzoyl peroxide is present at a concentration of less than 2.5% but greater than about 0.1%, 0.5%, 1%, 1.5%, or 2% (weight/volume).

In embodiments, the at least one anti-acne compound is salicylic acid. In embodiments, the salicylic acid is present at a concentration of 0.5% to 2% (weight/volume). In embodiments, the salicylic acid is present at a concentration of less than 0.5% but greater than about 0.1% (weight/volume). In embodiments, the salicylic acid is present at a concentration of 0.5% to 2%, *e.g.,* about 0.5%, 0.6%, 0.7%, 0.8%, 0.9%, 1%, 1.1%, 1.2%, 1.3%, 1.4%, 1.5%, 1.6%, 1.7%, 1.8%, 1.9%, or 2% (weight/volume). In embodiments, the salicylic acid is present at a concentration of less than 0.5% but greater than about 0.1% (weight/volume).

In non-claimed embodiments, the at least one anti-acne compound is sulfur. In embodiments, the sulfur is present at a concentration of 3% to 10% (weight/volume). In embodiments, the sulfur is present at a concentration of less than 3% but greater than about 0.1%, 0.5%, 1%, 1.5%, 2%, or 2.5% (weight/volume). In embodiments, the sulfur is present at a concentration of 3% to 10%, *e.g.,* about 3%, 3.5%, 4%, 4.5%, 5%, 5.5%, 6%, 6.5%, 7%, 7.5%, 8%, 8.5%, 9%, 9.5%, or 10% (weight/volume). In embodiments, the sulfur is present at a concentration of less than 3% but greater than about 0.1%, 0.5%, 1%, 1.5%, 2%, or 2.5% (weight/volume). In embodiments, resorcinol is present at a concentration of 2% and sulfur is present at a concentration of 3% to 8% *(e.g.,* about 2.5%, 3%, 3.5%, 4%, 4.5%, 5%, 5.5%, 6%, 6.5%, 7%, 7.5%, or 8%) (weight/volume).

In non-claimed embodiments, the at least one anti-acne compound is resorcinol and sulfur. In embodiments, the resorcinol is present at a concentration of 2% and sulfur is present at a concentration of 3% to 8% (weight/volume). In embodiments, resorcinol is present at a concentration of 2% and sulfur is present at a concentration of 3% to 8% *(e.g.,* about 2.5%, 3%, 3.5%, 4%, 4.5%, 5%, 5.5%, 6%, 6.5%, 7%, 7.5%, or 8%) (weight/volume).

In embodiments, the at least one anti-acne compound includes resorcinol monoacetate and sulfur. In embodiments, the resorcinol monoacetate is present at a concentration of 3% and sulfur is present at a concentration of 3% to 8% (weight/volume). In embodiments, resorcinol monoacetate is present at a concentration of 3% and sulfur is present at a concentration of 3% to *8% (e.g.,* about 2.5%, 3%, 3.5%, 4%, 4.5%, 5%, 5.5%, 6%, 6.5%, 7%, 7.5%, or 8%) (weight/volume).

In embodiments, the *P. acnes* bacteriophage is present in an amount of about 1x10⁶, 2x10⁶, 3x10⁶, 4x10⁶, 5x10⁶, 6x10⁶, 7x10⁶, 8x10⁶, 9x10⁶, 1x10⁷, 2x10⁷, 3x10⁷, 4x10⁷, 5x10⁷, 6x10⁷, 7x10⁷, 8x10⁷, 9x10⁷, 1x10⁸, 2x10⁸, 3x10⁸, 4x10⁸, 5x10⁸, 6x10⁸, 7x10⁸, 8x10⁸, 9x10⁸, 1x10⁹, 2x10⁹, 3x10⁹, 4x10⁹, 5x10⁹, 6x10⁹, 7x10⁹, 8x10⁹, 9x10⁹, 1x10¹⁰, 2x10¹⁰, 3x10¹⁰, 4x10¹⁰, 5x10¹⁰, 6x10¹⁰, 7x10¹⁰, 8x10¹⁰, 9x10¹⁰, or 1x10¹¹ plaque forming units (pfu). In embodiments, the *P. acnes* bacteriophage is present in an amount of about 1x10⁶ to 1x10¹¹ pfu. In embodiments, the *P. acnes* bacteriophage is present in an amount of about 1x10⁶ to 1x10⁸, about 1x10⁸ to 1x10⁹, about 1x10⁹ to 1x10¹⁰, about 1x10⁹ to 1x10¹¹ or about 1x10¹⁰ to 1x10¹¹ pfu.

In embodiments, a probiotic bacterium is present in an amount of about 1x10⁶, 2x10⁶, 3x10⁶, 4x10⁶, 5x10⁶, 6x10⁶, 7x10⁶, 8x10⁶, 9x10⁶, 1x10⁷, 2x10⁷, 3x10⁷, 4x10⁷, 5x10⁷, 6x10⁷, 7x10⁷, 8x10⁷, 9x10⁷, 1x10⁸, 2x10⁸, 3x10⁸, 4x10⁸, 5x10⁸, 6x10⁸, 7x10⁸, 8x10⁸, 9x10⁸, 1x10⁹, 2x10⁹, 3x10⁹, 4x10⁹, 5x10⁹, 6x10⁹, 7x10⁹, 8x10⁹, 9x10⁹, 1x10¹⁰, 2x10¹⁰, 3x10¹⁰, 4x10¹⁰, 5x10¹⁰, 6x10¹⁰, 7x10¹⁰, 8x10¹⁰, 9x10¹⁰, or 1x10¹¹ colony forming units (cfu). In embodiments, the probiotic bacterium is present in an amount of about 1x10⁶ to 1x10¹¹ cfu. In embodiments, the probiotic bacterium is present in an amount of about 1x10⁶ to 1x10⁸, about 1x10⁸ to 1x10⁹, about 1x10⁹ to 1x10¹⁰, about 1x10⁹ to 1x10¹¹ or about 1x10¹⁰ to 1x10¹¹ cfu.

In non-claimed embodiments, the anti-acne compound is an antibiotic, a retinoid, or an alpha-hydroxy acid.

In embodiments, the composition further includes an enzyme.

In an aspect, provided herein is a combination comprising, consisting essentially of, or consisting of at least one *P. acnes* bacteriophage, at least one anti-acne compound comprising salicylic acid, and a pharmaceutically acceptable carrier, wherein each of the at least one *Propionibacterium acnes* bacteriophage, the at least one anti-acne compound, and the pharmaceutically acceptable carrier are in a single composition in a container, and wherein the genome of the *P. acnes* bacteriophage comprises a nucleotide sequence that is at least about 98%, or 99% identical to the nucleotide sequence of SEQ ID NO: 1, and a separate container comprising an additional anti-acne compound comprising benzoyl peroxide.

In embodiments, the combination further includes an enzyme.

In embodiments, the *P. acnes* bacteriophage has a linear double stranded DNA genome.

In embodiments, the *P. acnes* bacteriophage is within the bacteriophage family *Siphoviridae*.

In embodiments, the bacteriophage is a wild-type bacteriophage. In embodiments, the bacteriophage has a genome with a nucleotide sequence that is at least about 80%, 81%, 82%, 83%, 84%, 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% identical to the genomic sequence of a wild-type *P. acnes* bacteriophage. A non-limiting example of an genomic sequence for a wild-type *P. acnes* bacteriophage is as follows:

1 AGTGAAATAC CTCCCTTTTG TGGTTTTGTC TGTTTGTCGA CTTTTTGTGT TGGTGGTGAG
61 TGTTGTGCAG CCTGAGCTTC CTGAGTCTCG TGAGTGGTGT GGGGAGACGC GTCGTTGGTG
121 GCGTGTGTGG GGTGAGGATA GTCGCGCGCC GTATGTGTCT GATGAGGAGT GGTTGTTTCT
181 TATGGATGCT GCGGTGATTC ATGATTGTGT GTGGCGTGAG GGTCGCGCGG ATTTGGTGGC
241 TTCGCTTCGT GCGCATGTGA AGGCTTTTAT GGGCATGTTG GATAGGTATT CGGTTGATGT
301 GGCGTCTGGT GGCCGTGGTG GGGGTTCTGC TGTGGCGATG ATTGACCGGT ATAGGAAGCG
361 TAGGGGGGCT TGAGTAGGTG TCTGGTGTTG TTGGGTCTCA GGTTCCTCGT CACCGTGTGG
421 CTGCGGCGTA TTCGGTGTCT GCTGGGGGTG ATGCTGGGGA GCTTGGTCGT GCGTATGGGT
481 TGACGCCTGA TCCGTGGCAG CAGCAGGTGT TGGATGATTG GCTGGCTGTC GGTAGCAATG
541 GCAGGCTTGC TTCTGGTGTG TGTGGGGTGT TTGTTCCGCG GCAGAATGGC AAGAATGCTA
601 TTTTGGAGAT TGTGGAGTTG TTTAAGGCGA CTATTCAGGG TCGCCGTATT TTGCATACGG

661 CTCACGAGTT GAAGTCGGCT CGTAAGGCGT TTATGCGGTT GAGGTCGTTT TTTGAGAATG
721 AGCGGCAGTT TCCTGACTTG TATCGTATGG TGAAGTCGAT TCGTGCGACG AATGGTCAGG
781 AGGCTATTGT GTTGCATCAT CCGGATTGTG CCACTTTTGA GAAGAAGTGT GGCTGCAGCG
841 GTTGGGGTTC GGTTGAGTTT GTGGCTCGTA GCCGGGGTTC GGCTCGCGGG TTTACGGTTG
901 ATGATTTGGT GTGTGATGAG GCTCAGGAGT TGTCGGATGA GCAGTTGGAG GCTTTGCTTC
961 CTACGGTAAG TGCTGCCCCG TCTGGTGATC CGCAGCAGAT TTTCCTTGGT ACGCCGCCTG
1021 GGCCGTTGGC TGATGGTTCT GTGGTGTTGC GTTTGCGTGG GCAGGCGCTT GGTGGCGGTA
1081 AAAGGTTTGC GTGGACGGAG TTTTCGATTC CTGACGAGTC TGATCCGGAT GATGTGTCGC
1141 GGCAGTGGCG GAAGTTGGCG GGGGATACGA ATCCGGCGTT GGGGCGTCGC CTGAATTTTG
1201 GGACCGTAAG CGATGAGCAT GAGTCGATGT CTGCTGCCGG TTTTGCTCGG GAGCGGCTTG
1261 GCTGGTGGGA TCGTGGCCAG TCTGCTGCGT CTGTGGTTCC TGCTGATAAG TGGGCTCAGT
1321 CTGCGGTGGA TGAGGCGAGT CTGGTTGGCG GGAAAGTGTT TGGTGTCTCG TTTTCTCGTT
1381 CTGGGGATCG GGTTGCTTTG GCGGGTGCCG GCAAGACTGA TGCTGGGGTT CATGTTGAGG
1441 TTATTGATGG GCTGTCGGGA ACGATTGTTG ATGGTGTGGG CCGGTTGGCT GACTGGTTGG
1501 CGGTTCGTTG GGGTGATACT GACCGGATCA TGGTTGCCGG GTCTGGTGCG GTGTTGTTGC
1561 AGAAGGCGTT GACGGATCGT GGTATTCCGG GCCGTGGCGT GGTGGTTGCT GATACTGGCG
1621 TTTATGTGGA GGCTTGTCAG GCGTTTCTTG AGGGTGTCAG GTCGGGTGTG ATCAGTCATC
1681 CTCGTGCTGA TTCTCGCCGT GACATGTTGG ATATTGCTGT GAGGTCGGCT GTGCAGAAGC
1741 GTAAGGGGTC TGCGTGGGGT TGGGGTTCCT CGTTTAAGGA TGGTTCTGAG GTTCCTTTGG
1801 AGGCTGTGTC TTTGGCGTTT TTGGGGGCTA AACGTGTTCG TCGTGGCCGT CGGGAGCGTA
1861 GTGGTAGGAA GCGGGTGTCT GTGGTATGAA CTCGGATGAG TTGGCTCTGA TTGAGGGCAT
1921 GTACGATCGT ATCCAAAGGT TGTCTTCGTG GCATTGTTGT ATTGAGGGCT ACTATGAGGG
1981 CTCTAATCGG GTGCGTGACC TTGGTGTGGC TATTCCGCCG GAGTTGCAGC GTGTGCAGAC
2041 TGTGGTGTCG TGGCCTGGTA TAGCTGTGGA TGCTTTGGAG GAGCGTCTGG ATTGGCTTGG
2101 CTGGACTAAT GGTGACGGCT ACGGCCTTGA TGGTGTGTAT GCTGCGAATC GGCTTGCTAC
2161 GGCGTCGTGT GATGTGCATT TGGATGCGCT GATTTTTGGG TTGTCGTTTG TTGCGATCAT
2221 TCCTCATGGT GATGGTACGG TGTCGGTTCG TCCGCAGTCA CCAAAGAATT GTACGGGCAA
2281 GTTTTCGGCT GACGGGTCTC GTTTGGATGC GGGTTTGGTG GTGCAGCAGA CGTGTGATCC
2341 TGAGGTTGTT GAGGCTGAGC TTTTGCTTCC TGATGTGATT GTTCAGGTGG AGCGGCGGGG
2401 TTCGCGTGAA TGGGTTGAGG TGGATCGTAT ACCGAATGTG TTGGGTGCGG TTCCGTTGGT
2461 GCCTATTGTG AATCGTCGCC GTACTTCTAG GATTGATGGC CGTTCGGAGA TTACGAGGTC
2521 TATTAGGGCT TACACGGATG AGGCTGTGCG CACACTGTTG GGGCAGTCTG TGAATCGTGA
2581 TTTTTATGCG TATCCTCAGC GTTGGGTGAC TGGCGTGAGC GCGGATGAGT TTTCGCAGCC
2641 TGGCTGGGTC CTGTCGATGG CTTCTGTGTG GGCTGTGGAT AAGGATGATG ACGGTGACAC
2701 TCCGAATGTG GGGTCGTTTC CTGTCAATAG TCCTACACCG TATTCGGATC AGATGAGACT
2761 GTTGGCGCAG TTGACTGCGG GTGAGGCGGC TGTTCCGGAA CGCTATTTCG GGTTTATCAC
2821 GTCTAACCCA CCTAGTGGGG AGGCTTTGGC TGCCGAGGAA TCTCGGCTTG TGAAGCGTGC

2881 TGAGCGGCGT CAAACGTCGT TTGGTCAGGG TTGGCTGTCG GTTGGTTTTT TGGCTGCCAA
2941 GGCGTTGGAT TCTCGTGTTG ATGAGGCCGA TTTTTTTGGT GATGTTGGTT TGCGTTGGCG
3001 TGATGCTTCG ACGCCTACCC GGGCGGCTAC GGCTGATGCT GTGACGAAGC TTGTTGGTGC
3061 CGGTATTTTG CCTGCTGATT CTCGTACGGT GTTGGAGATG TTGGGGCTTG ATGATGTGCA
3121 GGTTGAGGCT GTGATGCGTC ATCGTGCTGA GTCGTCTGAC CCGTTGGCGG TGCTTGCTGG
3181 GGCTATATCG CGTCAAACTA ACGAGGTATG ATAGGCGATG GCTTCGGGGG TTGAGGCGAG
3241 GCTTGCGGCG ACTGAGTATC AGCGTGAGGC GGTCAGGTTT GCTGGGAAGT ATGCGGGCTA
3301 TTATTCTGAG CTTGGTCGTT TGTGGCGTGC CGGCAGGATG AGTGACACGC AGTATGTGCG
3361 TTTGTGTGTG GAGTTGGAGC GTGCCGGCCA TGATGGTTCG GCATCGTTGG CTGCCAGGTT
3421 TGTGTCGGAT TTTCGCCGGT TGAATGGTGT GGATCCGGGT TTGATTGTGT ATGACGAGTT
3481 TGATGCTGCG GCGGCTTTGG CTAGGTCTAT TTCGACCACG AAGATTCTTG AGAGTGACCC
3541 GGATAGGGCG AATGACACGA TTGATGCGAT GGCGGCGGGT TTTGATCGGG CTGTTATGAA
3601 TGCTGGCCGT GACACGGTTG AGTGGTCTGC GGGTGCGCAG GGTAGGTCGT GGCGTCGGGT
3661 GACGGATGGT GATCCGTGTG CTTTTTGTGC CATGTTGGCT ACGAGGTCGG ATTATACGAC
3721 AAAAGAGAGG GCACTTACTA CTGGACATAC TCGGCGTCAT AAGCGTGGTG GTAAGCGTCC
3781 GTTTGGTTCG AAGTATCATG ATCATTGTGG TTGTACGGTG GTTGAGGTTG TTGGCCCTTG
3841 GGAACCAAAT AGGGCTGATG CCGAGTATCA GAGGACGTAT GAGAAGGCCT GTGAGTGGGT
3901 TGATGATCAT GGGTTGCAGC AATCGCCTGG CAATATTTTG AAGGCTATGC GTACTGTTGG
3961 CGACATGAGA TAATTTGATG TGGTTTCCGG TTGTGCGCCG CCGGTTATTG GTGCACAGGG
4021 TTGTCTCCCG CACGGGGGTC AACAATATTG TGTTGTTTTC CGCAAGGAGT GTAGGGTTAG
4081 GCTATGGCCG ATCAGAGTGT TGAGGAACAG AATGTTGACA ATGATGTTGT GGAGTCCGGA
4141 AAGGATAACG GCATTGTTGA TACAGTAAAA GACGATGGCG GGCAGGAGGT AGCCGACAAT
4201 CAGTTGAAGA ATGAAGGCGA GGGTAAATCG CCGGGGACTG ATTGGAAGGC TGAGGCCCGT
4261 AAGTGGGAGT CTCGTGCTAA AAGTAATTTT GCCGAGTTGG AGAAGCTTCG CGCCTCGGAT
4321 GGTGATGCGG GGTCTACGAT TGATGAGCTT CGCCGCAAGA ATGAGGAACT CGAAGACCGG
4381 ATCAATGGGT TTGTTCTTGA GGGTGTGAAG CGCGAGGTGG CTGCCGAGTG TGGCCTGTCG
4441 GGTGATGCTG TCGCTTTCTT GTCGGGTGGC GATAAGGAGT CGCTTGCCGA GTCTGCGAAA
4501 GCTTTGAAGG GTTTGATCGA CCATAGTAGT GGTGGCGCGG GTGTGCGCCG TCTTGCGGGG
4561 AGTGCCCCCG TTGATGATGT TAAACGACGT GAGGGTGTCG CGTTTGTGGA TGCTCTTGTC
4621 AATAATTCTA GGAGATGATT TGTGATGGCT GACGATTTTC TTTCTGCAGG GAAGCTTGAG
4681 CTTCCTGGTT CTATGATTGG TGCGGTTCGT GACCGTGCTA TCGATTCTGG TGTTTTGGCG
4741 AAGCTTTCGC CGGAGCAGCC GACTATTTTC GGGCCTGTGA AGGGTGCCGT GTTTAGTGGT
4801 GTTCCTCGCG CCAAGATTGT TGGTGAGGGC GAGGTTAAGC CTTCCGCGTC TGTTGATGTT
4861 TCGGCGTTTA CTGCGCAGCC TATCAAGGTT GTGACTCAGC AGCGTGTCTC GGATGAGTTT
4921 ATGTGGGCTG ATGCTGATTA CCGTCTGGGT GTGCTTCAGG ATCTGATTTC CCCGGCTCTT
4981 GGTGCTTCGA TTGGTCGCGC CGTGGATCTG ATTGCTTTCC ATGGTATTGA TCCTGCCACT
5041 GGTAAAGCGG CTTCCGCTGT GCATACTTCG CTGAATAAGA CGAAGAATAT TGTTGATGCC

5101 ACGGATTCTG CTACGGCTGA TCTTGTTAAG GCTGTCGGCC TGATTGCTGG TGCTGGTTTG
5161 CAGGTTCCTA ACGGGGTTGC TTTGGATCCG GCGTTCTCGT TTGCGCTGTC TACTGAGGTG
5221 TATCCGAAGG GGTCTCCGCT TGCCGGTCAG CCTATGTATC CTGCCGCCGG GTTTGCCGGT
5281 TTGGATAATT GGCGCGGGCT GAATGTTGGT GCTTCTTCGA CTGTTTCTGG CGCCCCGGAG
5341 ATGTCGCCTG CCTCTGGCGT TAAGGCTATT GTTGGTGATT TCTCTCGTGT TCATTGGGGT
5401 TTCCAGCGTA ACTTCCCGAT CGAGCTTATC GAGTATGGTG ACCCGGATCA GACTGGGCGT
5461 GACTTGAAGG GCCATAATGA GGTTATGGTT CGTGCCGAGG CTGTCCTGTA TGTTGCGATT
5521 GAGTCGCTTG ATTCGTTTGC TGTTGTGAAG GAGAAGGCTG CCCCGAAGCC TAATCCGCCG
5581 GCCGAGAACT GATTCATTTG TTGCGGTGAT GTTTTCTATG TGCAGGGGGT GGTGTTGATG
5641 GGTATCATTT TGAAGCCTGA GGATATTGAG CCTTTCGCCG ATATTCCTAG AGAGAAGCTT
5701 GAGGCGATGA TTGCCGATGT GGAGGCTGTG GCTGTCAGTG TCGCCCCCTG TATCGCTAAA
5761 CCGGATTTCA AATACAAGGA TGCCGCTAAG GCTATTCTGC GCAGGGCCCT GTTGCGCTGG
5821 AATGATACCG GGGTTTCGGG TCAGGTGCAG TACGAGTCTG CGGGCCCGTT TGCTCAGACT
5881 ACACGGTCGA ATACTCCCAC GAATTTGTTG TGGCCTTCTG AGATTGCCGC GTTGAAGAAG
5941 TTGTGTGAGG GTGATGGTGG GGCTGGTAAA GCGTTCACTA TTACACCGAC CATGAGGAGT
6001 AGTGTGAATC ATTCTGAGGT GTGTTCCACG GTGTGGGGTG AGGGTTGCTC GTGCGGATCT
6061 GATATTAACG GCTATGCTGG CCCTTTGTGG GAGATATGAT ATGACCGGTT TTCCTTACGG
6121 TGAAACGGTT GTGATGCTTC AACCGACTGT TCGTGTCGAT GATCTTGGCG ACAAGGTGGA
6181 AGACTGGTCT AAGCCTGTCG AGACTGTGTA CCATAACGTG GCCATCTATG CTTCCGTTTC
6241 GCAGGAGGAT GAGGCTGCCG GCCGTGACTC TGACTATGAG CATTGGTCGA TGCTTTTCAA
6301 GCAGCCTGTT GTGGGTGCCG GTTATCGTTG CCGGTGGCGT ATTCGGGGTG TGGTTTGGGA
6361 GGCGGACGGG TCTCCTATCG TGTGGCATCA TCCGATGTCT GGTTGGGATG CTGGTACGCA
6421 GGTTAATGTG AAGCGTAAGA AGGGCTGATG GGTTGTGGCT CAGGATGTGA ATGTGAAGCT
6481 GAACTTGCCG GGTATTCGTG AGGTGTTGAA GTCTTCTGGG GTGCAGTCGA TGTTGGCTGA
6541 GCGTGGCGAG CGGGTGAGGC GTGCGGCTTC GGCGAATGTT GGCGGTAATG CTTTTGATAG
6601 GGCCCAATAC CGTAGTGGTT TGTCGTCGGA GGTGCAGGTT CACCGTGTGG AGGCTGTGGC
6661 GAGGATTGGC ACCACCTATA AGGGTGGGAA GCGTATTGAG GCGAAGCATG GCACGTTGGC
6721 GAGGTCGATT GGGGCTGCGT CGTGATCGTT TACGGTGATC CGCGTGTGTG GGCTAAACGT
6781 GTGCTCAAGG ATGATGGCTG GCTGTCCGAT ATACCCTGTG TGGGGACGGT GCCTGACGAT
6841 TTCAGCGGTG ACCTGATTTG GTTGGCGTTG GATGGCGGCC CACAGTTGCA TGTTCGCGAG
6901 CAGGTGTTTT TGCGGGTGAA CGTGTTTTCT GATATGCCTG ATCGTGCCAT GTCGCTAGCC
6961 AGGCGGGTTG AGGCTGTCCT TGTAGACGGT GTGGACGGTG ACCCGGTGGT GTTTTGTCGA
7021 CGGTCTACTG GCCCTGATTT GCTGGTTGAT GGTGCACGTT TTGATGTGTA TTCGCTGTTT
7081 GAGCTGATAT GCAGGCCTGT CGAATCCGAG TAAACGTTTT GTTTTGATAT TGTTGTTTGT
7141 TTTTTGTTTG ATATTGTTTT TGGGGGTTAT GATGGCTGGA ACACGTAAAG CGTCTAATGT
7201 TCGTTCCGCG GTTACGGGTG ACGTCTATAT TGGTAAAGCT CATGCCGGTG ACACTATTGA
7261 TGGTGTGAAG ACGGTTCCTG ACGGGCTTAC AGCTTTAGGG TATCTGTCTG ATGACGGGTT

7321 TAAGATTAAA CCGGAGCGTA AAACGGATGA TTTGAAGGCT TGGCAGAATG CGGATGTTGT
7381 TCGCACTGTG GCTACGGAAT CGTCTATCGA GATTTCTTTC CAGCTGATCG AGTCTAAGAA
7441 GGAGGTTATC GAGCTGTTTT GGCAGTCGAA GGTTACTGCC GGAGCCGATT CGGGTTCGTT
7501 CGATATTTCT CCTGGTGCCA CGACGGGTGT TCATGCCCTG TTGATGGATA TTGTTGATGG
7561 CGATCAGGTT ATTCGCTACT ATTTCCCTGA GGTTGAGTTG ATCGATCGTG ACGAGATTAA
7621 GGGTAAGAAT GGCGAGGTGT ATGGGTATGG TGTGACGTTG AAGGCGTATC CTGCCCAGAT
7681 TAATAAGAAG GGTGATGCGG TGTCTGGTCG GGGGTGGATG ACGGCTTTAA AAGCTGATAC
7741 TCCTCCGACT CCTCCTCCGG CCCCGAATCC TCCGAAGCCT GAGCCGGATC CGAATCCGCC
7801 GTCTAATAAC TGATACACAT AGTTTGAGGG ATTGTTGATA GATGAGTGAC ACGGGTTACA
7861 CGTTGAAGAT TGGTGACCGT AGCTGGGTGT TGGCGGATGC GGAGGAGACG GCTCAGGCTG
7921 TTCCTGCCCG CGTTTTCCGT CGTGCTGCTA AGATTGCCCA GTCGGGTGAG TCTGCGGATT
7981 TCGCCCAGGT TGAGGTGATG TTTTCTATGT TGGAGGCTGC CGCCCCGGCT GACGCGGTGG
8041 AGGCCCTGGA GGGGCTTCCT ATGGTTCGTG TGGCCGAGAT TTTCCGCCAG TGGATGGAAT
8101 ACAAGCCTGA CGGTAAGGGT GCCTCGCTGG GGGAATAGTT TGGCTCCACG GCCTGATTGA
8161 TGATTATCGT GGGGCCATCG AATACGATTT CCGCACCAAG TTTGGTGTTT CTGTTTATAG
8221 TGTTGGTGGC CCGCAGATGT GTTGGGGTGA GGCTGTCCGG CTGGCTGGCG TGTTGTGTAC
8281 CGATACGTCT AGCCAGTTGG CGGCCCACCT GAATGGTTGG AAGCGCCCGT TTGAGTGGTG
8341 CGAGTGGGCT GTGTTGGACA TGCTGGATCA TTACAGGTCT GCTAATAGTG AGGGGCAGCC
8401 GGAGCCTGTG GCGAGGCCTA CGGATGAGCG TAGGGCCCGG TTTACGTCTG GGCAGGTGGA
8461 CGATATTTTG GCGCGTGTTC GTGCTGGTGG CGGGGTGTCT CGCGAGATTA ATATTATGGG
8521 GTGAATAGTG TATGTCTGGT GAGATTGCTT CCGCATATGT GTCGTTGTAT ACGAAGATGC
8581 CTGGTTTGAA GGCGGATGTT GGTAAACAGC TTTCTGGGGT GATGCCTGCT GAGGGTCAGC
8641 GTTCGGGTAG TTTGTTTGCT AAGGGAATGA AGTTGGCTCT TGGTGGTGCG GCGATGATGG
8701 GTGCCATCAA TGTTGCTAAG AAGGGCCTCA AGTCGATTTA TGATGTGACT ATTGGTGGCG
8761 GTATTGCTAG GGCGATGGCT ATTGATGAGG CTCAGGCTAA GTTGACTGGT TTGGGTCATA
8821 CGTCTTCTGA CACGTCTTCG ATTATGAATT CGGCTATTGA GGCTGTTACT GGTACGTCGT
8881 ATGCGTTGGG GGATGCGGCG TCTACGGCTG CGGCGTTGTC TGCTTCGGGT GTGAAGTCTG
8941 GCGGGCAGAT GACGGATGTG TTGAAGACTG TCGCCGATGT GTCTTATATT TCGGGTAAGT
9001 CGTTTCAGGA TACGGGCGCT ATTTTTACGT CTGTGATGGC TCGCGGTAAG TTGCAGGGCG
9061 ATGACATGTT GCAGCTTACT ATGGCGGGTG TTCCTGTCCT GTCTTTGCTT GCCAGGCAGA
9121 CTGGTAAAAC GTCTGCTGAG GTGTCGCAGA TGGTGTCAAA GGGGCAGATT GATTTTAACA
9181 CGTTTGCGGC TGCGATGAAG CTTGGCATGG GTGGTGCTGC GCAGGCGTCT GGTAAGACGT
9241 TTGAGGGCGC TATGAAGAAT GTTAAGGGCG CCCTGGGTTA TCTTGGTGCT ACGGCTATGG
9301 CCCCGTTTCT TAACGGGTTG CGGCAGATTT TTGTTGCGTT GAATCCGGTT ATCAAGTCTG
9361 TCACGGATTC CGTGAAGCCG ATGTTTGCTG CCGTCGATGC TGGTATTCAG CGTATGATGC
9421 CGTCTATTTT GGCGTGGATT AACCGTATGC CGGCTATGAT CACTCGAATG AATGCACAGA
9481 TGCGCGCCAA GGTGGAGCAG TTGAAGGGCG TTTTTGCAAG GTTGCATTTG CCTGTTCCTA

9541 AGGTGAATTT GGGTGCCATG TTTGCTGGCG GCACCGCAGT GTTCGGTATT GTTGCTGCGG
9601 GTGTTGGGAA GCTTGTCGCG GGGTTTGCCC CGTTGGCGGT GTCGTTGAAG AATCTGTTGC
9661 CGTCGTTTGG TGCTTTGAGG GGTGCCGCCG GGGGGCTTGG TGGCGTGTTT CGCGCCTTGG
9721 GTGGCCCTGT TGGTATTGTG ATCGGCTTGT TTGCTGCCAT GTTTGCTACG AACGCCCAGT
9781 TCCGTGCCGC TGTTATGCAG CTTGTGGGGG TGGTTGGCCG GGCTTTGGGG CAGATTATGG
9841 TCGCCTTGCA GCCATTGTTC GGGATTGTTG CTGGCGTGGT TGCCAGGTTG GCTCCCGTTT
9901 TTGGCCAGAT TATTGGTATG GTTGCTGGTT TGGCTGCCCG GCTGGTGCCT GTTATTGGTA
9961 TGCTTATTGC CCGGCTGGTT CCTGTTATCA CCCAGATTAT TGGTATGGTA ACCCAGGTTG
10021 CTGCCATGTT GTTGCCTATG CTGATGCCGG TTATTCAGGC TGTTGTTGCT GTGATACGGC
10081 AGGTTATTGG TGTGGTCATG CAGTTGATAC CTGTTTTGAT GCCGGTTGTG CAGCAGATTT
10141 TGGGTGCTGT CATGTCTGTT TTGCCGCCGA TTGTTGGTTT GATACGGTCG CTGATACCGG
10201 TGATCATGTC GATTATGCGT GTGGTGGTGC AGGTTGTTGG TGCCGTGCTA CAGGTGGTGG
10261 CCCGTATTAT TCCGGTTGTT ATGCCGATTT ATGTTTCGGT GATTGGATTC ATTGCCAAGA
10321 TTTATGCTGC GGTTATCGTT TTTGAGGCTA AGGTTATTGG CGCTATTCTT CGTACTATTA
10381 CGTGGATTGT GAATCATTCA GTGTCTGGCG TGAGGTCTAT GGGCACGGCC ATCCAGAATG
10441 GCTGGAATCA TATCAAATCG TTTACGTCGG CGTTTATTAA CGGTTTCAAG TCGATCATTT
10501 CTGCCGGTGT TGCCGCGGTT GTGGGGTTTT TTACGCGGCT TGGTTTGTCG GTTGCCTCCC
10561 ATGTGAGGTC TGGTTTTAAC GCGGCCCGTG GTGCTGTTTC TTCTGCGATG AATGCTATTC
10621 GGAGTGTTGT GTCTTCGGTG GCGTCTGCTG TTGGCGGGTT TTTCGGGTCG ATGGCGTCTA
10681 GGGTTCGTAG TGGTGCTGTG CGCGGGTTTA ATGGTGCCCG GAGTGCGGCT TCTTCTGCTA
10741 TGCATGCTAT GGGGTCTGCG GTGTCTAACG GTGTGCATGG TGTGCTGGGG TTTTTCCGGA
10801 ATTTGCCTGG CAATATTAGG GGCGCCTTGG GTAGTATGGG GTCCCTGTTG GTGTCGGCTG
10861 GCCGTGATGT GGTGTCTGGT TTGGGTAACG GTATCCGGAA TGCTTTGAGT GGCCTGTTGG
10921 ATACGGTGCG TAACATGGGT TCCCAGATTG CGAACGCGGC GAAGTCTGCG CTGGGTATTC
10981 ATTCCCCGTC TCGGGTGTTT CGTGACGAGG TTGGCCGTCA GGTTGTTGCC GGTTTGGCTG
11041 AGGGGATCAC CGGGAATGCT GGTTTGGCGT TGGATGCGAT GTCTGGTGTG GCTGGCCGTC
11101 TTCCGGATGC TGTGGATGCC CGGTTTGGTG TGCGATCGTC TGTGGGCTCG TTTACCCCGT
11161 ACGACCGGTA TCGGCGTGCG AACGAGAAGA GTGTTGTGGT GAATGTGAAC GGACCCACGT
11221 ATGGGGATCC TGCCGAGTTT GCGAAGCGGA TTGAGCGTCA GCAGCGTGAC GCTTTGAATG
11281 CGTTGGCTTA CGTGTGATCG AGGGGGTGTT GTGCATGTTT ATTCCTGACC CGTCTGATCG
11341 TGCCGGTTTG ACTGTGGATT GGACTATGTT TCCGTTGGTG GGTAATGCTC CGGAGCGTGT
11401 GCTTCATTTG ACGGATTATA CGGGGTCGTC TCCGGTCATG TTGTTGAATG ATTCGTTGCG
11461 CGGCCTGGGT ATGCCTGAGG TGGAGCAGTT TTCTCAAACG CATGTTGGTG TGCATGGTTC
11521 GGAGTGGCGC GGGTTTAATG TGAAGCCTCG CGAGGTGACT TTGCCGGTGT TGGTGTCGGG
11581 TGTTGACCCG GATCCGGTGG GCGGGTTTCG TGACGGTTTT TTGAAGGCGT ATGACGCGTT
11641 GTGGTCTGCG TTTCCTCCGG GCGAGGTGGG GGAGTTGTCT GTGAAGACTC CTGCCGGTCG
11701 TGAGCGTGTG TTGAAGTGCC GGTTTGATTC GGCTGATGAC ACGTTTACGG TTGATCCGGT

11761 GAACCGTGGC TATGCGCGCT ATCTGTTGCA TTTGACAGCT TATGATCCGT TTTGGTATGG
11821 GGATGAGCAA AAGTTTCGTT TTAGTAACGC GAAGTTGCAG GATTGGTTGG GTGGCGGCCC
11881 TGTCGGCAAG AAGGGTACCG CGTTTCCTGT GGTGTTAACA CCGGGTGTGG GCTCGGGCTG
11941 GGATAACCTG TCTAATAAGG GTGATGTGCC TGCGTGGCCT GTGATTCGTG TTGAGGGTCC
12001 TTTGGAGTCG TGGTCTGTGC AGATTGATGG TTTGCGTGTG TCTTCGGACT ATCCGGTCGA
12061 GGAGTTTGAT TGGATCACTA TTGATACGGA TCCTCGCCAG CAGTCTGCGT TGTTGAACGG
12121 GTTTGAGGAT GTGATGGATC GTTTGACAGA GTGGGAGTTT GCGCCTATCC CGCCTGGCGG
12181 TTCTAAGAGT GTGAATATTG AGATGGTTGG TTTGGGTGCT ATTGTTGTGT CGGTGCAGTA
12241 CAGGTTTTTG AGGGCTTGGT GAATAGTTGA TGGCTGGTCT TGTTCCGCAT GTAACATTGT
12301 TTACACCTGA TTATCGCCGT GTGGCGCCTA TCAATTTTTT TGAGTCGTTG AAGTTGTCGT
12361 TGAAGTGGAA TGGTTTGTCG ACTTTGGAGT TGGTGGTGTC GGGGGATCAT TCGAGGCTTG
12421 ACGGGTTGAC GAAGCCGGGT GCGCGGCTGG TTGTTGATTA TGGTGGTGGC CAGATTTTTT
12481 CTGGGCCTGT GCGTAAAGTG CATGGTGTGG GTCCGTGGCG TTCTTCCCGT GTGACTATAA
12541 CGTGTGAGGA TGATATTCGG CTGTTGTGGC GTATGTTGAT GTGGCCTGTG AATTATCGTC
12601 CTGGTTTGGT TGGTATGGAG TGGCGTGCGG ACAGGGATTA TGCCCACTAT TCGGGTGCGG
12661 CTGAGTCGGT TGCTAAGCAG GTGTTGGGGG ATAATGCTTG GCGTTTTCCG CCTGGTTTGT
12721 TTATGAACGA TGATGAGAGT CGTGGCCGCT ATATTAAGGA TTTTCAGGTG CGGTTTCACG
12781 TGTTTGCCGA TAAGTTGTTG CCGGTGTTGT CGTGGGCTCG GATGACTGTC ACGGTGAACC
12841 AGTTTGAGAA TGCGAAGTTT GATCAGCGTG GTTTGTTGTT TGATTGTGTG CCTGCTGTGA
12901 CCCGGACGCA TGTGTTGACT GCCGAGTCTG GTTCGATTGT GTCGTGGGAG TATGTGCGTG
12961 ACGCCCCGAA GGCTACTTCG GTGGTGGTTG GTGGCCGCGG CGAGGGCAAA GATCGGCTGT
13021 TTTGCGAGGA TGTTGATTCG ATGGCCGAGG ATGACTGGTT TGATCGTGTC GAGGTGTTTA
13081 AGGATGCCCG TAACACGGAT TCCGAGAATG TGCATCTTAT TGATGAGGCT GAGCGGGTGT
13141 TGTCCGAGTC GGGGGCTACG TCGGGGTTTA AGATCGAGTT GGCTGAGTCG GATGTGTTGC
13201 GGTTTGGGCC TGGCCGCCTG ATGCCGGGTG ATCTTATCTA TGTGGATGTG GGCTCGGGGC
13261 CTATTGCGGA GATTGTGCGC CAGATTGATG TGGAGTGTGA TTCGCCTGGT GATGGGTGGA
13321 CGAAGGTGAC TCCGGTTGCT GGGGATTATG AGGATAATCC GTCGGCGCTG TTGGCTCGCC
13381 GTGTGGCTGG TTTGGCTGCG GGTGTGCGGG ATTTGCAAAA ATTCTAATTG TTAGGGGTTT
13441 GTTGTGGGTA TTGTGTGTAA AGGGTTTGAT GGTGTGTTGA CCGAGTATGA TTGGGCTCAA
13501 ATGTCTGGTC TGATGGGTAA TATGCCGTCC GTGAAAGGGC CGGATGATTT TCGTGTCGGC
13561 ACTACGATTC AGGGTTCCAC GGTGTTGTGT GAGGTCCTGC CGGGGCAGGC TTGGGCTCAC
13621 GGGGTGATGT GCACGTCGAA TGCTGTTGAG ACGGTGACAG GTCAGCTTCC GGGCCCGGGT
13681 GAGACCCGCT ACGACTATGT TGTCCTGTCG CGGGATTGGC AGGAGAATAC GGCCAAGTTG
13741 GAGATTGTTC CTGGGGGGCG TGCGGAGCGT GCCCGTGACG TGTTGCGTGC GGAGCCTGGC
13801 GTGTACCATC AGCAGTTGTT GGCTACTTTG GTGGTGTCGT CTAACGGGTT GCAGCAGCAG
13861 CTTGACAGGA GGGCTATAGC GGCCCGTGTG GCGTTTGGGG AGTCTACTGC ATGTGATCCT
13921 ACCCCTGTGG AGGGTGACCG GGTGATGGTG CCTTCTGGGG CTGTGTTGGC TAATCATGCT

13981 AACGAGTGGA TGCTGTTGTC TCCGCGGATT GAGACGGGCA CTAAGTCGAT CATGTTTGGC
14041 GGGTCTGCTG TGTATGCTTA CACGATTCCG TTTGATCGCC AGTTTGCTAG TCCGCCTGTT
14101 GTGGTGGCGT CTATGGCTAC GGCGGCTGGG GGCACGACCC AGATTGATGT GAAAGCCTAC
14161 AATGTGACTG CCCAAAATTT TAGTTTGGCG TTTATTACGA ATGATGGTTC GAAGCCGAAT
14221 GGTGTGCCTG CGGTGGCTAA TTGGATTGCT GTCGGCGTGT GACTGTACAG GTGTTGTGGC
14281 GGATGGTGTG ATGTTGGGGG GCTGTGGTGT CGTGGTTTAC TCCTGCACTG GTGGCCTCTA
14341 TTTGTACCGC GTTGGCCACG GTTTTGGGTT CTGTTCAGGC TGTCACGTCT AAATCTAGGA
14401 GGCGTTTGCG CCGCCTGTCG GCGCAGGTGG ATGCGATGGA AGAGTATACG TGGGGTGTGC
14461 GGCGCGAGGT GCGAAGGTTT AACGCCGGGC TTCCTGACGA GGTGGAGCCT ATGCATCTCC
14521 CTGATTTGCC CGAGTTTTTG AAAGATACTG TTGATGGTGG AGGTGAGTAG GGTTGAGGGA
14581 GTTGGAGGAG GAGAAGCGGC AGCGCCGCAA TTTTGAGAAG GCTTCACTGG TGTTGCTGTT
14641 TTTGTCGCTT GTGTTATTGG CTGTGGTTGC TGCGGGTGCT TTGCGTTTCG GGCTGTATC
14701 CTCTGAGCGG GATTCGGAGC AGGCGAGGGC CCAGTCGAAT GGTACAGCCG CCAAGGGTTT
14761 AGCCAGCAGT GTGCGGCAGG TGTGTGCTCA GGGTGGACGG GAGTCTGTGC GGCTTCACCA
14821 GTCTGGTTTG TGTGTGGATG CTCAGCGTGT TGAGCGTAGT GTGCAGGGTG TGCCGGGTCC
14881 TGCCGGTGAG CGCGGCCCGC AAGGCCCGGC AGGTGTGGAC GGCCGGGATG GTGTTAATGG
14941 TTCGGCTGGG CTGGTTGGCC CTGTGGGTCC GCAGGGGTCC CCGGGTTTGA ATGGTGTGAA
15001 AGGTCCTGAC GGGTTGCCTG GCGCTAACGG TTCGGATGGC CGTGATGGTG TGGACGGTGT
15061 GAACGGCAAT GATGGCGCTG ATGGTCGGGA TGGTTCGGCC GGTGAGCGCG GTGATGTGGG
15121 CCCCTCAGGT CCTGCCGGCC CGCAAGGTGC ACAGGGTGAA CGGGGTGAGC GCGGCCCCGC
15181 CGGTGCGAAT GGCACGAATG GCAAGGACGG TAAGGATGGT GCCGACGGCC GTGATGGGCG
15241 TTCGGTTGTG TCTGTGTACT GTTTCGGTGG CCTGCCAGGG TGTGAAACCA TCACCTGTGG
15301 TTACCGTGTC ATCCCGTAAA TAGAAGAAGA GGGAAGGGTG TTACTAGTGT TGATTGTGGT
15361 TTTTGGTGGT GGTGTGTGGT GAGATACATT CCTGCAGCGC ATCACTCTGC CGGCTCTAAT
15421 AATCCGGTGA ACAGGGTTGT GATTCATGCA ACATGCCCGG ATGTGGGGTT TCCGTCCGCC
15481 TCACGTAAGG GGCGGGCGGT GTCTACAGCA AACTATTTCG CTTCCCCATC GTCTGGTGGT
15541 TCGGCGCATT ATGTGTGTGA TATTGGGGAG ACGGTGCAAT GCTTGTCGGA GTCTACGATT
15601 GGTTGGCATG CCCCGCCGAA TCCGCATTCT TTGGGTATCG AGATTTGCGC GGATGGGGGT
15661 TCGCATGCCT CGTTCCGTGT GCCGGGGCAT GCTTACACTC GGGAGCAGTG GCTTGATCCG
15721 CAGGTGTGGC CTGCCGTTGA GAGGGCGGCG GTGCTGTGTA GACGTTTGTG TGACAAATAT
15781 AATGTTCCGA AAAGGAAACT GTCGGCTGCC GATTTGAAGG CTGGCAGGCG GGGTGTGTGT
15841 GGCCATGTGG ATGTTACGGA TGCGTGGCAT CAGTCGGATC ATGACGATCC TGGGCCGTGG
15901 TTTCCGTGGG ACAAATTTAT GGCCGTCGTC AACGGCGGCA GTGGAGATAG TGGGGAGTTA
15961 ACTGTGGCTG ATGTGAAAGC CTTGCATGAT CAGATTAAAC AATTGTCTGC TCAGCTTACT
16021 GGTTCGGTGA ATAAGCTGCA CCATGATGTT GGTGTGGTTC AGGTTCAGAA TGGTGATTTG
16081 GGTAAACGTG TTGATGCCTT GTCGTGGGTG AAGAATCCTG TGACGGGGAA GCTGTGGCGC
16141 ACTAAGGATG CCCTGTGGAG TGTCTGGTAT TACGTGTTGG AGTGTCGTAG CCGTCTTGAC

16201 AGGCTCGAGT CTGCTGTCAA CGATTTGAAA AAGTGATGGT GGTTTGTTGT GGGTAAACAG
16261 TTTTGGTTAG GTTTGCTAGA GCGGGCGGCT AAGACTTTTG TGCAAACGTT TGTTGCTGTG
16321 TTGGGGGTGA CGGCGGGTGT CACGTATACG GCGGAGTCGT TTCGTGGTTT GCCGTGGGAG
16381 TCTGCGTTGA TTACGGCTAC GGTTGCTGCG GTCCTGTCGG TGGCTACCTC GTTTGGTAGC
16441 CCGTCGTTTG TGGCTGGTAA GCCGAAAACC ACGCCTGTGG ATGCGGGTTT GGTTCCGCCG
16501 GATGATCCCG GAATAGTGGA GCCTCACATG GTGGATGTGT CGGATCCTGG CATGATCGAG
16561 CCTGCAGATG ATGTGGATCT TGGTGTAGGC TATGTGCCGA AACATGCTGC CGAGTCGGAG
16621 GTTGGCACGG TAGAGTCGAC TGTTGCATAA GTGAATATAG ATGTGTGCCC CAGCGGTGCT
16681 GCCACGATTG TGTGGTGGTT GCCGCTGGGG CACTATTTTT GTATATTGCG GTGTGGCTAT
16741 GATTCGTTGC TGTCGATGGT GTCTTCGAGC ATCTGGTACA GGTGGAGGCA GGTAGAGATA
16801 GTTTCGCTGG CCTGGTCGAG AACGTTCCGG CCGATAACAT TTTTGTTGTT GTCGCGGTGG
16861 CGGATGATAG ACCACATGAT CTCGTCGGCT GCCGCCTGCA ATAGTTTTGC CTGGTATGCG
16921 ATTCCAGCGA GCCAGTCTAG TGCTTCCTGG CTTGCATAGG GTGTCTGGTC CTCGCTGTTG
16981 CTTGTGGGGT GTCCTGCACT GTCGCATAGC CACAGGATTT CGCTGCACTC GTCTAGCGTG
17041 TCCTGGTCTA TAGCGAGATC GTCGAGGCTG ACATTGTTGA CGGTAAGGTT CACGTTGTCG
17101 AGGGAGATGG GTACACCGTA CTGGTTTTCG ACACCGTCAA CAATGTTTTC CAATTGCTGC
17161 ATGTTGGTGG GCTGTTGTTG GACGATACGG TGTATCGCTG TGTTGAGGGT GGTGTAGGTG
17221 ATATTGTGTG TGTTGTTCAT CGTGTTATGC CATTCCTTCG TTATCGTCTG GCCTGTAGTA
17281 TGTGCTGTTT GCGTACTCGG TTAACGTCAT CAGTGTTTGG TCTGCCCACT GTTTCACAGT
17341 CTGCCTTGTC ACTCCGAGTC GTTGGGCGGC TGTGGCGTAG GTTTGGTCAT ACCCGTATAC
17401 TTCCCTGAAT GCTGCCAACC GTGCCAAATG TTTTCGCTGT TTGGATGGCT GGCAGGCGAG
17461 GGTGTAGTCG TCGATGGCTA GCTGTAGATC GATCATGGTG GCAATGTTGT TGCCGTGGTG
17521 TTGTGGCGCG GTTGGTGGGG GTGGCATTCC TGGCTCCACA CTGGGTTTCC ATGGGCCTCC
17581 GTTCCAGATC CATTGGGCGG CTTGGATGAT GTCTGCGGTG GTGTAGGTTC GGTTCACTGG
17641 TCATCCCCTG AACAGGTTGT CTGGGTTGCT GGTGCGGATT GTGTCGAATC GTCCGACGCA
17701 GTGGCAGTAG TCGTACATGA GTTTGATAAT GTGTTGGTGG TCTCCCAAAT AGGTGTTTCC
17761 GCTGATGCTG TAGGTGGCTG TGCCGTCTTT ACTAATAGTG TATTTGGCGG TGATGGTTTC
17821 GGGGTTTTCG GTGTCGGTGA TGATGGCTGT GGTGGTGGTG CCTACGGTTT GGAGCACGGT
17881 GGTTTGGGTT CCGTCGTCGA TGGTGGTTTT AACCATGAGG TGTGTTCTCC CTTTGTGTTA
17941 GTTGCTGGTT TGGTTGTCGG CTAGATGAAT GATGTCGGGT AAGGGTTTCG GCTGGTCTAA
18001 ATGTTGTGTG GTTTTGTTGG CTAGCCGTTT GGCTACCCTG TAGCACATTT TGGTGTAGTG
18061 TTTGTTGTCT AGGTTGTGGT ATTGTTCCCG CACCGCAATA TATAGCAGGG AGTCTTGGTA
18121 CAGGTCGTCT GCATTGATTG CGGGGTAGTG TGCGGCTGTT TTAGTGCATG CCCGGTTGAG
18181 TGTGCGTAGA TGATGGTCTG TGGCCCACAC CCACGATGCG GTGGTGGCTA GGTCGGCTTT
18241 TGTTGGTCGT CGGCTCATGG CATCTCTTTC ATCTGGCTAT CTGGTAGTTG TTTGGTGTTT
18301 TGTTGTTGAT AGTGTAGCAC ACGAGTCCGG GGTTTCCGGT GGTGCCCGTC TTGTGCCGGT
18361 ACCATGTGGA TTCGCCTTCC ATGGATGGGC ATTGGATGAA GGTGCGTTGT CCTTGTTCGG

18421 AGATTTCTAG GTGGTGCCTG TGTCCGGCCA TGAGGATGTG GGATGTGGTG CCGTTGTGGA
18481 ATTCTTGTCC GCGCCACCAA TCATAGTGTT TGCCGGTGCG CCATTGGTGG CCGTGGGCGT
18541 GTAGTATCCG TGTGCCGGCT ACTTCGACGG TGGTGGTCAT TTCGTCTCGG CTGGGGAAAT
18601 AAAAGTGTAG GTTGGGGTAT TGGTTGGTGA GCTGGTAGGC TTCTGCGATG GCGCGGCAGC
18661 AGTCTACGTC GAAGGAGTCG TCGTAGGTGG TGACTCCTTT GCCGAAGCGT ACGGCTTCTC
18721 CGTGGTTGCC GGGGATGGAT GTGATGGTCA CGTTTTTGCA GTGGTCGAAC ATGTGGATGA
18781 GTTGCATCAT GGCCATGCGG GTGAGCCTGA TTTGTTCCGT CAAGGGGGTT TGTGTGCGCC
18841 AGGCGTTGTT GCCTCCTTGT GACACGTATC CTTCGATCAT GTCGCCGAGG AATGCGATGT
18901 GGACTCGTTC GGGTTTGCCT GCCTGCTGCC AGTAGTGTTT AGCTGATGTG AGGGAGCGCA
18961 GGTAGTCGTC GGCGAAGTGT GATGTTTCCC CGCCGGGGAT GCCTTTGCCG ATTTGGAAGT
19021 CGCCTGCCCC GATGACGAAG GCCGCAGTGC TGTAGTCGGT GCGGGTGTCC TGTTCGGGTT
19081 TTGGGGGTGT CCATTCGGCT AGTTTATCGA CGAGTTCGTC TACAGGGTAG GGGTTTGTTG
19141 CGGGTTGGTG GTCGATGATT TTTTGTACGG ATCTGCCTGT TTCTCCGTTG GGGAGTGTCC
19201 ATTCGGAGAT GCGTGTGCGG CGTACGGTGC CGTTTGCGAG ATCATCGCAG ATGGTGTCTG
19261 CTTCGCTATC GTGGTTGGCT AGCTGGGTGA GTAGCCGGTC TATGTTGTCT ATCACTGGGT
19321 ATCCTCTTCT TGCGGGGTGG TGTTGGCTTG TTTGCGGCGG TAGTCTTTTA TAACGGTGGC
19381 GGAGATGGGG TATCCTGCCT GGGTGAGCTG TTTTGCTAGC CATGAGGCGG GGATGGTTTT
19441 GTCGGCGAGC ACGTCGGCAG CCTTGTTGCC GTAGCGTTGG ATGAGTGTTT CAGTTTTGGT
19501 TGCCATGGTG TCCTATCGGT TGTGTGGTGG GCTGCCATCC TGTGCGGCAG TCGCCGTCGT
19561 GGCCTGGTTT GCGTGTGCAC CACGATACGG TTCTGTCTGT GTGGTTGAGT GTTTTGCCGC
19621 ACATGACGTT TTGTAGATGC TCTGGCAGTG CGCCGTCACC CTGGTTGCTG GTTTGTGTGT
19681 CGAAGAGTGT TTTCTGGTTG GTGAAATGCT CGGACACGGT GCCATTATGT ACGGGTAGTA
19741 TCCATGTTTT CCATTGTTGT TGTAGCCGGG TGTTCCAGTG GAATTGTTTT GCTGCGTTCG
19801 TGGCTTGTTT GATGGTTTTG TAGTAGCCGA CGAGGATGCG CTGGTGTTCA CTGTCGGGAG
19861 GGTTTTGGCC TCGCCAGTAT TGTGCCGCCA CGGCGTAGCG GTTGCTGGCT GTGAAGGCGT
19921 CCCAGCAGTA TTCAATAATG TGTTGTAGTA CACTATCGGG CATGTCTCGT ACTTGGTTTT
19981 CGTCGAGCCA CGCGTCGACA ATGATGTTGC GTATGGCGCG TTTGTCTTTG GTGGTGGGTT
20041 TGAATGCGAT GCTCACAGTA CGGGCCTGTC GTCTTGCATG AAATCATTAA AGGATGATTC
20101 GCTTGCGCGG CGTGCTTGTG TGATTTGCTG GTCAGACCAG TCGGGGTGTT GCTGTTTCAG
20161 ATAGTACCAG TGGCACGCAT TGTAGGTTTC GTCTTGTAGC CGGGTGAGAT GGTTTTCGGT
20221 GATGATTTGT TTCCACATAG TCCATGACAC GTCGAGCCGG TCCAATATTT CCATTGCTGG
20281 AATGTTGAAC TGGTTCAGGA AGAGTATTTC GTGGGTGTAG TATTCCTTCT CGTACTGGTC
20341 CCATCCACTT CGGTGCCTGT TGGGCTGGTT TTTGGGGTAG GCTTCCCGGC ATACTTTGTG
20401 CAAATGTTTG GCCATGTCGT CGGGTAGTTT AATGTCAGGG TTGGCGCGGA TCATGGATCG
20461 CATCCCATCA TAGGTGGTGC CCCAGGTGTG CATGATGTAG GTGGGGTCTT CACCATCAGC
20521 CCATTTTTCT GCACAGATGG CGAGGCGGAT GCGTCTCCTG GCTGATTGGC TGGTGTTGCG
20581 CCGGTTGGGG ATGGGGCACG TGTCGAGGGG ATCCATGATG TTTTGGTGTA CCTTTCTTGG

20641 TTTAGGTTGC TTGTGTGGTT TTATTGTAGC ACTGTGTCTA GTGCTTGTGT CAACCCTGTT
20701 TTGCCGGCCT GAAGGTAGGT GTCTGTGACA TCCCCCAGGG TGAGGGGCAC ATGGGTGGCT
20761 TGGGGGAGTG CGGCCTGGAG TGTTTGGGCC ATCTGGTGGC CCGCCTTGTC TGGGTCTGAC
20821 CAGATGTAGA TGTGGTCGTA GCCTTCAAAA AATTTGGTCC AAAAAGTTTG CCACGAGGTT
20881 GCGCCGGGTA GGGCTACGGC TGGCCATCCG CATTGTTCGA GGATCATGGA GTCGAATTCG
20941 CCTTCGCAAA TGTGCATTTC GGCTGCCGGG TTGGCCATGG CGGCCATGTT GTAGATGGAG
21001 CCTGTGTCTC CTGCCGGGGT TAGATATTTG GGGTGGTTGT GGGTTTTGCA ATCATGTTGG
21061 AGTGAGCAGC GGAAACGCAT TTTTCGTATT TCGGCTGGCC CTTCCCAGAC GGGGTACATG
21121 TATGGGATGG TGATGCACTG GTTGTAGTTT TCGTGGCCTT GGATGGGGTC ATTGTCGATG
21181 TATCCAAGGT GGTGGTAGCG GGCTGTTTCT TCGCTGATGC CTCTTGCCGA GAGCAGGTCG
21241 AGTATGTTTT CGAGGTGGGT TTCGTAGCGG GCTGAGGCTT TCTGGATTCG GCGGCGTTCC
21301 GCAATGTTGT AGGGGCGTAT GCTGTCGTAC ATTCGGGTTT TCTTCCTCTA ATCGTTGTTT
21361 CAGTTTGTGG AGTCCGCCTC CGATACCGCA TGTGTGGCAG TACCAGACGC CCTTGTCGAG
21421 GTTGATGCTC ATGGAGGGCT GGTGGTCGTC GTGGAACGGG CAGAGGATGT GTTGCTCGTT
21481 CCGTGACGGG TTGTAGCGTA TCTGGTGGGC GTCTAGGAGG CGGCAGGTGT CAGAGGTGTG
21541 GGAGGAGCTC GTTGAGGGTT GATACCACAT AGGCTTCGCT CCAGGGTTTG TTGCGCTGTT
21601 TCATGATGAC GAGTCCGATG GTGGATTGGT TTTCGCGGTT TCGGTGTGTT TCGTAGTTGC
21661 GTGCCTCCCG GCTGGCTTGT TTCACGAATT CGGCTAGGTG TGCCTGTCCT GCTTTGGCTT
21721 CGATCACATA GGTTTTGTTG CCGGTTGTGA GGATGAGGTC GCCTTCGTCT TCTTTACCGT
21781 TGAGGTGGAG GCGTTCTATA TCATAGCCGG TGTCGCGTAG CTGGTGGAGG AGTCTTGTTT
21841 CCCATTCGGC GCCGGCTCGG CGGTTGCGTG CCTGTTGTGT TGACATGATA GTCCTTTATG
21901 TTCTTGTGTC ATGTTCCAGG GCTGTTTTTC TACTAGGGGC CCGAAGAATG TGTATTCGGG
21961 GTAGGCTCGT AGTCGTTCGT ATTTTGTTCC GTCTGGGCTG GATTTGCCGG TTCTCTGTTT
22021 CAGGACGGCG ATGCGTGCCT CGGCGGGGAT GGTGAGGCCG TTGCCGTTGT CTTCGCCACC
22081 ATACAGGGAG ACTCCCAATA TGAGTTGTGG TTTTTCGGAG AGGCCGTTTT TGATTTCCCG
22141 CCTAGCTGGG GGGTGTTCGA TGTCGGTGCC GGTTTTGTCG GTTGCGTGGT GGGTGACGAT
22201 GATGGTGGAG CCAGTATCTC TACCTAAGGC TGTGATCCAT TGCATGGCTT CTTGCTGTGC
22261 CTGATAGTCG GATTCGCAGT CTTGGATGTC CATCAGGTTG TCTATAACAA TAATGGGTGG
22321 GAAGGTGTTC CACATTTCCA TGTAGGCTTG CAGTTCCATG GTGATGTCTG TCCATGTGAT
22381 GGGTGACTGG AATGAGAAGG TGATGTGTCC GCCGTGGTGG ATGCTGTCTC GATAGTATTC
22441 TGGCCCGTAG TTGTCGATGT TGTGTTGTAT CTGTTGGGTG GTGTGTTGGG TGTTGAGTGA
22501 GATGATTCGT GTGGAGGCCT CCCAGGGTGT CATGTCCCCT GATATGTAGA GGGCTGGCTG
22561 GTTGAGCATC GCGGTGATGA ACATGGCTAG CCCTGATTTT TGGCTGCCGG ACCGCCCCGC
22621 GATCATGACC AAATCCCCTT TGTGGATGTG CATGTCCAGG TTGTCATACA AGGGTGCTAG
22681 TTGGGGTATG CGGGGCAGTT CGGCGGCTGT TTGGGAGGCC CTCTCGAAGG ATCTTTGGAG
22741 AGAGAGCATC GGGACCTTAA TCTATCTGTT GGTTGGGTGT GTTTTGGTGG TCAGATGGAG
22801 TCGATGTCGA TGTCAGCATC GGCGGGGGCT GTGGTGTCGT CTAGCTGGCC GTTGTCGCGT

22861 TTGTCTACAT ATTCGGCAAC CTTATCGTAG ATGGCGTCGT CGAGGGGTTT GAGGACGACC
22921 GCGTTGAACC CGTTTTTGGT GCGCACGGTG GCAAGTTTGA AGGCTTGTTC TTCGCCGAGA
22981 TATGCTTCTA GGTCGCGGAT CATGGAGTGT GGGCGGTCGT TGTTGCCGCG TGCTTTTTCG
23041 ATGATGGCGT TGGGGATGGT TTCTGGGGTG CCGTTGTTGA GATCCTGGAG GGTGTGGAAG
23101 ATTGTGACAT CAGCGTAGAT GCGGTCTGCG ACCTGTCCAC CGTAGCCTTC GGTGTTGTGT
23161 TCTACGTCGC GGATTTTGAA GGCGATGGCG GTGGCGTCCT GGTTTCGGGA GGGGTTGAAG
23221 AAGGTGCTGT TGCTGTTGTT GTGGTAGTTG GCGAGTGCCA TGATTGTGTT ATCCTTTACT
23281 GTTGTGTCTG TTTTTGTTGT CTTATATTGG TTTATCGGGT GAGGCTGTTT CGTTTGCTGC
23341 GGAAAGCCTC GGAAACGTCA CTGTTACTGG TGATGGTCTT CTTGTACTGT TTGAGTAGGT
23401 CTGCTAGCTG TGTCTTGCTG GTGGCTTTGT TTATCCGGTC GATGATGATG TCGTTTTCCT
23461 GTGATGCGAT TTTGTTGACG TAGTCTTTGG CGGCTTTATC GTATCGGTCT TGAAGCAGGA
23521 TTGCTGCGCT AGCGATGAGG GTTGCGAGAT CCCAGTCTTT GGATACGGTT TCGTCTTTCA
23581 ATCCTCCTAG CAGATCAATA ATGGATTGTT TGATGTCTTC TGCGGTGTCT CCGCGGATGA
23641 CTGTCCATGG GGCGGCATAG TCGCCACCGT ATTTGAGTGT GATAGTTAGT TTTCCGCTGT
23701 CTGTGGTGTG CTCGTCGGTC ACGTGTTTTC CTTTTCGTTG TTTTCGGCTT CTGGTGGCTG
23761 TACGGTGGTT TCTATCGGGT ATCTGTAGGC GTCTTTCCCG TTGACGGCCC AGCAGGCGTC
23821 CTTGACGGGG CATCCTTTGC AGAGTGTGGT GACGTGGGGT ACGAAGATGC CTTGGCTGAT
23881 TCCTTTCATT GCTTGACTGT ACATGGATGA TACATGCCGG TAGGTGTTGT TGTCAAGATC
23941 AATGAGTTCG GTTGCTGTGC CCTGCTCGAC TGATTGCTCG TCTCCCTTGG TGGTGGCGGG
24001 TGTCCAAAAC ATGCCTTTCG TCACATGGAT GCCGTGTTGG GCGAGCATGT ACCGGTATGT
24061 GTGCAGCTGC ATACTGTCTG CGGGTAGGCG TCCGGTTTTG AGGTCCAAAA TGAAGGTTTC
24121 GCCGGTGTCG GTGTCGGTGA ATACCCGGTC AATATATCCG ACTATTTTTG TGTCATCGTC
24181 GAGGGTGGTT TCTACCGGGT ATTCGATGCC TGGCTGGCCG TCAATAACAG CGGTGGCGTA
24241 TTCTGGGTGG TTGCGCCTCC ATGTTTTCCA GCGGTCCACA AAGGTGGGGC CGTACATCAT
24301 CCACCAATTG TAGTCTTTCT TGTGTGGCCC GCCTGACTCG CACATGTTTT TGCATATTCT
24361 GCCGGAGGGC TTTATGTTTG TGCCTTCGGA TTCGGCGAGG GCGATTTGGG TGTCGAAAAT
24421 GTTTGTGAAG GATGAGAGTT TGTCTGGCAG TGCAGGGTAT TCGGCGGGGT TGTACAGGTG
24481 TAGGTCGTAT TGTTCGGTGA TGTGGTGTAT GGCGCTTCCG GCGATGGTGG CGTACCAGGT
24541 GTGGTGTTGG GCGTGGTAGC CGTGTGCTAG GCGCCATTTT TCGCCGCATT CGGCCCACTG
24601 TGTGAGTGAA CTGTAGGAGA TGTGGCCTGG ATGGTTGATG GTTTTCGGGT ATTGTGCTAG
24661 GGGCATTACT TGTCGCCTTT GTGGGTGTTC CATGGGTTGC GGGTGTCTTT GCCGGCGTGG
24721 TGTTGCTGGT AGGCGAGGAG TGCGAGGCAG TGCCAGGCAG CGTGTGCCAG ATGCGGCAAA
24781 TGTGATTCGT TGTCGAGGTT GTTGCCTTGC TGCCATGATA ACAGGTGCCG GTAGAGGGCG
24841 TCGACACTGT GGCTCCACGG GTATCCTCCG GTCCAGTTGT TGTCGCCGTA CTTGGTGGCA
24901 CCGTAGCCTG CCACGGAGCC TAGGGCGTGC AAGGCTGCGG GGTCGATGAG GGAGAGCCTG
24961 CAGAGTTTCA ATTCTTTTCG GGCACCGCTG TTGGGGTCGG TGTACATGCT GGTGGGCTCA
25021 TCCATGGTGT GTGTGCTCCT TAAGCGTGGG TTACTGGTTA TTGTCGTGGG CGAGTGCTAC

25081 GGCGAGAATA ATGATGGCGA GGGTTTCAGC GATCAGTATG GGTGTTGTGA TCATTTAGTG
25141 TCTCGGGGAT TATTGGTGAG TGTTGATGCA CCTAGGAGGG TGGCGAGGGC GCATGCGGCG
25201 ATGGTGGCGA GGGCTGCCTT GTGTGGGGTG CCGGTTGCGT ACATCCATGT GATGATGCCG
25261 CCTTGGATCC AGGCTAGACT GGTGAAGAAC GTTTCGTAAC TGTGTAGCTC AATGTTGTTG
25321 TTGGGTGTGT TCATGCTTGC TCCTGAAGAA TGGTGTTGAT GGTTTTATAA ATGTTGTACA
25381 GGTCGGTTTC GATAGATAAC AGTTGGTTGA TTTGGTGGTC GAGATCAATG TCTGGGTTGA
25441 GGGTGTCGAT GCGGGCGGCG ATATCGGTGG CGGTGCGTAG GCTTACTGCT GCACCGTGGA
25501 TGATGTGGCA CATGTCGGTG AGGCCGACTT TGGCGATATA GTGTGACATG AGAGGCATAA
25561 TAGGTGTGCT GTCTTTCTGG TCAGCGTGAA GGGTTGATGG ACATATCCTC TACCTGTGGT
25621 TTGTCTTCGG TGCCGGAGAC TTGGCAGAAG ACTTTCACAT GCGTCTTGGA TGCTCCGGCC
25681 TGTTTGGCGG TGGCACCGTA GGCGATAGTA AAGGTGTCTT TGTGGGCGCC GATGACTTTG
25741 TGTAGGAAGA GGTCGATGTC GGGGTTGCCG TTCCATTTGA CACCGTTTTC TGCGGCTGTC
25801 TGGGTGGCTT TCTGATTGCA GGCGTGTGCG GCGGTGATCA TGGTGAGACC CTTGCTGGTT
25861 TCTTCACCCC TTGCTTGGGC TTGCCGGTGG GCTTTGGCCT GCTCGGCTTG TAGGGAGCGG
25921 ACTGCTGCGG CCTGGCGGGC CTTCTTCTCA GCCTTGCGCT GCTGGACGGT TTTGGGTGTC
25981 CATTCGGTGT TGGCTGTGGT TACCTGTGGT GCGGGTTGTG AGGCGAGTGG CGGATTGTCG
26041 TCTGGGGCTG GCATGAAGGA TGCTGCGGCA ATAATGGCGA CTGTGGCGCC TGCGATGGTG
26101 TAGCCTGTTT TCTTGTTCAT GATTTTATGT TCCCCTTTCC GGGGTGTTGT TCGTTGCTGA
26161 CATGGTTAAT ACTTTCAGCG GCTGGGCCCA CTGTCAAGGC TGCGCTCAGT TTGTGTGAGC
26221 GTTTCTTGTG TGGCTAGGGG TGATGGCTTC TTTCGCCCAA TAGGATGTGC CACCGCTGGT
26281 CCAGTATCCG AGTTTGTTGC GCTGCATGCC CTTGGCGTCC ATCTCGTCGA TAGTGAGGCA
26341 CCTGCGGCGA TTGGGGCCTG TCTTGACCCC GTGGTCGCCT GTCCGGTGCA TGTCGCCTGA
26401 GGTGGTACTC GTGAATGTTT CATGGCAGAT GGTACAGTGC TCTGGTCGAT ATCCGGTGAT
26461 TGTGCTATCG CACTTGTGGC ATGTCCATTC CATGATTGCT CCTATTTTCC ATTATAAGAC
26521 TTCCTGTAGT GCCATTTTAG CGCCTTGCGG GTCTTGGGGG TACAACTATA TAGGTCAGGT
26581 GTTTCTAGGC GATTCTAGGC TCATTGTGTG TGGCTGGGGT TTTATCGGGC ACACAGGGTG
26641 AGCAGGTGGC CAACATTGAT GCGGGTCACA TTCCAGTAGA GTTGCGTGGC TTCCCCACTG
26701 GTGAGCGGCT TCCACTCGTC ATGGCTGAAC ACGGTGCCAT CGGATGCGAT GAACGTGTTG
26761 GGGCGTAGCT TGTGGAGTTC GGCTTCCACG CTCTGCCGGT AGGCTTCGGC GAGGCCCTCA
26821 AAATCCATGT GGTCGCAGGG GAGGTTTTCG AGGCGTGTCA GGTCGAAGGG TGTGGGGCAG
26881 TCGTAGCTGG CGGGGGTGTA GAGCTGGGTG AAGTGGTTGG CGATCTTCTG CATCATGATT
26941 CCTTTTCTGA TGATGGTGTG TTGAGGGTTT ATCGGGTGGA TGCGACAAGG ATGGCGTCTA
27001 CATCGATCAT GTCGATGAGA TCGTGGAGTT CCTCGGCCTC GTTCTCAGTG AGTGGCTGCC
27061 AGGCGTAGTC GCCGTATACG GCGCCGTCGA GGGTGACAGT CCACGGGGGC CGGATGAGTC
27121 GTATGGCTTC TTGTACTTTA GCGTGGTACA TGCGGCGCAC CATATCCAGA TCGATGTCGT
27181 CTGAATGGTT TCCGGTGAGG CTGTGGAGGC TGAGCGGGTC GATGTCTGTC TGCCTGTAGA
27241 GGGATGTGAA GGATGGGGTG ATGAGTGTGC CATCCATGAG TGTGCTCCTT TCGGTGGTTG

27301 TAGGGGTTGT TGTGGTTTCT AGAGTGTGCG GGCTGCGACC CCACAGTCAA GGTGTCGCTC
27361 AAACTCAGTG AGCGTTTCAT ATGGGTGTGT TGGGTGTGAC AGATGTCACT TAAGCCTTGA
27421 TGGCCTCTCT CAGCGCCTCA AATCTTCTAG GGGTAGGATT ATGAAGGGTT GGCCCTGCTG
27481 ATCGATTCTA GGCCCCATAC AGGGCGTCTG AGGGGTGTGT CTGAGTGATA GTGGGTGTGG
27541 CAGATGATCT AGCGAGTCAA GGTGCCGAGC TGAGACATAA GATCTATCAT CTAGGTGTGT
27601 GAGATGTATC ACATCCTCCC GGCTTGGTGT GCACCCTCAA GGCCACCCAG TCGATCTGAC
27661 GTGGAGGGTG TAGCCCAGAA ATACTGTTTA AAGCCTTCAC ACGGCGCCTA GGAGCGCCTT
27721 ACAGGGTGGG GGCTAGGTAT TTATACCCCC AGCACATTCT GATCGATTCT AGACGCCTAC
27781 AGGAGCCCGA TACACGATCA GCCATCCAGA CGCAGATCAT CAGCACCTAT CATGGTTAGC
27841 TAAGCCTCAA CTATGTGGAC AGTGTTGGTT ACTGTGGGGG AAGAAGGACA CGGTAAAAGA
27901 AAGAGGGGGA GTATCAGCTT TAAAGCCTTA AGGTCTTAGC GCTTAGCACC GATGGTCTTA
27961 GCAGTTAGCA CCGAGCCCCC TCAAGGGCTC GGCATCAGCC CGAACAGGCA CAGCCATGAA
28021 AGGAGTACAC GCCATCAGGG AAGGCTTTCG AGTACGAGGA GCCTCAGCGA CGAGTACTCG
28081 AAAGCCTGAG GGAACACCCA TCAGCACTGA TGAGCCTAGC GTATTCGGAA AGGACACAAG
28141 AGTGAAGTGT GACAGCTGTC CGGGAGTGAA CCCCGTTCTG ACTAGGGGTT TCAGCCTTAA
28201 CCACCCTCAA AGGTTACAAG ACTCTAAGAA AATTTAAGGA AAAGTTTAGG TTTAATTTTT
28261 GGACCTTTAC TACCAAAAAC ACCCGTTTAC AGCCCTCAAA CCCGCCTATA GAGCCAAAAC
28321 CACCAGTTTG ACTCATCCCA GGTGGGGTAT GATAGGCTGG ACAGGTAGCC AGCTGGACGC
28381 AAGGCCGGAA AGTGCTAACG CACTTTCCAA CCTCGCTTAC CATCAGTCTA CCAAACACTT
28441 AAAGACCTAA GGGCTTAGCG CTAAGGTGCT GATAGCTTAG CACCGAGCCC CCTCAAGGGC
28501 TCGGCATCAG TCTTAAAGCC TTAAATACTT AAAGTAACTA TAAAACTTTA AAAGCTTAAC
28561 ACTTAAGGAT ATAAACTTTA CATCAGTGTT TAAGACTTAA AAACTTAAAA TAACTATTAA
28621 GACTTAAAGT AACTATAAAA CATTAAAGAC CTTAAGTACT TAAAGTTAAC CATCAGTCTT
28681 AAACTTTACT ATGATAACCT ATAAGTCTTA AAGCTTATAG GTATAATAAT ATAATATAAG
28741 TATTAAAGCT TATAAGTTAT AAAAGTTTTA GAAGAGTTAA AGGGTTAACT TCTTTACTTC
28801 TCTTCTCTCT TTGGTTCTTT CTCTCTTCTC TTCTTTTCTT CATCGGGGGA GAAGAGGAAC
28861 CTTTAACGTC AACGCTGATG GACTTTTCGC CGTGTGTCTC GTGTGCTTCT GGTCGCAAGC
28921 TCCCATCGCA CACTCCCCAC ACTCTTTCAC CTGTGTCCCT TTCAGGCTTA GCGTGTTCAG
28981 CTGAAGGCGT ACAGCGTGTC ACGCTTAAAC CCTTAACACC AGGTAAGACT TAAAGTGCAT
29041 ATTATAAGTA GAAGACTTTA AAACCTTAAG GGTGTTCCTG CTTAGCCTGT GTCCTTTAAC
29101 GCTAGGCGCT AAGCCGTGAA ACGTGAACAC CCATCCACCC CTCTTCTTTT TACCGTGTCC
29161 TTCTTCTTTT GACACCGCTG GGGGGCGATG TGATCTTTTT AACATGCCAG GGGGTGCGGG
29221 TAGAAAACAA CCACCCCACC ACAAACAGAA CACCCCCTCA AACGCACAAA ACAGCCCCCA
29281 GGATCGATGA ACAGGGCAAG GGCAAGGTAT TCATACCCCC AGACGATTCC AGGCCGTTAG
29341 AGAGGCAAAT AAGACCCGTA CAGGGCTAGG TGAGGAATAG ACACATCATG GCACGCACCA
29401 ATCGCACAGC TAGCCAAGCC CACCGACGCT GGCGGCAACG ACTCATCACC CAAGCCCAAC
29461 AACAAGGCCA AACCGAATGC CCACTCTGCG GAGTCACCAT CACCTGGGAC ACACACGACC

29521 TACCAACCAG CCCCGAAGCC GACCACATCA CACCCGTCAG CAGGGGAGGA CTCAACACCC
29581 TCGACAACGG GCAAATCATC TGCAGAACAT GCAACAGAAG CAAAGGCAAT CGCAGCGAAC
29641 CAAACATCAA ATTCCAACAA CAAACCACAA AAACATTGAT TCCATGGTGA CAAACCCGCC
29701 AACCCCCACC GGGGACACCC CCTGCACAGG CGTGCAAGAC CTCGTACGGC TT
(SEQ ID NO: 1)

In embodiments, the bacteriophage is a bacteriophage as deposited under Accession No. NCIMB 41349, 41350, or 41351. In embodiments, the bacteriophage has a genome with a nucleotide sequence that is at least about 80%, 81%, 82%, 83%, 84%, 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% identical to the genome of the bacteriophage deposited under Accession No. NCIMB 41349. In embodiments, the bacteriophage has a genome with a nucleotide sequence that is at least about 80%, 81%, 82%, 83%, 84%, 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% identical to the genome of the bacteriophage deposited under Accession No. NCIMB 41350. In embodiments, the bacteriophage has a genome with a nucleotide sequence that is at least about 80%, 81%, 82%, 83%, 84%, 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% identical to the genome of the bacteriophage deposited under Accession No. NCIMB 41351.

The bacteriophage has a genome with a nucleotide sequence that is at least about 98% or 99%, identical to the nucleotide sequence of SEQ ID NO: 1. In embodiments, the bacteriophage has a genome with a nucleotide sequence that is identical to the nucleotide sequence of SEQ ID NO: 1.

In embodiments, the genome of the bacteriophage encodes, from the 5' to the 3' end, a small terminase, a large terminase, a portal protein, gp4, a scaffold protein, a major head protein, gp7, gp8, gp9, gp10, a major tail protein, gp12, gp13, a tape measure protein, a minor tail subunit, optionally a protease, gp17, gp18, a tail protein, an amidase, a holin, gp22, gp23, a sigma factor, gp25, gp26, gp27, gp28, gp29, gp30, a DNA primase, a DNA primase 2, gp33, a DNA helicase, gp35, gp36, an exonuclease, gp38, gp39, gp40, gp41, gp42, gp43, gp44, gp45, gp46, gp47, and gp48.

In embodiments, the composition further includes a *P*. *acnes* biofilm degrading enzyme.

In embodiments, the enzyme is an anti-aging enzyme. In embodiments, the anti-aging enzyme is a superoxide dismutase or a peroxidase.

In embodiments, the enzyme is a *P. acnes* biofilm degrading enzyme. In embodiments, the enzyme is a glycosidase, a protease, a DNAse, or a restriction endonuclease. In embodiments, the enzyme is a glycosidase. In embodiments, the glycosidase is a glycoside hydrolase. In embodiments, the enzyme catalyzes the hydrolysis of linear polymers of N-acetyl-D-glucosamines. In embodiments, the enzyme is a β-hexosaminidase. In embodiments, the enzyme hydrolyzes β-1,6-glycosidic linkages of acetylglucosamine polymers. In embodiments, the enzyme is a DNAse I, a restriction endonuclease, papain, bromelain, Trypsin, Proteinase K, Subtilisin, serratiopeptidase, dispersin, alginate lyase, amylase, or cellulase. In embodiments, the enzyme is Dispersin B. In embodiments, the enzyme is a protease, and the protease is proteinase K or subtilisin.

In embodiments, the enzyme is a dispersin. In embodiments, the enzyme is Dispersin B. In embodiments, the enzyme is a naturally occurring form, homolog, isoform or variant of a dispersin (such as Dispersin B) that maintains the enzymatic activity (e.g., within at least 50%, 80%, 90%, 95%, 96%, 97%, 98%, 99% or 100% activity compared to the native protein). In embodiments, variants have at least 90%, 95%, 96%, 97%, 98%, 99% or 100% amino acid sequence identity across the whole sequence or a portion of the sequence (e.g. a 50, 100, 150 or 200 continuous amino acid portion) compared to a naturally occurring form. A non-limiting example of a DNA sequence that encodes Dispersin B is as follows:

ATGAATTGTTGCGTAAAAGGCAATTCCATATATCCGCAAAAAACAAGTACCAAGCA
GACCGGATTAATGCTGGACATCGCCCGACATTTTATTCACCCGAGGTGATTAAATC
CTTTATTGATACCATCAGCCTTTCCGGCGGTAATTTCTGCACCTGCATTTTTCCGAC
CATGAAAACTATGCGATAGAAAGCCATTTACTTAATCAACGTGCGGAAAATGCCGT
GCAGGGCAAAGACGGTATTTATATTAATCCTTATACCGGAAAGCCATTCTTGAGTTA
TCGGCAACTTGACGATATCAAAGCCTATGCTAAGGCAAAAGGCATTGAGTTGATTCC
CGAACTTGACAGCCCGAATCACATGACGGCGATCTTTAAACTGGTGCAAAAAGACA
GAGGGGTCAAGTACCTTCAAGGATTAAAATCACGCCAGGTAGATGATGAAATTGAT
ATTACTAATGCTGACAGTATTACTTTTATGCAATCTTTAATGAGTGAGGTTATTGATA
TTTTTGGCGACACGAGTCAGCATTTTCATATTGGTGGCGATGAATTTGGTTATTCTGT
GGAAAGTAATCATGAGTTTATTACGTATGCCAATAAACTATCCTACTTTTTAGAGAA
AAAAGGGTTGAAAACCCGAATGTGGAATGACGGATTAATTAAAAATACTTTTGAGC
AAATCAACCCGAATATTGAAATTACTTATTGGAGCTATGATGGCGATACGCAGGAC

AAAAATGAAGCTGCCGAGCGCCGTGATATGCGGGTCAGTTTGCCGGAGTTGCTGGC
GAAAGGCTTTACTGTCCTGAACTATAATTCCTATTATCTTTACATTGTTCCGAAAGCT
TCACCAACCTTCTCGCAAGATGCCGCCTTTGCCGCCAAAGATGTTATAAAAAATTGG
GATCTTGGTGTTTGGGATGGACGAAACACCAAAAACCGCGTACAAAATACTCATGA
AATAGCCGGCGCAGCATTATCGATCTGGGGAGAAGATGCAAAAGCGCTGAAAGACG
AAACAATTCAGAAAAACACGAAAAGTTTATTGGAAGCGGTGATTCATAAGACGAAT
GGGGATGAGTGA

(SEQ ID NO: 11)

A non-limiting example of a Dispersin B amino acid sequence is as follows:

MNCCVKGNSIYPQKTSTKQTGLMLDIARHFYSPEVIKSFIDTISLSGGNFLHLHFSDHENY
AIESHLLNQRAENAVQGKDGIYINPYTGKPFLSYRQLDDIKAYAKAKGIELIPELDSPNH
MTAIFKLVQKDRGVKYLQGLKSRQVDDEIDITNADSITFMQSLMSEVIDIFGDTSQHFHI
GGDEFGYSVESNHEFITYANKLSYFLEKKGLKTRMWNDGLIKNTFEQINPNIEITYWSYD
GDTQDKNEAAERRDMRVSLPELLAKGFTVLNYNSYYLYIVPKASPTFSQDAAFAAKDVI
KNWDLGVWDGRNTKNRVQNTHEIAGAALSIWGEDAKALKDETIQKNTKSLLEAVIHK
TNGDE
(SEQ ID NO: 12)

In embodiments, the enzyme is an alginate lyase. In embodiments, the enzyme is a naturally occurring form, a homolog, an isoform or a variant of an alginate lyase that maintains the enzymatic activity of the alginate lyase (e.g., within at least 50%, 80%, 90%, 95%, 96%, 97%, 98%, 99% or 100% activity compared to the native protein). In embodiments, variants have at least 90%, 95%, 96%, 97%, 98%, 99% or 100% amino acid sequence identity across the whole sequence or a portion of the sequence *(e.g.* a 50, 100, 150 or 200 continuous amino acid portion) compared to a naturally occurring form. A non-limiting example of a DNA sequence that encodes an alginate lyase is as follows:

ATGAAAACGTCCCACCTGATCCGTATCGCCCTGCCGGTGCCCTCGCCGCGGCATTG
CTCGCCAGCCAGGTCAGCCAGGCCGCCGACCTGGTACCCCCGCCCGGCTACTACGC
GGCGGTCGGCGAGCGCAAGGGCAGCGCCGGCAGCTGCCCCGCGGTGCCGCCGCCGT
ATACCGGCAGCCTGGTCTTCACCAGCAAGTACGAAGGCTCCGATTCGGCGCGGGCG
ACCCTCAACGTCAAGGCGGAGAAGACCTTCCGCTCGCAGATCAAGGACATCACCGA
CATGGAGCGCGGCGCCACCAAGCTGGTCACCCAGTACATGCGCAGCGGCCGCGACG

GCGACCTGGCCTGCGCACTGAACTGGATGAGCGCCTGGGCCCGCGCCGGCGCCCTG
CAGAGCGACGACTTCAACCACACCGGCAAGTCCATGCGCAAATGGGCGCTGGGCAG
CCTCTCCGGCGCCTACATGCGCCTGAAGTTCTCCAGCTCGCGGCCGCTCGCGGCCCA
CGCCGAGCAGAGCCGGGAAATCGAGGACTGGTTCGCCCGGCTCGGCACCCAGGTAG
TCCGCGACTGGAGCGGCCTGCCGCTGAAGAAGATCAACAACCATTCCTACTGGGCG
GCCTGGTCGGTGATGTCCACCGCGGTGGTGACCAACCGCCGCGACCTCTTCGACTGG
GCGGTGAGCGAGTTCAAGGTCGCCGCCAACCAGGTCGACGAGCAGGGCTTCCTGCC
CAACGAACTCAAGCGCCGCCAGCGCGCCCTCGCCTACCACAACTATGCGCTGCCAC
CGCTGGCGATGATCGCCGCGTTCGCCCAGGTCAACGGCGTCGACCTGCGCCAGGAG
AACCACGGCGCCCTGCAGCGCCTGGCCGAGCGGGTGATGAAGGGAGTCGACGACGA
GGAAACCTTCGAGGAGAAGACCGGCGAGGACCAGGACATGACCGACCTCAAGGTC
GACAACAAGTACGCCTGGCTGGAGCCCTACTGCGCCCTCTACCGCTGCGAGCCGAA
GATGCTCGAGGCGAAGAAGGACCGCGAGCCGTTCAACAGTTTCCGCCTCGGCGGCG
AAGTGACGCGGGTGTTCAGCCGCGAAGGGGGAAGTTG

(SEQ ID NO: 13)

A non-limiting example of an alginate lyase amino acid sequence is as follows:

MKTSHLIRIALPGALAAALLASQVSQAADLVPPPGYYAAVGERKGSAGSCPAVPPPYTG
SLVFTSKYEGSDSARATLNVKAEKTFRSQIKDITDMERGATKLVTQYMRSGRDGDLAC
ALNWMSAWARAGALQSDDFNHTGKSMRKWALGSLSGAYMRLKFSSSRPLAAHAEQS
REIEDWFARLGTQVVRDWSGLPLKKINNHSYWAAWSVMSTAVVTNRRDLFDWAVSEF
KVAANQVDEQGFLPNELKRRQRALAYHNYALPPLAMIAAFAQVNGVDLRQENHGALQ
RLAERVMKGVDDEETFEEKTGEDQDMTDLKVDNKYAWLEPYCALYRCEPKMLEAKK
DREPFNSFRLGGEVTRVFSREGGS

(SEQ ID NO: 14)

In embodiments, the enzyme is an amylase. In embodiments, enzyme is a naturally occurring form, a homolog, an isoform or a variant of an amylase that maintains the enzymatic activity of the amylase (e.g., within at least 50%, 80%, 90%, 95%, 96%, 97%, 98%, 99% or 100% activity compared to the native protein). In embodiments, variants have at least 90%, 95%, 96%, 97%, 98%, 99% or 100% amino acid sequence identity across the whole sequence or a portion of the sequence *(e.g.* a 50, 100, 150 or 200 continuous amino acid portion) compared to a naturally occurring form. A non-limiting example of a DNA sequence that encodes an amylase is as follows:

ATGAAACAACAAAAACGGCTTTACGCCCGATTGCTGACGCTGTTATTTGCGCTCATC
TTCTTGCTGCCTCATTCTGCAGCAGCGGCAAATCTTAATGGGACGCTGATGCAG
TATTTTGAATGGTACATGCCCAATGACGGCCAACATTGGAAGCGTTTGCAAAACGAC
TCGGCATATTTGGCTGAACACGGTATTACTGCCGTCTGGATTCCCCCGGCATATAAG
GGAACGAGCCAAGCGGATGTGGGCTACGGTGCTTACGACCTTTATGATTTAGGGGA
GTTTCATCAAAAAGGGACGGTTCGGACAAAGTACGGCACAAAAGGAGAGCTGCAAT
CTGCGATCAAAAGTCTTCATTCCCGCGACATTAACGTTTACGGGGATGTGGTCATCA
ACCACAAAGGCGGCGCTGATGCGACCGAAGATGTAACCGCGGTTGAAGTCGATCCC
GCTGACCGCAACCGCGTAATTTCAGGAGAACACCTAATTAAAGCCTGGACACATTTT
CATTTTCCGGGGCGCGGCAGCACATACAGCGATTTTAAATGGCATTGGTACCATTTT
GACGGAACCGATTGGGACGAGTCCCGAAAGCTGAACCGCATCTATAAGTTTCAAGG
AAAGGCTTGGGATTGGGAAGTTTCCAATGAAAACGGCAACTATGATTATTTGATGTA
TGCCGACATCGATTATGACCATCCTGATGTCGCAGCAGAAATTAAGAGATGGGGCA
CTTGGTATGCCAATGAACTGCAATTGGACGGTTTCCGTCTTGATGCTGTCAAACACA
TTAAATTTTCTTTTTTGCGGGATTGGGTTAATCATGTCAGGGAAAAAACGGGGAAGG
AAATGTTTACGGTAGCTGAATATTGGCAGAATGACTTGGGCGCGCTGGAAAACTATT
TGAACAAAACAAATTTTAATCATTCAGTGTTTGACGTGCCGCTTCATTATCAGTTCC
ATGCTGCATCGACACAGGGAGGCGGCTATGATATGAGGAAATTGCTGAACGGTACG
GTCGTTTCCAAGCATCCGTTGAAATCGGTTACATTTGTCGATAACCATGATACACAG
CCGGGGCAATCGCTTGAGTCGACTGTCCAAACATGGTTTAAGCCGCTTGCTTACGCT
TTTATTCTCACAAGGGAATCTGGATACCCTCAGGTTTTCTACGGGGATATGTACGGG
ACGAAAGGAGACTCCCAGCGCGAAATTCCTGCCTTGAAACACAAAATTGAACCGAT
CTTAAAAGCGAGAAAACAGTATGCGTACGGAGCACAGCATGATTATTTCGACCACC
ATGACATTGTCGGCTGGACAAGGGAAGGCGACAGCTCGGTTGCAAATTCAGGTTTG
GCGGCATTAATAACAGACGGACCCGGTGGGGCAAAGCGAATGTATGTCGGCCGGCA
AAACGCCGGTGAGACATGGCATGACATTACCGGAAACCGTTCGGAGCCGGTTGTCA
TCAATTCGGAAGGCTGGGGAGAGTTTCACGTAAACGGCGGGTCGGTTTCAATTTATG
TTCAAAGATAG

(SEQ ID NO: 15)

A non-limiting example of an amylase amino acid sequence is as follows:

MKQQKRLYARLLTLLFALIFLLPHSAAAAANLNGTLMQYFEWYMPNDGQHWKRLQN
DSAYLAEHGITAVWIPPAYKGTSQADVGYGAYDLYDLGEFHQKGTVRTKYGTKGELQS
AIKSLHSRDINVYGDVVINHKGGADATEDVTAVEVDPADRNRVISGEHLIKAWTHFHFP
GRGSTYSDFKWHWYHFDGTDWDESRKLNRIYKFQGKAWDWEVSNENGNYDYLMYA
DIDYDHPDVAAEIKRWGTWYANELQLDGFRLDAVKHIKFSFLRDWVNHVREKTGKEM
FTVAEYWQNDLGALENYLNKTNFNHSVFDVPLHYQFHAASTQGGGYDMRKLLNGTV
VSKHPLKSVTFVDNHDTQPGQSLESTVQTWFKPLAYAFILTRESGYPQVFYGDMYGTK
GDSQREIPALKHKIEPILKARKQYAYGAQHDYFDHHDIVGWTREGDSSVANSGLAALIT
DGPGGAKRMYVGRQNAGETWHDITGNRSEPVVINSEGWGEFHVNGGSVSIYVQR

(SEQ ID NO: 16)

In embodiments, the enzyme is a cellulase. In embodiments, enzyme is a naturally occurring form, a homolog, an isoform or a variant of a cellulase that maintains the enzymatic activity of the cellulase (e.g., within at least 50%, 80%, 90%, 95%, 96%, 97%, 98%, 99% or 100% activity compared to the native protein). In embodiments, variants have at least 90%, 95%, 96%, 97%, 98%, 99% or 100% amino acid sequence identity across the whole sequence or a portion of the sequence *(e.g.* a 50, 100, 150 or 200 continuous amino acid portion) compared to a naturally occurring form. A non-limiting example of a DNA sequence that encodes a cellulase is as follows:

ATGAAGTTTCAGAGCACTTTGCTTCTTGCCGCCGCGGCTGGTTCCGCGTTGGCTGTG
CCTCATGGCTCCGGACATAAGAAGAGGGCGTCTGTGTTTGAATGGTTCGGATCGAAC
GAGTCTGGTGCTGAATTTGGGACCAATATCCCAGGCGTCTGGGGAACCGACTACATC
TTCCCCGACCCCTCGACCATCTCTACGTTGATTGGCAAGGGAATGAACTTCTTCCGC
GTCCAGTTCATGATGGAGAGGTTGCTTCCTGACTCGATGACTGGTTCATACGACGAG
GAGTATCTGGCCAACTTGACGACTGTGGTGAAAGCGGTCACGGATGGAGGCGCGCA
TGCGCTCATCGACCCTCATAACTATGGCAGATACAACGGGGAGATCATCTCCAGTAC
ATCGGATTTCCAGACTTTCTGGCAGAATCTGGCGGGCCAGTACAAAGATAACGACTT
GGTCATGTTTGATACCAACAACGAATACTACGACATGGACCAGGATCTCGTGCTGA
ATCTCAACCAAGCAGCCATTAACGGCATCCGCGCTGCAGGTGCAAGCCAGTACATTT
TCGTCGAAGGCAACTCCTGGACCGGAGCTTGGACATGGGTCGATGTCAACGATAAT
ATGAAGAATTTGACCGACCCAGAAGACAAGATCGTCTATGAAATGCACCAGTACCT
AGACTCCGACGGTTCCGGCACTTCGGAGACCTGTGTCTCCGGGACAATCGGAAAGG
AGCGGATCACTGATGCTACACAGTGGCTCAAGGACAATAAGAAGGTCGGCTTCATC

GGCGAATATGCCGGGGGGTCCAATGATGTGTGTCGGAGTGCCGTGTCCGGGATGCT
AGAGTACATGGCGAACAACACCGACGTATGGAAGGGTGCGTCGTGGTGGGCAGCCG
GGCCATGGTGGGGAGACTACATTTTCAGCCTGGAGCCCCCAGATGGAACTGCTTAC
ACGGGTATGCTGGATATCCTGGAGACGTATCTCTGA
(SEQ ID NO: 17)

A non-limiting example of a cellulase amino acid sequence is as follows:

MKFQSTLLLAAAAGSALAVPHGSGHKKRASVFEWFGSNESGAEFGTNIPGVWGTDYIFP
DPSTISTLIGKGMNFFRVQFMMERLLPDSMTGSYDEEYLANLTTVVKAVTDGGAHALID
PHNYGRYNGEIISSTSDFQTFWQNLAGQYKDNDLVMFDTNNEYYDMDQDLVLNLNQA
AINGIRAAGASQYIFVEGNSWTGAWTWVDVNDNMKNLTDPEDKIVYEMHQYLDSDGS
GTSETCVSGTIGKERITDATQWLKDNKKVGFIGEYAGGSNDVCRSAVSGMLEYMANNT
DVWKGASWWAAGPWWGDYIFSLEPPDGTAYTGMLDILETYL

(SEQ ID NO: 18)

In embodiments, the enzyme is proteinase K. In embodiments, the enzyme is a naturally occurring form, a homolog, an isoform or a variant of proteinase K that maintains the enzymatic activity of proteinase K (e.g., within at least 50%, 80%, 90%, 95%, 96%, 97%, 98%, 99% or 100% activity compared to the native protein). In embodiments, variants have at least 90%, 95%, 96%, 97%, 98%, 99% or 100% amino acid sequence identity across the whole sequence or a portion of the sequence *(e.g.* a 50, 100, 150 or 200 continuous amino acid portion) compared to a naturally occurring form. A non-limiting example of a DNA sequence that encodes proteinase K is as follows:

ATGCGTTTGTCTGTTCTTCTGAGTCTTCTTCCCCTCGCTCTCGGCGCTCCTGCCGTTGA
GCAGCGCTCCGAGGCTGCTCCTCTGATCGAGGCCCGCGGCGAGATGGTTGCCAACA
AGTACATTGTCAAGTTCAAGGAGGGTAGCGCTCTTTCTGCTCTCGATGCTGCCATGG
AGAAGATTTCTGGCAAGCCCGACCACGTCTACAAGAACGTCTTCAGTGGTTTCGCTG
CGACCCTTGACGAGAACATGGTTCGGGTTCTCCGCGCCCATCCCGATGTTGAGTACA
TTGAGCAGGATGCTGTTGTCACCATCAACGCTGCGCAGACCAACGCTCCCTGGGGCC
TTGCTCGCATCTCCAGCACCAGCCCCGGTACCTCTACTTACTACTATGACGAATCTG
CCGGCCAAGGCTCCTGCGTCTACGTGATTGACACCGGTATCGAGGCATCGCACCCCG
AGTTTGAGGGTCGTGCCCAGATGGTCAAGACCTACTACTACTCCAGTCGCGACGGTA
ACGGTCACGGCACTCACTGCGCTGGTACCGTTGGCTCCCGAACCTACGGTGTCGCCA

AGAAGACCCAGCTCTTTGGTGTCAAGGTCCTCGATGACAACGGCAGTGGCCAGTAC
TCCACCATCATCGCCGGTATGGACTTTGTTGCCAGCGACAAGAACAACCGCAACTGC
CCCAAAGGTGTCGTTGCCTCCTTGTCCCTTGGCGGTGGTTACTCCTCCTCCGTGAACA
GCGCCGCTGCCAGGCTCCAGAGCTCTGGTGTCATGGTCGCCGTCGCTGCCGGTAACA
ACAACGCTGACGCCCGCAACTACTCCCCTGCTTCTGAGCCCTCGGTCTGCACTGTCG
GTGCTTCTGACCGCTACGACAGACGCTCCAGCTTCTCCAACTACGGCAGCGTTTTGG
ACATCTTTGGCCCTGGTACCAGCATTCTCTCCACCTGGATCGGCGGCAGCACCCGCT
CCATCTCTGGAACTTCCATGGCTACTCCCCACGTTGCCGGTCTCGCTGCCTACCTCAT
GACTCTTGGAAAGACTACCGCCGCCAGCGCTTGCCGATACATTGCCGACACCGCCA
ACAAGGGCGACTTGAGCAACATTCCCTTCGGCACTGTCAACCTGCTTGCCTACAACA
ACTACCAGGCTTAA

(SEQ ID NO: 19)

A non-limiting example of a proteinase K amino acid sequence is as follows:

MRLSVLLSLLPLALGAPAVEQRSEAAPLIEARGEMVANKYIVKFKEGSALSALDAAMEK
ISGKPDHVYKNVFSGFAATLDENMVRVLRAHPDVEYIEQDAVVTINAAQTNAPWGLAR
ISSTSPGTSTYYYDESAGQGSCVYVIDTGIEASHPEFEGRAQMVKTYYYSSRDGNGHGT
HCAGTVGSRTYGVAKKTQLFGVKVLDDNGSGQYSTIIAGMDFVASDKNNRNCPKGVV
ASLSLGGGYSSSVNSAAARLQSSGVMVAVAAGNNNADARNYSPASEPSVCTVGASDRY
DRRSSFSNYGSVLDIFGPGTSILSTWIGGSTRSISGTSMATPHVAGLAAYLMTLGKTTAAS
ACRYIADTANKGDLSNIPFGTVNLLAYNNYQA

(SEQ ID NO: 20)

In embodiments, the enzyme is subtilisin. In embodiments, the enzyme is a naturally occurring form, a homolog, an isoform or a variant of subtilisin that maintains the enzymatic activity of subtilisin (e.g., within at least 50%, 80%, 90%, 95%, 96%, 97%, 98%, 99% or 100% activity compared to the native protein). In embodiments, variants have at least 90%, 95%, 96%, 97%, 98%, 99% or 100% amino acid sequence identity across the whole sequence or a portion of the sequence *(e.g.* a 50, 100, 150 or 200 continuous amino acid portion) compared to a naturally occurring form. A non-limiting example of a DNA sequence that encodes subtilisin is as follows:

ATGATGAGGAAAAAGAGTTTTTGGCTTGGGATGCTGACGGCCTTCATGCTCGTGTTC
ACGATGGCATTCAGCGATTCCGCTTCTGCTGCTCAACCGGCGAAAAATGTTGAAAAG

GATTATATTGTCGGATTTAAGTCAGGAGTGAAAACCGCATCTGTCAAAAAGGACAT
CATCAAAGAGAGCGGCGGAAAAGTGGACAAGCAGTTTAGAATCATCAACGCGGCA
AAAGCGAAGCTAGACAAAGAAGCGCTTAAGGAAGTCAAAAATGATCCGGATGTCG
CTTATGTGGAAGAGGATCATGTGGCCCATGCCTTGGCGCAAACCGTTCCTTACGGCA
TTCCTCTCATTAAAGCGGACAAAGTGCAGGCTCAAGGCTTTAAGGGAGCGAATGTA
AAAGTAGCCGTCCTGGATACAGGAATCCAAGCTTCTCATCCGGACTTGAACGTAGTC
GGCGGAGCAAGCTTTGTGGCTGGCGAAGCTTATAACACCGACGGCAACGGACACGG
CACACATGTTGCCGGTACAGTAGCTGCGCTTGACAATACAACGGGTGTATTAGGCGT
TGCGCCAAGCGTATCCTTGTACGCGGTTAAAGTACTGAATTCAAGCGGAAGCGGAA
CTTACAGCGGCATTGTAAGCGGAATCGAGTGGGCGACGACAAACGGCATGGATGTT
ATCAACATGAGTCTTGGAGGACCATCAGGCTCAACAGCGATGAAACAGGCGGTTGA
CAATGCATATGCAAGAGGGGTTGTCGTTGTGGCGGCTGCTGGGAACAGCGGATCTT
CAGGAAACACGAATACAATCGGCTATCCTGCGAAATACGACTCTGTCATCGCAGTT
GGCGCGGTAGACTCTAACAGCAACAGAGCTTCATTTCCAGCGTCGGAGCAGAGCT
TGAAGTCATGGCTCCTGGCGCAGGCGTGTACAGCACTTACCCAACCAGCACTTATGC
AACATTGAACGGAACGTCAATGGCTTCTCCTCATGTAGCGGGAGCAGCAGCTTTGAT
CTTGTCAAAACATCCGAACCTTTCAGCTTCACAAGTCCGCAACCGTCTCTCCAGTAC
GGCGACTTATTTGGGAAGCTCCTTCTACTATGGAAAAGGTCTGATCAATGTCGAAGC
TGCCGCTCAATAA

(SEQ ID NO: 21)

A non-limiting example of a subtilisin amino acid sequence is as follows:

MMRKKSFWLGMLTAFMLVFTMAFSDSASAAQPAKNVEKDYIVGFKSGVKTASVKKDII
KESGGKVDKQFRIINAAKAKLDKEALKEVKNDPDVAYVEEDHVAHALAQTVPYGIPLI
KADKVQAQGFKGANVKVAVLDTGIQASHPDLNVVGGASFVAGEAYNTDGNGHGTHV
AGTVAALDNTTGVLGVAPSVSLYAVKVLNSSGSGSYSGIVSGIEWATTNGMDVINMSL
GGASGSTAMKQAVDNAYAKGVVVVAAAGNSGSSGNTNTIGYPAKYDSVIAVGAVDSN
SNRASFSSVGAELEVMAPGAGVYSTYPTNTYATLNGTSMASPHVAGAAALILSKHPNLS
ASQVRNRLSSTATYLGSSFYYGKGLINVEAAAQ

(SEQ ID NO: 22)

In embodiments, the enzyme is trypsin. In embodiments, the enzyme is a naturally occurring form, a homolog, an isoform or a variant of trypsin that maintains the enzymatic activity of trypsin (e.g., within at least 50%, 80%, 90%, 95%, 96%, 97%, 98%, 99% or 100% activity compared to the native protein). In embodiments, variants have at least 90%, 95%, 96%, 97%, 98%, 99% or 100% amino acid sequence identity across the whole sequence or a portion of the sequence *(e.g.* a 50, 100, 150 or 200 continuous amino acid portion) compared to a naturally occurring form. A non-limiting example of a DNA sequence that encodes trypsin is as follows:

ATCGTCGGGGGCTACACCTGCGCAGAGAATTCCGTCCCTTACCAGGTGTCCCTGAAT
GCTGGCTACCACTTCTGCGGGGGCTCCCTCATCAATGACCAGTGGGTGGTGTCCGCG
GCTCACTGCTACCAGTACCACATCCAGGTGAGGCTGGGAGAATACAACATTGATGT
CTTGGAGGGTGGTGAGCAGTTCATCGATGCGTCCAAGATCATCCGCCACCCCAAGTA
CAGCAGCTGGACTCTGGACAATGACATCCTGCTGATCAAACTCTCCACGCCTGCGGT
CATCAATGCCCGGGTGTCCACCTTGCTGCTGCCCAGTGCCTGTGCTTCCGCAGGCAC
AGAGTGCCTCATCTCCGGCTGGGGCAACACCCTGAGCAGTGGCGTCAACTACCCGG
ACCTGCTGCAATGCCTGGTGGCCCCGCTGCTGAGCCACGCCGACTGTGAAGCCTCAT
ACCCTGGACAGATCACTAACAACATGATCTGCGCTGGCTTCCTGGAAGGAGGCAAG
GATTCCTGCCAGGGTGACTCTGGCGGCCCTGTGGCTTGCAACGGACAGCTCCAGGGC
ATTGTGTCCTGGGGCTACGGCTGTGCCCAGAAGGGCAAGCCTGGGGTCTACACCAA
GGTCTGCAACTACGTGGACTGGATTCAGGAGACCATCGCCGCCAAC

(SEQ ID NO: 23)

A non-limiting example of a trypsin amino acid sequence is as follows:

IVGGYTCGANTVPYQVSLNSGYHFCGGSLINSQWVVSAAHCYKSGIQVRLGEDNINVVE
GNEQFISASKSIVHPSYNSNTLNNDIMLIKLKSAASLNSRVASISLPTSCASAGTQCLISGW
GNTKSSGTSYPDVLKCLKAPILSDSSCKSAYPGQITSNMFCAGYLEGGKDSCQGDSGGP
VVCSGKLQGIVSWGSGCAQKNKPGVYTKVCNYVSWIKQTIASN
(SEQ ID NO: 24)

In embodiments, the enzyme is serratiopeptidase. In embodiments, the enzyme is a naturally occurring form, a homolog, an isoform or a variant of serratiopeptidase that maintains the enzymatic activity of serratiopeptidase (e.g., within at least 50%, 80%, 90%, 95%, 96%, 97%, 98%, 99% or 100% activity compared to the native protein). In embodiments, variants have at least 90%, 95%, 96%, 97%, 98%, 99% or 100% amino acid sequence identity across the whole sequence or a portion of the sequence *(e.g.* a 50, 100, 150 or 200 continuous amino acid portion) compared to a naturally occurring form. A non-limiting example of a DNA sequence that encodes serratiopeptidase is as follows:

ATGCAATCTACTAAAAAGGCAATTGAAATTACTGAATCCAGCCTCGCTGCCGCGAC
AACCGGTTACGATGCTGTAGACGACCTGCTGCATTATCATGAGCGGGGTAACGGGA
TTCAGATTAATGGCAAGGATTCATTTTCTAACGAGCAAGCTGGGCTGTTTATTACCC
GTGAGAACCAAACCTGGAACGGTTACAAGGTATTTGGCCAGCCGGTCAAATTAACC
TTCTCGTTCCCGGACTATAAGTTCTCTTCCACCAACGTCGCCGGCGACACCGGGCTG
AGCAAGTTCAGCGCGGAACAGCAGCAGGCTAAGCTGTCGCTGCAGTCCTGGGC
CGACGTCGCCAATATCACCTTCACCGAAGTGGCGGCCGGTCAAAAGGCCAATATCA
CCTTCGGCAACTACAGCCAGGATCGTCCCGGCCACTATGATTACGGCACCCAGGCCT
ACGCCTTCCTGCCGAACACCATTTGGCAGGGCCAGGATTTGGGCGGCCAGACTTGGT
ACAACGTAAACCAATCCAACGTGAAGCATCCGGCGACCGAAGACTACGGCCGCCAG
ACGTTCACCCATGAGATTGGCCATGCGCTGGGCCTGAGCCACCCGGGCGACTACAA
CGCCGGTGAGGGCAACCCGACCTATAGAGATGTCACCTATGCGGAAGATACCCGCC
AGTTCAGCCTGATGAGCTACTGGAGTGAAACCAATACCGGTGGCGACAACGGCGGT
CACTATGCCGCGGCTCCGCTGCTGGATGACATTGCCGCCATTCAGCATCTGTATGGC
GCCAACCTGTCGACCCGCACCGGCGACACCGTGTACGGCTTTAACTCCAATACCGGT
CGTGACTTCCTCAGCACCACCAGCAACTCGCAGAAAGTGATCTTTGCGGCCTGGGAT
GCGGGCGGCAACGATACCTTCGACTTCTCCGGTTACACCGCTAACCAGCGCATCAAC
CTGAACGAGAAATGGTTCTCCGACGTGGGCGGCCTGAAGGGCAACGTGTCGATCGC
CGCCGGTGTGACCATTGAGAACGCCATTGGCGGTTCCGGCAACGACGTGATCGTCG
GCAACGCGGCCAATAACGTGCTGAAAGGCGGCGCGGGTAACGACGTGCTGTTCGGC
GGCGGCGGGGCGGATGAATTGTGGGGCGGTGCCGGCAAAGACATCTTCGTGTTCTC
TGCCGCCAGCGATTCCGCACCGGGCGCTTCAGACTGGATCCGCGACTTCCAGAAAG
GGATCGACAAGATCGACCTGTCGTTCTTCAATAAAGAAGCGCAGAGCAGCGATTTC
ATTCACTTCGTCGATCACTTCAGCGGCACGGCCGGTGAGGCGCTGCTGAGCTACAAC
GCGTCCAGCAACGTGACCGATTTGTCGGTGAACATCGGTGGGCATCAGGCGCCGGA
CTTCCTGGTGAAAATCGTCGGCCAGGTAGACGTCGCCACGGACTTTATCGTGTAA

(SEQ ID NO: 25)

A non-limiting example of a serratiopeptidase amino acid sequence is as follows:

MQSTKKAIEITESSLAAATTGYDAVDDLLHYHERGNGIQINGKDSFSNEQAGLFITRENQ
TWNGYKVFGQPVKLTFSFPDYKFSSTNVAGDTGLSKFSAEQQQQAKLSLQSWADVANI

TFTEVAAGQKANITFGNYSQDRPGHYDYGTQAYAFLPNTIWQGQDLGGQTWYNVNQS
NVKHPATEDYGRQTFTHEIGHALGLSHPGDYNAGEGNPTYRDVTYAEDTRQFSLMSYW
SETNTGGDNGGHYAAAPLLDDIAAIQHLYGANLSTRTGDTVYGFNSNTGRDFLSTTSNS
QKVIFAAWDAGGNDTFDFSGYTANQRINLNEKWFSDVGGLKGNVSIAAGVTIENAIGGS
GNDVIVGNAANNVLKGGAGNDVLFGGGGADELWGGAGKDIFVFSAASDSAPGASDWI
RDFQKGIDKIDLSFFNKEAQSSDFIHFVDHFSGTAGEALLSYNASSNVTDLSVNIGGHQA
PDFLVKIVGQVDVATDFIV

(SEQ ID NO: 26)

In embodiments, the enzyme is a DNAse. In embodiments, the enzyme is a naturally occurring form, a homolog, an isoform or a variant of a DNAse that maintains the enzymatic activity of the DNAse (e.g., within at least 50%, 80%, 90%, 95%, 96%, 97%, 98%, 99% or 100% activity compared to the native protein). In embodiments, variants have at least 90%, 95%, 96%, 97%, 98%, 99% or 100% amino acid sequence identity across the whole sequence or a portion of the sequence *(e.g.* a 50, 100, 150 or 200 continuous amino acid portion) compared to a naturally occurring form. In embodiments, the enzyme is a DNAse I. In embodiments, the DNAse I is bovine pancreatic DNAse I. A non-limiting example of a DNA sequence that encodes a bovine pancreatic DNAse I is as follows:

TTGAAGATTGCTGCTTTCAACATTAGAACTTTCGGTGAAACTAAAATGTCTAACGCT
ACTTTGGCATCTTACATCGTTAGAATTGTCAGAAGATATGATATCGTTTTAATTCAA
GAAGTTAGAGACTCTCACTTGGTTGCAGTTGGTAAATTGTTAGACTACTTGAACCAA
GATGACCCAAACACTTACCACTACGTTGTTTCTGAACCATTGGGTAGAAACTCTTAC
AAAGAAAGATACTTATTCTTGTTCAGACCAAACAAAGTTTCAGTTTTGGATACTTAC
CAATACGACGACGGTTGCGAATCTTGTGGTAACGATTCTTTCTCCAGAGAACCTGCT
GTTGTTAAATTCTCATCACACTCTACCAAGGTTAAAGAGTTCGCTATCGTTGCTTTGC
ATTCTGCTCCTTCTGACGCTGTTGCTGAAATTAACTCTTTGTACGACGTTTACTTAGA
TGTTCAACAGAAATGGCACTTGAACGACGTCATGTTGATGGGTGACTTTAACGCTGA
TTGCTCTTATGTTACTTCTTCTCAATGGTCTTCAATTAGATTGAGAACATCTTCAACT
TTCCAATGGTTAATTCCTGATTCCGCTGATACCACTGCTACTAGTACCAACTGTGCTT
ACGATAGAATCGTTGTTGCTGGATCATTATTGCAATCTTCTGTTGTCCCAGGTTCAGC
GGCCCCTTTCGATTTCCAAGCTGCATATGGTTTGTCTAATGAAATGGCTTTAGCCATT
TCTGATCACTACCCAGTTGAAGTCACATTGACATAA

(SEQ ID NO: 27)

A non-limiting example of a bovine pancreatic DNAse I amino acid sequence is as follows:

LKIAAFNIRTFGETKMSNATLASYIVRIVRRYDIVLIQEVRDSHLVAVGKLLDYLNQDDP
NTYHYVVSEPLGRNSYKERYLFLFRPNKVSVLDTYQYDDGCESCGNDSFSREPAVVKFS
SHSTKVKEFAIVALHSAPSDAVAEINSLYDVYLDVQQKWHLNDVMLMGDFNADCSYV
TSSQWSSIRLRTSSTFQWLIPDSADTTATSTNCAYDRIVVAGSLLQSSVVPGSAAPFDFQA
AYGLSNEMALAISDHYPVEVTLT

(SEQ ID NO: 28)

In embodiments, the composition or combination includes a probiotic bacterium.

In embodiments, the probiotic bacterium is a probiotic a *P.* sp., *Staphylococcus* sp., and/or *Corynebacterium* sp. bacterium.

In embodiments, the probiotic bacterium is a bacterium within the class *Betaproteobacteria.*

In embodiments, the probiotic bacterium is a probiotic *P. acnes* bacterium.

In embodiments, the *P. acnes* bacterium (a) has a 16S ribosomal DNA (rDNA) sequence with a T992C mutation compared to the KPA171202 type strain 16S rDNA sequence set forth as SEQ ID NO: 2; (b) has a 16S rDNA sequence with a T838C mutation compared to the KPA171202 type strain 16S rDNA sequence set forth as SEQ ID NO: 2; (c) has a 16S rDNA sequence with a C1322T mutation compared to the KPA171202 type strain 16S rDNA sequence set forth as SEQ ID NO: 2; (d) has a 16S rDNA sequence with a C986T mutation compared to the KPA171202 type strain 16S rDNA sequence set forth as SEQ ID NO: 2; (e) has a 16S rDNA sequence that is identical to the sequence of SEQ ID NO: 3; (f) has a 16S rDNA sequence that is identical to the sequence of SEQ ID NO: 4; (g) does not comprise a linear plasmid; (h) does not comprise a plasmid that has a virulence factor; and/or (i) does not have a plasmid that encodes an extrachromosomal lipase and/or a tight adhesion virulence factor.

In embodiments, the *P. acnes* bacterium (a) produces less than about 1%, 5%, 10%, 15%, 20%, 25%, 30%, 35%, 40%, 45%, 50%, 55%, 60%, 65%, 70%, 75%, 80%, 85%, 90% or 95% of the level of lipase that is produced by a pathogenic *P. acnes* strain when grown in a planktonic culture; (b) produces less than about 1%, 5%, 10%, 15%, 20%, 25%, 30%, 35%, 40%, 45%, 50%, 55%, 60%, 65%, 70%, 75%, 80%, 85%, 90% or 95% of the level of lipase that is produced by a pathogenic *P. acnes* strain when grown in an adherent culture; (c) adheres to epithelial cells at least 50% less than a pathogenic *P. acnes* strain; and/or (d) is less inflammatory than a pathogenic *P. acnes* strain.

In embodiments, the combination or composition includes at least one additional probiotic bacterium. In embodiments, the at least one additional probiotic bacterium includes *Propionibacterium granulosum* and/or *Propionibacterium avidum.*

In embodiments, a pathogenic *P. acnes* strain (a) has a 16S rDNA sequence with a G1058C mutation compared to the KPA171202 type strain 16S rDNA sequence set forth as SEQ ID NO: 2; (b) has a 16S rDNA sequence with a G1058C and an A1201C mutation compared to the KPA171202 type strain 16S rDNA sequence set forth as SEQ ID NO: 2; (c) has a 16S rDNA sequence with a G529A mutation compared to the KPA171202 type strain 16S rDNA sequence set forth as SEQ ID NO: 2; (d) has a 16S rDNA sequence with a G1004A and a T1007C mutation compared to the KPA171202 type strain 16S rDNA sequence set forth as SEQ ID NO: 2; (e) has a 16S rDNA sequence with a G1268A mutation compared to the KPA171202 type strain 16S rDNA sequence set forth as SEQ ID NO: 2; (f) has a 16S rDNA sequence with a T554C and a G1058C mutation compared to the KPA171202 type strain 16S rDNA sequence set forth as SEQ ID NO: 2; (g) has a 16S rDNA sequence that is identical to the sequence of SEQ ID NO: 5; (h) has a 16S rDNA sequence that is identical to the sequence of SEQ ID NO: 6; (i) has a 16S rDNA sequence that is identical to the sequence of SEQ ID NO: 7; (j) has a 16S rDNA sequence that is identical to the sequence of SEQ ID NO: 8; (k) has a 16S rDNA sequence that is identical to the sequence of SEQ ID NO: 9; and/or (l) has a 16S rDNA sequence that is identical to the sequence of SEQ ID NO: 10.

In embodiments, the combination or composition further includes at least one additional *P. acnes* bacteriophage.

The composition or combination includes a pharmaceutically acceptable carrier. In embodiments, the pharmaceutically acceptable carrier includes an emulsion. In embodiments, the emulsion is an oil-in-water emulsion or a water-in-oil emulsion. In embodiments, a combination or combination includes or is in the form of a cream, lotion, suspension, or aqueous solution.

In embodiments, a composition that includes a bacteriophage is provided. In embodiments, the composition is formulated for topical application to the skin *(i.e.,* the composition is a topical composition). In embodiments, the composition is a pharmaceutical composition.

Also disclosed but not claimed is a pharmaceutical composition including a wild-type *P. acnes* bacteriophage and an isolated probiotic *P. acnes* bacterium. In embodiments, the composition further includes a pharmaceutically acceptable carrier.

Also disclosed but not claimed is a pharmaceutical composition including a bacteriophage and/or an isolated probiotic *P. acnes* bacterium and a pharmaceutically acceptable carrier.

In embodiments, the pharmaceutical composition is formulated for topical administration to the skin. In embodiments, the pharmaceutically acceptable carrier includes an emulsion. In embodiments, the emulsion is an oil-in-water emulsion or a water-in-oil emulsion. In embodiments, the pharmaceutical composition is in the form of a cream, lotion, suspension, or aqueous solution.

In embodiments, a composition or combination includes at least about 2, 3, 4, 5, 6, 7, 8, 9, or 10 *P. acnes* bacteriophages. In embodiments, the *P. acnes* bacteriophages include more than one type of *P. acnes* bacteriophage.

In embodiments, a combination or composition including an isolated probiotic *P*. *acnes* bacterium may further comprise at least one additional bacterium.

In embodiments, a *P. acnes* bacterium has a 16S rDNA sequence that includes a T992C, T838C, C1322T, and/or a C986T mutation compared to the KPA171202 type strain 16S rDNA sequence set forth as SEQ ID NO: 2. In embodiments, the *P. acnes* bacterium includes a 16S rDNA sequence with a T838C and a C1322T mutation compared to the KPA171202 type strain 16S rDNA sequence set forth as SEQ ID NO: 2. In embodiments, the *P. acnes* bacterium is the ProI strain. In embodiments, the *P. acnes* bacterium includes a 16S rDNA sequence with a C986T and a T992C mutation compared to the KPA171202 type strain 16S rDNA sequence set forth as SEQ ID NO: 2. In embodiments, the *P. acnes* bacterium is the ProII strain. In embodiments, the *P. acnes* bacterium: (a) includes a 16S rDNA sequence with a T992C mutation compared to the KPA171202 type strain 16S rDNA sequence set forth as SEQ ID NO: 2; (b) includes a 16S rDNA sequence with a T838C mutation compared to the KPA171202 type strain 16S rDNA sequence set forth as SEQ ID NO: 2; (c) includes a 16S rDNA sequence with a C1322T mutation compared to the KPA171202 type strain 16S rDNA sequence set forth as SEQ ID NO: 2; (d) includes a 16S rDNA sequence with a C986T mutation compared to the KPA171202 type strain 16S rDNA sequence set forth as SEQ ID NO: 2; (e) includes a 16S rDNA sequence that is identical to the sequence of SEQ ID NO: 3; (f) includes a 16S rDNA sequence that is identical to the sequence of SEQ ID NO: 4; (g) does not comprise a linear plasmid; (h) does not include a plasmid that includes a virulence factor; and/or (i) does not include a plasmid that encodes an extrachromosomal lipase and/or a tight adhesion virulence factor. In embodiments, the *P. acnes* bacterium has a 16S rDNA sequence that is identical to the sequence of SEQ ID NO: 3 or 4.

In embodiments, the *P. acnes* bacterium: (a) produces less than about 1%, 5%, 10%, 15%, 20%, 25%, 30%, 35%, 40%, 45%, 50%, 55%, 60%, 65%, 70%, 75%, 80%, 85%, 90% or 95% of the level of lipase that is produced by a pathogenic *P. acnes* strain when grown in a planktonic culture; (b) produces less than about 1%, 5%, 10%, 15%, 20%, 25%, 30%, 35%, 40%, 45%, 50%, 55%, 60%, 65%, 70%, 75%, 80%, 85%, 90% or 95% of the level of lipase that is produced by a pathogenic *P. acnes* strain when grown in an adherent culture; (c) adheres to epithelial cells at least 50% less than a pathogenic *P. acnes* strain; and/or (d) is less inflammatory than a pathogenic *P. acnes* strain. In embodiments, the pathogenic *P. acnes* strain (a) has a 16S rDNA sequence with a G1058C mutation compared to the KPA171202 type strain 16S rDNA sequence set forth as SEQ ID NO: 2; (b) has a 16S rDNA sequence with a G1058C and an A1201C mutation compared to the KPA171202 type strain 16S rDNA sequence set forth as SEQ ID NO: 2; (c) has a 16S rDNA sequence with a G529A mutation compared to the KPA171202 type strain 16S rDNA sequence set forth as SEQ ID NO: 2; (d) has a 16S rDNA sequence with a G1004A and a T1007C mutation compared to the KPA171202 type strain 16S rDNA sequence set forth as SEQ ID NO: 2; (e) has a 16S rDNA sequence with a G1268A mutation compared to the KPA171202 type strain 16S rDNA sequence set forth as SEQ ID NO: 2; (f) has a 16S rDNA sequence with a T554C and a G1058C mutation compared to the KPA171202 type strain 16S rDNA sequence set forth as SEQ ID NO: 2; (g) has a 16S rDNA sequence that is identical to the sequence of SEQ ID NO: 5; (h) has a 16S rDNA sequence that is identical to the sequence of SEQ ID NO: 6; (i) has a 16S rDNA sequence that is identical to the sequence of SEQ ID NO: 7; (j) has a 16S rDNA sequence that is identical to the sequence of SEQ ID NO: 8; (k) has a 16S rDNA sequence that is identical to the sequence of SEQ ID NO: 9; and/or (l) has a 16S rDNA sequence that is identical to the sequence of SEQ ID NO: 10.

SEQ ID NO: 2 is the 16S rDNA sequence for the KPA171202 type strain, and is as follows:

1 TTTTTCATTG GAGAGTTTGA TCCTGGCTCA GGACGAACGC TGGCGGCGTG CTTAACACAT
61 GCAAGTCGAA CGGAAAGGCC CTGCTTTTGT GGGGTGCTCG AGTGGCGAAC GGGTGAGTAA
121 CACGTGAGTA ACCTGCCCTT GACTTTGGGA TAACTTCAGG AAACTGGGGC TAATACCGGA
181 TAGGAGCTCC TGCTGCATGG TGGGGGTTGG AAAGTTTCGG CGGTTGGGGA TGGACTCGCG
241 GCTTATCAGC TTGTTGGTGG GGTAGTGGCT TACCAAGGCT TTGACGGGTA GCCGGCCTGA
301 GAGGGTGACC GGCCACATTG GGACTGAGAT ACGGCCCAGA CTCCTACGGG AGGCAGCAGT
361 GGGGAATATT GCACAATGGG CGGAAGCCTG ATGCAGCAAC GCCGCGTGCG GGATGACGGC
421 CTTCGGGTTG TAAACCGCTT TCGCCTGTGA CGAAGCGTGA GTGACGGTAA TGGGTAAAGA
481 AGCACCGGCT AACTACGTGC CAGCAGCCGC GGTGATACGT AGGGTGCGAG CGTTGTCCGG
541 ATTTATTGGG CGTAAAGGGC TCGTAGGTGG TTGATCGCGT CGGAAGTGTA ATCTTGGGGC
601 TTAACCCTGA GCGTGCTTTC GATACGGGTT GACTTGAGGA AGGTAGGGGA GAATGGAATT
661 CCTGGTGGAG CGGTGGAATG CGCAGATATC AGGAGGAACA CCAGTGGCGA AGGCGGTTCT
721 CTGGGCCTTT CCTGACGCTG AGGAGCGAAA GCGTGGGGAG CGAACAGGCT TAGATACCCT
781 GGTAGTCCAC GCTGTAAACG GTGGGTACTA GGTGTGGGGT CCATTCCACG GGTTCCGTGC
841 CGTAGCTAAC GCTTTAAGTA CCCCGCCTGG GGAGTACGGC CGCAAGGCTA AAACTCAAAG
901 GAATTGACGG GGCCCCGCAC AAGCGGCGGA GCATGCGGAT TAATTCGATG CAACGCGTAG
961 AACCTTACCT GGGTTTGACA TGGATCGGGA GTGCTCAGAG ATGGGTGTGC CTCTTTTGGG
1021 GTCGGTTCAC AGGTGGTGCA TGGCTGTCGT CAGCTCGTGT CGTGAGATGT TGGGTTAAGT
1081 CCCGCAACGA GCGCAACCCT TGTTCACTGT TGCCAGCACG TTATGGTGGG GACTCAGTGG
1141 AGACCGCCGG GGTCAACTCG GAGGAAGGTG GGGATGACGT CAAGTCATCA TGCCCCTTAT
1201 GTCCAGGGCT TCACGCATGC TACAATGGCT GGTACAGAGA GTGGCGAGCC TGTGAGGGTG
1261 AGCGAATCTC GGAAAGCCGG TCTCAGTTCG GATTGGGGTC TGCAACTCGA CCTCATGAAG
1321 TCGGAGTCGC TAGTAATCGC AGATCAGCAA CGCTGCGGTG AATACGTTCC CGGGGCTTGT
1381 ACACACCGCC CGTCAAGTCA TGAAAGTTGG TAACACCCGA AGCCGGTGGC CTAACCGTTG
1441 TGGGGGAGCC GTCGAAGGTG GGACTGGTGA TTAGGACTAA GTCGTAACAA GGTAGCCGTA
1501 CCGGAAGGTG CGGCTGGATC ACCTCCTTTC TAAGGAG

SEQ ID NO: 3 is the 16S rDNA sequence for the ProI probiotic strain, and is as follows:
Nucleotides 838..838
   ProI Mutation T838C
Nucleotides 1322..1322
   ProI Mutation C1322T

1 TTTTTCATTG GAGAGTTTGA TCCTGGCTCA GGACGAACGC TGGCGGCGTG CTTAACACAT
61 GCAAGTCGAA CGGAAAGGCC CTGCTTTTGT GGGGTGCTCG AGTGGCGAAC GGGTGAGTAA
121 CACGTGAGTA ACCTGCCCTT GACTTTGGGA TAACTTCAGG AAACTGGGGC TAATACCGGA

181 TAGGAGCTCC TGCTGCATGG TGGGGGTTGG AAAGTTTCGG CGGTTGGGGA TGGACTCGCG

241 GCTTATCAGC TTGTTGGTGG GGTAGTGGCT TACCAAGGCT TTGACGGGTA GCCGGCCTGA

301 GAGGGTGACC GGCCACATTG GGACTGAGAT ACGGCCCAGA CTCCTACGGG AGGCAGCAGT

361 GGGGAATATT GCACAATGGG CGGAAGCCTG ATGCAGCAAC GCCGCGTGCG GGATGACGGC

421 CTTCGGGTTG TAAACCGCTT TCGCCTGTGA CGAAGCGTGA GTGACGGTAA TGGGTAAAGA

481 AGCACCGGCT AACTACGTGC CAGCAGCCGC GGTGATACGT AGGGTGCGAG CGTTGTCCGG

541 ATTTATTGGG CGTAAAGGGC TCGTAGGTGG TTGATCGCGT CGGAAGTGTA ATCTTGGGGC

601 TTAACCCTGA GCGTGCTTTC GATACGGGTT GACTTGAGGA AGGTAGGGGA GAATGGAATT

661 CCTGGTGGAG CGGTGGAATG CGCAGATATC AGGAGGAACA CCAGTGGCGA AGGCGGTTCT

721 CTGGGCCTTT CCTGACGCTG AGGAGCGAAA GCGTGGGGAG CGAACAGGCT TAGATACCCT

781 GGTAGTCCAC GCTGTAAACG GTGGGTACTA GGTGTGGGGT CCATTCCACG GGTTCCGCGC

841 CGTAGCTAAC GCTTTAAGTA CCCCGCCTGG GGAGTACGGC CGCAAGGCTA AAACTCAAAG

901 GAATTGACGG GGCCCCGCAC AAGCGGCGGA GCATGCGGAT TAATTCGATG CAACGCGTAG

961 AACCTTACCT GGGTTTGACA TGGATCGGGA GTGCTCAGAG ATGGGTGTGC CTCTTTTGGG

1021 GTCGGTTCAC AGGTGGTGCA TGGCTGTCGT CAGCTCGTGT CGTGAGATGT TGGGTTAAGT

1081 CCCGCAACGA GCGCAACCCT TGTTCACTGT TGCCAGCACG TTATGGTGGG GACTCAGTGG

1141 AGACCGCCGG GGTCAACTCG GAGGAAGGTG GGGATGACGT CAAGTCATCA TGCCCCTTAT

1201 GTCCAGGGCT TCACGCATGC TACAATGGCT GGTACAGAGA GTGGCGAGCC TGTGAGGGTG

1261 AGCGAATCTC GGAAAGCCGG TCTCAGTTCG GATTGGGGTC TGCAACTCGA CCTCATGAAG

1321 TTGGAGTCGC TAGTAATCGC AGATCAGCAA CGCTGCGGTG AATACGTTCC CGGGGCTTGT

1381 ACACACCGCC CGTCAAGTCA TGAAAGTTGG TAACACCCGA AGCCGGTGGC CTAACCGTTG

1441 TGGGGGAGCC GTCGAAGGTG GGACTGGTGA TTAGGACTAA GTCGTAACAA GGTAGCCGTA

1501 CCGGAAGGTG CGGCTGGATC ACCTCCTTTC TAAGGAG

SEQ ID NO: 4 is the 16S rDNA sequence for the ProII probiotic strain, and is as follows:
Nucleotides 986..986
   ProII Mutation C986T
Nucleotides 992..992
   ProII Mutation T992C

1 TTTTTCATTG GAGAGTTTGA TCCTGGCTCA GGACGAACGC TGGCGGCGTG CTTAACACAT
61 GCAAGTCGAA CGGAAAGGCC CTGCTTTTGT GGGGTGCTCG AGTGGCGAAC GGGTGAGTAA
121 CACGTGAGTA ACCTGCCCTT GACTTTGGGA TAACTTCAGG AAACTGGGGC TAATACCGGA
181 TAGGAGCTCC TGCTGCATGG TGGGGGTTGG AAAGTTTCGG CGGTTGGGGA TGGACTCGCG
241 GCTTATCAGC TTGTTGGTGG GGTAGTGGCT TACCAAGGCT TTGACGGGTA GCCGGCCTGA
301 GAGGGTGACC GGCCACATTG GGACTGAGAT ACGGCCCAGA CTCCTACGGG AGGCAGCAGT

361 GGGGAATATT GCACAATGGG CGGAAGCCTG ATGCAGCAAC GCCGCGTGCG GGATGACGGC

421 CTTCGGGTTG TAAACCGCTT TCGCCTGTGA CGAAGCGTGA GTGACGGTAA TGGGTAAAGA

481 AGCACCGGCT AACTACGTGC CAGCAGCCGC GGTGATACGT AGGGTGCGAG CGTTGTCCGG

541 ATTTATTGGG CGTAAAGGGC TCGTAGGTGG TTGATCGCGT CGGAAGTGTA ATCTTGGGGC

601 TTAACCCTGA GCGTGCTTTC GATACGGGTT GACTTGAGGA AGGTAGGGGA GAATGGAATT

661 CCTGGTGGAG CGGTGGAATG CGCAGATATC AGGAGGAACA CCAGTGGCGA AGGCGGTTCT

721 CTGGGCCTTT CCTGACGCTG AGGAGCGAAA GCGTGGGGAG CGAACAGGCT TAGATACCCT

781 GGTAGTCCAC GCTGTAAACG GTGGGTACTA GGTGTGGGGT CCATTCCACG GGTTCCGTGC

841 CGTAGCTAAC GCTTTAAGTA CCCCGCCTGG GGAGTACGGC CGCAAGGCTA AAACTCAAAG

901 GAATTGACGG GGCCCCGCAC AAGCGGCGGA GCATGCGGAT TAATTCGATG CAACGCGTAG

961 AACCTTACCT GGGTTTGACA TGGATTGGGA GCGCTCAGAG ATGGGTGTGC CTCTTTTGGG

1021 GTCGGTTCAC AGGTGGTGCA TGGCTGTCGT CAGCTCGTGT CGTGAGATGT TGGGTTAAGT

1081 CCCGCAACGA GCGCAACCCT TGTTCACTGT TGCCAGCACG TTATGGTGGG GACTCAGTGG

1141 AGACCGCCGG GGTCAACTCG GAGGAAGGTG GGGATGACGT CAAGTCATCA TGCCCCTTAT

1201 GTCCAGGGCT TCACGCATGC TACAATGGCT GGTACAGAGA GTGGCGAGCC TGTGAGGGTG

1261 AGCGAATCTC GGAAAGCCGG TCTCAGTTCG GATTGGGGTC TGCAACTCGA CCTCATGAAG

1321 TCGGAGTCGC TAGTAATCGC AGATCAGCAA CGCTGCGGTG AATACGTTCC CGGGGCTTGT

1381 ACACACCGCC CGTCAAGTCA TGAAAGTTGG TAACACCCGA AGCCGGTGGC CTAACCGTTG

1441 TGGGGGAGCC GTCGAAGGTG GGACTGGTGA TTAGGACTAA GTCGTAACAA GGTAGCCGTA

1501 CCGGAAGGTG CGGCTGGATC ACCTCCTTTC TAAGGAG

In embodiments, the *P. acnes* bacterium produces less than about 1%, 5%, 10%, 15%, 20%, 25%, 30%, 35%, 40%, 45%, 50%, 55%, 60%, 65%, 70%, 75%, 80%, 85%, 90% or 95% of the level of lipase that is produced by a pathogenic *P. acnes* strain when grown in a planktonic culture. In embodiments, the *P. acnes* bacterium produces about 1-5%, 1-10%, 1-20%, 1-30%, 5-50%, 5-40%, 5-30%, 5-20%, 5-10%, 10-50%, 10-40%, 10-30%, 10-20%, 20-50%, 20-40%, or 20-30% of the level of lipase that is produced by a pathogenic *P. acnes* strain when grown in a planktonic culture. In embodiments, the *P. acnes* bacterium produces less than about 5% of the level of lipase that is produced by a pathogenic *P. acnes* strain when grown in a planktonic culture. In embodiments, the *P. acnes* bacterium produces less than about 10% of the level of lipase that is produced by a pathogenic *P. acnes* strain when grown in a planktonic culture. In embodiments, the *P. acnes* bacterium produces less than about 20% of the level of lipase that is produced by a pathogenic *P. acnes* strain when grown in a planktonic culture. In embodiments, the *P. acnes* bacterium produces less than about 30% of the level of lipase that is produced by a pathogenic *P. acnes* strain when grown in a planktonic culture. In embodiments, the *P. acnes* bacterium produces less than about 40% of the level of lipase that is produced by a pathogenic *P*. *acnes* strain when grown in a planktonic culture. In embodiments, the *P. acnes* bacterium produces less than about 50% of the level of lipase that is produced by a pathogenic *P. acnes* strain when grown in a planktonic culture. In embodiments, the *P. acnes* bacterium produces less than about 1%, 5%, 10%, 15%, 20%, 25%, 30%, 35%, 40%, 45%, 50%, 55%, 60%, 65%, 70%, 75%, 80%, 85%, 90% or 95% of the level of lipase that is produced by a pathogenic *P*. *acnes* strain when grown in an adherent culture. In embodiments, the *P. acnes* bacterium produces less than about 5% of the level of lipase that is produced by a pathogenic *P. acnes* strain when grown in an adherent culture. In embodiments, the *P. acnes* bacterium produces less than about 10% of the level of lipase that is produced by a pathogenic *P. acnes* strain when grown in an adherent culture. In embodiments, the *P. acnes* bacterium produces less than about 20% of the level of lipase that is produced by a pathogenic *P. acnes* strain when grown in an adherent culture. In embodiments, the *P. acnes* bacterium produces less than about 30% of the level of lipase that is produced by a pathogenic *P. acnes* strain when grown in an adherent culture. In embodiments, the *P. acnes* bacterium produces less than about 40% of the level of lipase that is produced by a pathogenic *P. acnes* strain when grown in an adherent culture. In embodiments, the *P. acnes* bacterium produces less than about 50% of the level of lipase that is produced by a pathogenic *P. acnes* strain when grown in an adherent culture. In embodiments, the *P. acnes* bacterium produces about 1-5%, 1-10%, 1-20%, 1-30%, 5-50%, 5-40%, 5-30%, 5-20%, 5-10%, 10-50%, 10-40%, 10-30%, 10-20%, 20-50%, 20-40%, or 20-30% of the level of lipase that is produced by a pathogenic *P. acnes* strain when grown in an adherent culture. In embodiments, the lipase is extracellular lipase.

In embodiments, the level of lipase produced by a *P. acnes* bacterium *(e.g.,* a probiotic or a pathogenic *P. acnes* bacterium, such as for comparison) is the level of lipase in culture supernatant. In embodiments, the culture supernatant is filtered. In embodiments, the culture supernatant is from a liquid (planktonic) culture. In embodiments, the culture supernatant is from an adherent culture. Non-limiting examples of methods for detecting a level of lipase include absorbance, Bradford protein assays, Biuret test derived assays, fluorescamine, amino black, colloidal gold, nitrogen detection, High-performance liquid chromatography (HPLC), Liquid chromatography-mass spectrometry (LC/MS), enzyme-linked immunosorbent assay (ELISA), protein immunoprecipitation, immunoelectrophoresis, and Western blot.

In embodiments, the *P. acnes* bacterium adheres to epithelial cells at least about 1%, 5%, 10%, 15%, 20%, 25%, 30%, 35%, 40%, 45%, 50%, 55%, 60%, 65%, 70%, 75%, 80%, 85%, 90% or 95% less than a pathogenic *P. acnes* strain. In embodiments, the *P. acnes* bacterium adheres to epithelial cells at least about 50% less than a pathogenic *P. acnes* strain. In embodiments, the *P. acnes* bacterium adheres to epithelial cells at least about 60% less than a pathogenic *P. acnes* strain. In embodiments, the *P. acnes* bacterium adheres to epithelial cells at least about 70% less than a pathogenic *P. acnes* strain. In embodiments, the *P. acnes* bacterium adheres to epithelial cells at least about 80% less than a pathogenic *P. acnes* strain. In embodiments, the *P. acnes* bacterium adheres to epithelial cells at least about 90% less than a pathogenic *P. acnes* strain. In embodiments, the *P. acnes* bacterium adheres to epithelial cells 1-5%, 1-10%, 1-20%, 1-30%, 5-50%, 5-40%, 5-30%, 5-20%, 5-10%, 10-50%, 10-40%, 10-30%, 10-20%, 20-50%, 20-40%, 20-30%, 50-60, 50-70, 50-80, 50-90, 60-80, 70-90 less than a pathogenic *P. acnes* strain.

In embodiments, adherence of a *P. acnes* bacterium *(e.g.,* a probiotic or a pathogenic *P*. *acnes* bacterium, such as for comparison) to epithelial cells is determined using A-432 epithelial cells. In embodiments, the epithelial cells are confluent on a tissue culture plate or flask. In embodiments, adherence is detected by determining a number of colonies that are formed by *P*. *acnes* bacteria that have adhered to cultured epithelial cells.

In embodiments, the *P. acnes* bacterium is less inflammatory than a pathogenic *P*. *acnes* strain.

In embodiments, a *P*. *acnes* bacterium is less inflammatory than a pathogenic *P. acnes* strain if a lower level of an inflammatory cytokine *(e.g.,* at least about 10%, 20%, 30%, 40%, 50%, 60%, 70%, 80%, or 90% less) is released by an immune cell that contacts the *P. acnes* bacterium or a compound produced by the *P. acnes* bacterium compared to a bacterium of the pathogenic *P. acnes* strain or a compound produced by the bacterium of the pathogenic *P. acnes* strain. In embodiments, a *P. acnes* bacterium is less inflammatory than a pathogenic *P. acnes* strain if a lower level of an inflammatory cytokine is released in tissue (such as skin tissue) that is contacted with *P. acnes* bacterium. In embodiments, the tissue is skin tissue. In embodiments, the tissue is ear tissue, *e.g.,* of a mouse. In embodiments, the inflammatory cytokine is IL-1β, IL-6, IL-17, or TNFα, or any combination thereof.

In embodiments, the pathogenic *P. acnes* strain (a) has a 16S rDNA sequence with a G1058C mutation compared to the KPA171202 type strain 16S rDNA sequence set forth as SEQ ID NO: 2; (b) has a 16S rDNA sequence with a G1058C and an A1201C mutation compared to the KPA171202 type strain 16S rDNA sequence set forth as SEQ ID NO: 2; (c) has a 16S rDNA sequence with a G529A mutation compared to the KPA171202 type strain 16S rDNA sequence set forth as SEQ ID NO: 2; (d) has a 16S rDNA sequence with a G1004A and a T1007C mutation compared to the KPA171202 type strain 16S rDNA sequence set forth as SEQ ID NO: 2; (e) has a 16S rDNA sequence with a G1268A mutation compared to the KPA171202 type strain 16S rDNA sequence set forth as SEQ ID NO: 2; (f) has a 16S rDNA sequence with a T554C and a G1058C mutation compared to the KPA171202 type strain 16S rDNA sequence set forth as SEQ ID NO: 2; (g) has a 16S rDNA sequence that is identical to the sequence of SEQ ID NO: 5; (h) has a 16S rDNA sequence that is identical to the sequence of SEQ ID NO: 6; (i) has a 16S rDNA sequence that is identical to the sequence of SEQ ID NO: 7; (j) has a 16S rDNA sequence that is identical to the sequence of SEQ ID NO: 8; (k) has a 16S rDNA sequence that is identical to the sequence of SEQ ID NO: 9; and/or (l) has a 16S rDNA sequence that is identical to the sequence of SEQ ID NO: 10.

SEQ ID NO: 5 is as follows (mutations compared to the 16S sequence of the type strain KPA171202 are underlined):

AGAGTTTGATCCTGGCTCAGGACGAACGCTGGCGGCGTGCTTAACACATGCAAGTC
GAACGGAAAGGCCCTGCTTTTGTGGGGTGCTCGAGTGGCGAACGGGTGAGTAACAC
GTGAGTAACCTGCCCTTGACTTTGGGATAACTTCAGGAAACTGGGGCTAATACCGGA
TAGGAGCTCCTGCTGCATGGTGGGGGTTGGAAAGTTTCGGCGGTTGGGGATGGACT
CGCGGCTTATCAGCTTGTTGGTGGGGTAGTGGCTTACCAAGGCTTTGACGGGTAGCC
GGCCTGAGAGGGTGACCGGCCACATTGGGACTGAGATACGGCCCAGACTCCTACGG
GAGGCAGCAGTGGGGAATATTGCACAATGGGCGGAAGCCTGATGCAGCAACGCCGC
GTGCGGGATGACGGCCTTCGGGTTGTAAACCGCTTTCGCCTGTGACGAAGCGTGAGT
GACGGTAATGGGTAAAGAAGCACCGGCTAACTACGTGCCAGCAGCCGCGGTGATAC
GTAGGGTGCGAGCGTTGTCCGGATTTATTGGGCGTAAAGGGCTCGTAGGTGGTTGAT
CGCGTCGGAAGTGTAATCTTGGGGCTTAACCCTGAGCGTGCTTTCGATACGGGTTGA
CTTGAGGAAGGTAGGGGAGAATGGAATTCCTGGTGGAGCGGTGGAATGCGCAGATA
TCAGGAGGAACACCAGTGGCGAAGGCGGTTCTCTGGGCCTTTCCTGACGCTGAGGA
GCGAAAGCGTGGGGAGCGAACAGGCTTAGATACCCTGGTAGTCCACGCTGTAAACG
GTGGGTACTAGGTGTGGGGTCCATTCCACGGGTTCCGTGCCGTAGCTAACGCTTTAA
GTACCCCGCCTGGGGAGTACGGCCGCAAGGCTAAAACTCAAAGGAATTGACGGGGC
CCCGCACAAGCGGCGGAGCATGCGGATTAATTCGATGCAACGCGTAGAACCTTACC
TGGGTTTGACATGGATCGGGAGTGCTCAGAGATGGGTGTGCCTCTTTTGGGGTCGGT
TCACAGGTGGTGCATGCCTGTCGTCAGCTCGTGTCGTGAGATGTTGGGTTAAGTCCC

GCAACGAGCGCAACCCTTGTTCACTGTTGCCAGCACGTTATGGTGGGGACTCAGTGG
AGACCGCCGGGGTCAACTCGGAGGAAGGTGGGGATGACGTCAAGTCCTCATGCCCC
TTATGTCCAGGGCTTCACGCATGCTACAATGGCTGGTACAGAGAGTGGCGAGCCTGT
GAGGGTGAGCGAATCTCGGAAAGCCGGTCTCAGTTCGGATTGGGGTCTGCAACTCG
ACCTCATGAAGTCGGAGTCGCTAGTAATCGCAGATCAGCAACGCTGCGGTGAATAC
GTTCCCGGGGCTTGTACACACCGCCCGTCAAGTCATGAAAGTTGGTAACACCCGAA
GCCGGTGGCCTAACCGTTGTGGGGGAGCCGTCGAAGGTGGGACTGGTGATTAGGAC
TAAGTCGTAACAAGGTAGCCGTACCGGAAGGTGCGGCTGGATCACCTCCTTTCTAAG
GA

SEQ ID NO: 6 is as follows (a mutation compared to the 16S sequence of the type strain KPA171202 is underlined):

AGAGTTTGATCCTGGCTCAGGACGAACGCTGGCGGCGTGCTTAACACATGCAAGTC
GAACGGAAAGGCCCTGCTTTTGTGGGGTGCTCGAGTGGCGAACGGGTGAGTAACAC
GTGAGTAACCTGCCCTTGACTTTGGGATAACTTCAGGAAACTGGGGCTAATACCGGA
TAGGAGCTCCTGCTGCATGGTGGGGGTTGGAAAGTTTCGGCGGTTGGGGATGGACT
CGCGGCTTATCAGCTTGTTGGTGGGGTAGTGGCTTACCAAGGCTTTGACGGGTAGCC
GGCCTGAGAGGGTGACCGGCCACATTGGGACTGAGATACGGCCCAGACTCCTACGG
GAGGCAGCAGTGGGGAATATTGCACAATGGGCGGAAGCCTGATGCAGCAACGCCGC
GTGCGGGATGACGGCCTTCGGGTTGTAAACCGCTTTCGCCTGTGACGAAGCGTGAGT
GACGGTAATGGGTAAAGAAGCACCGGCTAACTACGTGCCAGCAGCCGCGGTGATAC
GTAGGGTGCGAGCGTTGTCCGGATTTATTGGGCGTAAAGGGCTCGTAGGTGGTTGAT
CGCGTCGGAAGTGTAATCTTGGGGCTTAACCCTGAGCGTGCTTTCGATACGGGTTGA
CTTGAGGAAGGTAGGGGAGAATGGAATTCCTGGTGGAGCGGTGGAATGCGCAGATA
TCAGGAGGAACACCAGTGGCGAAGGCGGTTCTCTGGGCCTTTCCTGACGCTGAGGA
GCGAAAGCGTGGGGAGCGAACAGGCTTAGATACCCTGGTAGTCCACGCTGTAAACG
GTGGGTACTAGGTGTGGGGTCCATTCCACGGGTTCCGTGCCGTAGCTAACGCTTTAA
GTACCCCGCCTGGGGAGTACGGCCGCAAGGCTAAAACTCAAAGGAATTGACGGGGC
CCCGCACAAGCGGCGGAGCATGCGGATTAATTCGATGCAACGCGTAGAACCTTACC
TGGGTTTGACATGGATCGGGAGTGCTCAGAGATGGGTGTGCCTCTTTTGGGGTCGGT
TCACAGGTGGTGCATGCCTGTCGTCAGCTCGTGTCGTGAGATGTTGGGTTAAGTCCC
GCAACGAGCGCAACCCTTGTTCACTGTTGCCAGCACGTTATGGTGGGGACTCAGTGG
AGACCGCCGGGGTCAACTCGGAGGAAGGTGGGGATGACGTCAAGTCATCATGCCCC

TTATGTCCAGGGCTTCACGCATGCTACAATGGCTGGTACAGAGAGTGGCGAGCCTGT
GAGGGTGAGCGAATCTCGGAAAGCCGGTCTCAGTTCGGATTGGGGTCTGCAACTCG
ACCTCATGAAGTCGGAGTCGCTAGTAATCGCAGATCAGCAACGCTGCGGTGAATAC
GTTCCCGGGGCTTGTACACACCGCCCGTCAAGTCATGAAAGTTGGTAACACCCGAA
GCCGGTGGCCTAACCGTTGTGGGGGAGCCGTCGAAGGTGGGACTGGTGATTAGGAC
TAAGTCGTAACAAGGTAGCCGTACCGGAAGGTGCGGCTGGATCACCTCCTTTCTAAG
GA

SEQ ID NO: 7 is as follows (a mutation compared to the 16S sequence of the type strain KPA171202 is underlined):

AGAGTTTGATCCTGGCTCAGGACGAACGCTGGCGGCGTGCTTAACACATGCAAGTC
GAACGGAAAGGCCCTGCTTTTGTGGGGTGCTCGAGTGGCGAACGGGTGAGTAACAC
GTGAGTAACCTGCCCTTGACTTTGGGATAACTTCAGGAAACTGGGGCTAATACCGGA
TAGGAGCTCCTGCTGCATGGTGGGGGTTGGAAAGTTTCGGCGGTTGGGGATGGACT
CGCGGCTTATCAGCTTGTTGGTGGGGTAGTGGCTTACCAAGGCTTTGACGGGTAGCC
GGCCTGAGAGGGTGACCGGCCACATTGGGACTGAGATACGGCCCAGACTCCTACGG
GAGGCAGCAGTGGGGAATATTGCACAATGGGCGGAAGCCTGATGCAGCAACGCCGC
GTGCGGGATGACGGCCTTCGGGTTGTAAACCGCTTTCGCCTGTGACGAAGCGTGAGT
GACGGTAATGGGTAAAGAAGCACCGGCTAACTACGTGCCAGCAGCCGCGGTAATAC
GTAGGGTGCGAGCGTTGTCCGGATTTATTGGGCGTAAAGGGCTCGTAGGTGGTTGAT
CGCGTCGGAAGTGTAATCTTGGGGCTTAACCCTGAGCGTGCTTTCGATACGGGTTGA
CTTGAGGAAGGTAGGGGAGAATGGAATTCCTGGTGGAGCGGTGGAATGCGCAGATA
TCAGGAGGAACACCAGTGGCGAAGGCGGTTCTCTGGGCCTTTCCTGACGCTGAGGA
GCGAAAGCGTGGGGAGCGAACAGGCTTAGATACCCTGGTAGTCCACGCTGTAAACG
GTGGGTACTAGGTGTGGGGTCCATTCCACGGGTTCCGTGCCGTAGCTAACGCTTTAA
GTACCCCGCCTGGGGAGTACGGCCGCAAGGCTAAAACTCAAAGGAATTGACGGGGC
CCCGCACAAGCGGCGGAGCATGCGGATTAATTCGATGCAACGCGTAGAACCTTACC
TGGGTTTGACATGGATCGGGAGTGCTCAGAGATGGGTGTGCCTCTTTTGGGGTCGGT
TCACAGGTGGTGCATGGCTGTCGTCAGCTCGTGTCGTGAGATGTTGGGTTAAGTCCC
GCAACGAGCGCAACCCTTGTTCACTGTTGCCAGCACGTTATGGTGGGGACTCAGTGG
AGACCGCCGGGGTCAACTCGGAGGAAGGTGGGGATGACGTCAAGTCATCATGCCCC
TTATGTCCAGGGCTTCACGCATGCTACAATGGCTGGTACAGAGAGTGGCGAGCCTGT
GAGGGTGAGCGAATCTCGGAAAGCCGGTCTCAGTTCGGATTGGGGTCTGCAACTCG

ACCTCATGAAGTCGGAGTCGCTAGTAATCGCAGATCAGCAACGCTGCGGTGAATAC
GTTCCCGGGGCTTGTACACACCGCCCGTCAAGTCATGAAAGTTGGTAACACCCGAA
GCCGGTGGCCTAACCGTTGTGGGGGAGCCGTCGAAGGTGGGACTGGTGATTAGGAC
TAAGTCGTAACAAGGTAGCCGTACCGGAAGGTGCGGCTGGATCACCTCCTTTCTAAG
GA

SEQ ID NO: 8 is as follows (mutations compared to the 16S sequence of the type strain KPA171202 are underlined):

AGAGTTTGATCCTGGCTCAGGACGAACGCTGGCGGCGTGCTTAACACATGCAAGTC
GAACGGAAAGGCCCTGCTTTTGTGGGGTGCTCGAGTGGCGAACGGGTGAGTAACAC
GTGAGTAACCTGCCCTTGACTTTGGGATAACTTCAGGAAACTGGGGCTAATACCGGA
TAGGAGCTCCTGCTGCATGGTGGGGGTTGGAAAGTTTCGGCGGTTGGGGATGGACT
CGCGGCTTATCAGCTTGTTGGTGGGGTAGTGGCTTACCAAGGCTTTGACGGGTAGCC
GGCCTGAGAGGGTGACCGGCCACATTGGGACTGAGATACGGCCCAGACTCCTACGG
GAGGCAGCAGTGGGGAATATTGCACAATGGGCGGAAGCCTGATGCAGCAACGCCGC
GTGCGGGATGACGGCCTTCGGGTTGTAAACCGCTTTCGCCTGTGACGAAGCGTGAGT
GACGGTAATGGGTAAAGAAGCACCGGCTAACTACGTGCCAGCAGCCGCGGTGATAC
GTAGGGTGCGAGCGTTGTCCGGATTTATTGGGCGTAAAGGGCTCGTAGGTGGTTGAT
CGCGTCGGAAGTGTAATCTTGGGGCTTAACCCTGAGCGTGCTTTCGATACGGGTTGA
CTTGAGGAAGGTAGGGGAGAATGGAATTCCTGGTGGAGCGGTGGAATGCGCAGATA
TCAGGAGGAACACCAGTGGCGAAGGCGGTTCTCTGGGCCTTTCCTGACGCTGAGGA
GCGAAAGCGTGGGGAGCGAACAGGCTTAGATACCCTGGTAGTCCACGCTGTAAACG
GTGGGTACTAGGTGTGGGGTCCATTCCACGGGTTCCGTGCCGTAGCTAACGCTTTAA
GTACCCCGCCTGGGGAGTACGGCCGCAAGGCTAAAACTCAAAGGAATTGACGGGGC
CCCGCACAAGCGGCGGAGCATGCGGATTAATTCGATGCAACGCGTAGAACCTTACC
TGGGTTTGACATGGATCGGAAGCGCTCAGAGATGGGTGTGCCTCTTTTGGGGTCGGT
TCACAGGTGGTGCATGGCTGTCGTCAGCTCGTGTCGTGAGATGTTGGGTTAAGTCCC
GCAACGAGCGCAACCCTTGTTCACTGTTGCCAGCACGTTATGGTGGGGACTCAGTGG
AGACCGCCGGGGTCAACTCGGAGGAAGGTGGGGATGACGTCAAGTCATCATGCCCC
TTATGTCCAGGGCTTCACGCATGCTACAATGGCTGGTACAGAGAGTGGCGAGCCTGT
GAGGGTGAGCGAATCTCGGAAAGCCGGTCTCAGTTCGGATTGGGGTCTGCAACTCG
ACCTCATGAAGTCGGAGTCGCTAGTAATCGCAGATCAGCAACGCTGCGGTGAATAC
GTTCCCGGGGCTTGTACACACCGCCCGTCAAGTCATGAAAGTTGGTAACACCCGAA

GCCGGTGGCCTAACCGTTGTGGGGAGCCGTCGAAGGTGGGACTGGTGATTAGGAC
TAAGTCGTAACAAGGTAGCCGTACCGGAAGGTGCGGCTGGATCACCTCCTTTCTAAG
GA

SEQ ID NO: 9 is as follows (a mutation compared to the 16S sequence of the type strain KPA171202 is underlined):

AGAGTTTGATCCTGGCTCAGGACGAACGCTGGCGGCGTGCTTAACACATGCAAGTC
GAACGGAAAGGCCCTGCTTTGTGGGGTGCTCGAGTGGCGAACGGGTGAGTAACAC
GTGAGTAACCTGCCCTTGACTTTGGGATAACTTCAGGAAACTGGGGCTAATACCGGA
TAGGAGCTCCTGCTGCATGGTGGGGGTTGGAAAGTTTCGGCGGTTGGGGATGGACT
CGCGGCTTATCAGCTTGTTGGTGGGGTAGTGGCTTACCAAGGCTTTGACGGGTAGCC
GGCCTGAGAGGGTGACCGGCCACATTGGGACTGAGATACGGCCCAGACTCCTACGG
GAGGCAGCAGTGGGGAATATTGCACAATGGGCGGAAGCCTGATGCAGCAACGCCGC
GTGCGGGATGACGGCCTTCGGGTTGTAAACCGCTTTCGCCTGTGACGAAGCGTGAGT
GACGGTAATGGGTAAAGAAGCACCGGCTAACTACGTGCCAGCAGCCGCGGTGATAC
GTAGGGTGCGAGCGTTGTCCGGATTTATTGGGCGTAAAGGGCTCGTAGGTGGTTGAT
CGCGTCGGAAGTGTAATCTTGGGGCTTAACCCTGAGCGTGCTTTCGATACGGGTTGA
CTTGAGGAAGGTAGGGGAGAATGGAATTCCTGGTGGAGCGGTGGAATGCGCAGATA
TCAGGAGGAACACCAGTGGCGAAGGCGGTTCTCTGGGCCTTTCCTGACGCTGAGGA
GCGAAAGCGTGGGGAGCGAACAGGCTTAGATACCCTGGTAGTCCACGCTGTAAACG
GTGGGTACTAGGTGTGGGGTCCATTCCACGGGTTCCGTGCCGTAGCTAACGCTTTAA
GTACCCCGCCTGGGGAGTACGGCCGCAAGGCTAAAACTCAAAGGAATTGACGGGGC
CCCGCACAAGCGGCGGAGCATGCGGATTAATTCGATGCAACGCGTAGAACCTTACC
TGGGTTTGACATGGATCGGGAGTGCTCAGAGATGGGTGTGCCTCTTTTGGGGTCGGT
TCACAGGTGGTGCATGGCTGTCGTCAGCTCGTGTCGTGAGATGTTGGGTTAAGTCCC
GCAACGAGCGCAACCCTTGTTCACTGTTGCCAGCACGTTATGGTGGGGACTCAGTGG
AGACCGCCGGGGTCAACTCGGAGGAAGGTGGGGATGACGTCAAGTCATCATGCCCC
TTATGTCCAGGGCTTCACGCATGCTACAATGGCTGGTACAGAGAGTGGCGAGCCTAT
GAGGGTGAGCGAATCTCGGAAAGCCGGTCTCAGTTCGGATTGGGGTCTGCAACTCG
ACCTCATGAAGTCGGAGTCGCTAGTAATCGCAGATCAGCAACGCTGCGGTGAATAC
GTTCCCGGGGCTTGTACACACCGCCCGTCAAGTCATGAAAGTTGGTAACACCCGAA
GCCGGTGGCCTAACCGTTGTGGGGGAGCCGTCGAAGGTGGGACTGGTGATTAGGAC

TAAGTCGTAACAAGGTAGCCGTACCGGAAGGTGCGGCTGGATCACCTCCTTTCTAAG
GA

SEQ ID NO: 10 is as follows (mutations compared to the 16S sequence of the type strain KPA171202 are underlined):

AGAGTTTGATCCTGGCTCAGGACGAACGCTGGCGGCGTGCTTAACACATGCAAGTC
GAACGGAAAGGCCCTGCTTTGTGGGGTGCTCGAGTGGCGAACGGGTGAGTAACAC
GTGAGTAACCTGCCCTTGACTTTGGGATAACTTCAGGAAACTGGGGCTAATACCGGA
TAGGAGCTCCTGCTGCATGGTGGGGGTTGGAAAGTTTCGGCGGTTGGGGATGGACT
CGCGGCTTATCAGCTTGTTGGTGGGGTAGTGGCTTACCAAGGCTTTGACGGGTAGCC
GGCCTGAGAGGGTGACCGGCCACATTGGGACTGAGATACGGCCCAGACTCCTACGG
GAGGCAGCAGTGGGGAATATTGCACAATGGGCGGAAGCCTGATGCAGCAACGCCGC
GTGCGGGATGACGGCCTTCGGGTTGTAAACCGCTTTCGCCTGTGACGAAGCGTGAGT
GACGGTAATGGGTAAAGAAGCACCGGCTAACTACGTGCCAGCAGCCGCGGTGATAC
GTAGGGTGCGAGCGTTGCCCGGATTTATTGGGCGTAAAGGGCTCGTAGGTGGTTGAT
CGCGTCGGAAGTGTAATCTTGGGGCTTAACCCTGAGCGTGCTTTCGATACGGGTTGA
CTTGAGGAAGGTAGGGGAGAATGGAATTCCTGGTGGAGCGGTGGAATGCGCAGATA
TCAGGAGGAACACCAGTGGCGAAGGCGGTTCTCTGGGCCTTTCCTGACGCTGAGGA
GCGAAAGCGTGGGGAGCGAACAGGCTTAGATACCCTGGTAGTCCACGCTGTAAACG
GTGGGTACTAGGTGTGGGGTCCATTCCACGGGTTCCGTGCCGTAGCTAACGCTTTAA
GTACCCCGCCTGGGGAGTACGGCCGCAAGGCTAAAACTCAAAGGAATTGACGGGGC
CCCGCACAAGCGGCGGAGCATGCGGATTAATTCGATGCAACGCGTAGAACCTTACC
TGGGTTTGACATGGATCGGGAGTGCTCAGAGATGGGTGTGCCTCTTTTGGGGTCGGT
TCACAGGTGGTGCATGCCTGTCGTCAGCTCGTGTCGTGAGATGTTGGGTTAAGTCCC
GCAACGAGCGCAACCCTTGTTCACTGTTGCCAGCACGTTATGGTGGGGACTCAGTGG
AGACCGCCGGGGTCAACTCGGAGGAAGGTGGGGATGACGTCAAGTCATCATGCCCC
TTATGTCCAGGGCTTCACGCATGCTACAATGGCTGGTACAGAGAGTGGCGAGCCTGT
GAGGGTGAGCGAATCTCGGAAAGCCGGTCTCAGTTCGGATTGGGGTCTGCAACTCG
ACCTCATGAAGTCGGAGTCGCTAGTAATCGCAGATCAGCAACGCTGCGGTGAATAC
GTTCCCGGGGCTTGTACACACCGCCCGTCAAGTCATGAAAGTTGGTAACACCCGAA
GCCGGTGGCCTAACCGTTGTGGGGGAGCCGTCGAAGGTGGGACTGGTGATTAGGAC
TAAGTCGTAACAAGGTAGCCGTACCGGAAGGTGCGGCTGGATCACCTCCTTTCTAAG
GA

In embodiments, the at least one additional bacterium comprises, consists essentially of, or consists of a probiotic bacterium. In embodiments, the at least one bacterium includes 1, 2, 3, 4, 5, 6, 7, 8, 9, or 10 bacterial strains and/or species, less than about 10, 9, 8, 7, 6, 5, 4, 3, or 2 bacterial strains and/or species, or 1-10, 2-10, 3-10, 4-10, 5-10, 1-5, 2-5, 3-5, or 4-5 bacterial strains and/or species. In embodiments, the at least one bacterium includes a plurality of bacterial strains and/or species, *e.g.,* at least about 2, 3, 4, 5, 6, 7, 8, 9, 10 bacterial strains and/or species. In embodiments, the least one bacterium includes an isolated *Propionibacterium granulosum* bacterium, an isolated *Propionibacterium avidum* bacterium, an isolated *Staphylococcus epidermidis* bacterium, an isolated *Staphylococcus aureus* bacterium, and/or an isolated *Corynebacterium jeikeium* bacterium. In embodiments, the least one bacterium includes 1, 2 (of any combination of), 3 (of any combination of), 4 (of any combination of), or 5 of an isolated *Propionibacterium granulosum* bacterium, an isolated *Propionibacterium avidum* bacterium, an isolated *Staphylococcus epidermidis* bacterium, an isolated *Staphylococcus aureus* bacterium, and/or an isolated *Corynebacterium jeikeium* bacterium.

In embodiments, a composition or combination provided herein includes an enhancing peptide or enzyme. In embodiments, the enhancing peptide or enzyme has one or more or any combination of the following properties: biofilm degradation, improving skin penetration, antibacterial, reducing inflammation *(e.g.,* of the skin), reducing irritation *(e.g.,* of the skin), reducing redness *(e.g.,* of the skin), firming skin, removing lines, removing wrinkles, or otherwise improving appearance (e.g., of the skin).

In an aspect of the invention, a composition that includes a *P. acnes* bacteriophage and an anti-acne compound comprising salicylic acid, and a pharmaceutically acceptable carrier, wherein the genome of the *P. acnes* bacteriophage comprises a nucleotide sequence that is at least about 98%, or 99% identical to the nucleotide sequence of SEQ ID NO: 1 is providedIn embodiments, the dose of the *P. acnes* bacteriophage is adjusted *(e.g.,* increased or decreased) for stability. In embodiments, the dose of the *P. acnes* bacteriophage is adjusted up or down depending on the anti-acne compound to adjust for its stability in combination with the anti-acne compound.

In an aspect of the invention, a combination or system that includes a *P. acnes* bacteriophage and at least one anti-acne compound comprising salicylic acid, and a pharmaceutically acceptable carrier, wherein each of the at least one *Propionibacterium acnes* bacteriophage, the at least one anti-acne compound, and the pharmaceutically acceptable carrier are in a single composition in a container, and wherein the genome of the *P. acnes* bacteriophage comprises a nucleotide sequence that is at least about 98%, or 99% identical to the nucleotide sequence of SEQ ID NO: 1, and a separate container comprising an additional anti-acne compound comprising benzoyl peroxide is provided. In an example, the bacteriophage is within one composition (e.g., within one vessel such as a bottle, tube, or other container), and the one or more anti-acne compounds are in a separate composition (within another vessel such as a bottle, tube, or other container). In embodiments, an additional one or more compounds (e.g. an enzyme, a hydrating compound, an ultraviolet radiation absorbing or blocking compound, *etc.)* are present in the composition that includes the bacteriophage, the composition that includes the one or more anti-acne compounds, or a third separate composition (within a third vessel such as a bottle, tube, or other container). In embodiments, one or more probiotic bacteria are present in the composition that includes the bacteriophage, the composition that includes the one or more anti-acne compounds, or a third separate composition (within a third vessel such as a bottle, tube, or other container). In embodiments, the combination or system further includes instructions for administration. In embodiments, the combination or system includes at least about 2, 3, 4, 5, 6, 7, 8, 9, or 10 P. *acnes* bacteriophages.

Also disclosed but not claimed is a combination or system that includes a *P. acnes* bacteriophage and one or more probiotic bacteria and/or one or more compounds (such as one or more enzymes or anti-acne compounds) is provided. In an example, the bacteriophage is within one composition (e.g., within one vessel such as a bottle, tube, or other container), and the one or more probiotic bacteria are in a separate composition (within another vessel such as a bottle, tube, or other container), and optionally, an additional one or more compounds are present in the composition that includes the bacteriophage, the composition that includes the one or more probiotic bacteria, or a third separate composition (within a third vessel such as a bottle, tube, or other container). In embodiments, the combination or system further includes instructions for administration. In embodiments, the combination or system includes at least about 2, 3, 4, 5, 6, 7, 8, 9, or 10 *P. acnes* bacteriophages.

In embodiments, a system, combination, or composition includes an enzyme such as a biofilm degradation enzyme or an anti-aging enzyme. Non-limiting examples of biofilm degradation enzymes include DNAses *(e.g.,* DNAse I), proteases *(e.g.,* papain, bromelain, Trypsin, Proteinase K, Subtilisin, or serratiopeptidase), glycosidases (e.g., dispersin, alginate lyase, amylase, or cellulase). Non-limiting examples of anti-aging enzymes include superoxide dismutase, and peroxidase.

In embodiments, a system, combination, or composition includes a topical retinoid, an antibiotic, and/or an alpha-hydroxy acid. In embodiments, a system or composition further includes a topical retinoid. In embodiments, a system or composition further includes an antibiotic. In embodiments, a system or composition further includes an alpha-hydroxy acid. In embodiments, the system or composition further includes benzoyl peroxide, salicylic acid, sulfur, resorcinol, resorcinol monoacetate, or any combination thereof. In embodiments, the benzoyl peroxide is present at a concentration of 2.5% to 10%, *e.g.,* about 2.5%, 3%, 3.5%, 4%, 4.5%, 5%, 5.5%, 6%, 6.5%, 7%, 7.5%, 8%, 8.5%, 9%, 9.5%, or 10% (weight/volume). In embodiments, the benzoyl peroxide is present at a concentration of less than 2.5% but greater than about 0.1%, 0.5%, 1%, 1.5%, or 2% (weight/volume). In embodiments, the salicylic acid is present at a concentration of 0.5% to 2%, *e.g.,* about 0.5%, 0.6%, 0.7%, 0.8%, 0.9%, 1%, 1.1%, 1.2%, 1.3%, 1.4%, 1.5%, 1.6%, 1.7%, 1.8%, 1.9%, or 2% (weight/volume). In embodiments, the salicylic acid is present at a concentration of less than 0.5% but greater than about 0.1% (weight/volume). In embodiments, the sulfur is present at a concentration of 3% to 10%, *e.g.,* about 3%, 3.5%, 4%, 4.5%, 5%, 5.5%, 6%, 6.5%, 7%, 7.5%, 8%, 8.5%, 9%, 9.5%, or 10% (weight/volume). In embodiments, the sulfur is present at a concentration of less than 3% but greater than about 0.1%, 0.5%, 1%, 1.5%, 2%, or 2.5% (weight/volume). In embodiments, resorcinol is present at a concentration of 2% and sulfur is present at a concentration of 3% to *8% (e.g.,* about 2.5%, 3%, 3.5%, 4%, 4.5%, 5%, 5.5%, 6%, 6.5%, 7%, 7.5%, or 8%) (weight/volume). In embodiments, resorcinol monoacetate is present at a concentration of 3% and sulfur is present at a concentration of 3% to 8% *(e.g.,* about 2.5%, 3%, 3.5%, 4%, 4.5%, 5%, 5.5%, 6%, 6.5%, 7%, 7.5%, or 8%) (weight/volume). In embodiments, the resorcinol is present at a concentration of less than 2% but greater than about 0.1%, 0.5%, 1%, 1.5% (weight/volume). In embodiments, the resorcinol monoacetate is present at a concentration of less than 3% but greater than about 0.1%, 0.5%, 1%, 1.5%, 2%, or 2.5% (weight/volume).

In embodiments, a composition provide herein includes a moisturizer.

### METHODS OF TREATING ACNE

Also disclosed, but not claimed, is a method of preventing or treating acne in a subject in need thereof, the method including administering an effective amount of a composition or combination provided herein. In embodiments, an effective amount of a composition comprising, consisting essentially of, or consisting of at least one *P. acnes* bacteriophage, at least one anti-acne compound, and a pharmaceutically acceptable carrier is administered to the subject. In embodiments, an effective amount of a composition that includes at least one P. *acnes* bacteriophage, at least one anti-acne compound and a pharmaceutically acceptable carrier, wherein the composition does not comprise a probiotic bacterium, is administered to the subject. In embodiments, an effective amount of a composition that includes a *P. acnes* bacteriophage and an enzyme is administered to the subject.

It may be that an effective amount of a composition that includes a bacteriophage as described herein, including embodiments thereof, is administered to the subject. In embodiments, the bacteriophage is a wild-type bacteriophage.

It may be thatthe bacteriophage is administered topically. In embodiments, the bacteriophage is in a composition (e.g., a pharmaceutical or cosmetic composition) that further includes a pharmaceutically or cosmetically acceptable carrier.

It may be thatthe method further includes administering a probiotic bacterium to the subject.

Also disclosed, but not claimed, is a method of treating acne in a subject in need thereof is provided. The method includes administering an effective amount of a probiotic *P. acnes* bacterium to the subject. In embodiments, the method further includes administering a bacteriophage to the subject.

It may be thatthe P. *acnes* bacterium has a 16S rDNA sequence that includes a T992C, T838C, C1322T, and/or a C986T mutation compared to the KPA171202 type strain 16S rDNA sequence set forth as SEQ ID NO: 2. In embodiments, the *P. acnes* bacterium includes a 16S rDNA sequence with a T838C and a C1322T mutation compared to the KPA171202 type strain 16S rDNA sequence set forth as SEQ ID NO: 2. In embodiments, the *P. acnes* bacterium is the ProI strain. In embodiments, the *P. acnes* bacterium includes a 16S rDNA sequence with a C986T and a T992C mutation compared to the KPA171202 type strain 16S rDNA sequence set forth as SEQ ID NO: 2. In embodiments, the *P. acnes* bacterium is the ProII strain.

It may be thatthe *P. acnes* bacterium: (a) includes a 16S rDNA sequence with a T992C mutation compared to the KPA171202 type strain 16S rDNA sequence set forth as SEQ ID NO: 2; (b) includes a 16S rDNA sequence with a T838C mutation compared to the KPA171202 type strain 16S rDNA sequence set forth as SEQ ID NO: 2; (c) includes a 16S rDNA sequence with a C1322T mutation compared to the KPA171202 type strain 16S rDNA sequence set forth as SEQ ID NO: 2; (d) includes a 16S rDNA sequence with a C986T mutation compared to the KPA171202 type strain 16S rDNA sequence set forth as SEQ ID NO: 2; (e) includes a 16S rDNA sequence that is identical to the sequence of SEQ ID NO: 3; (g) includes a 16S rDNA sequence that is identical to the sequence of SEQ ID NO: 4; (h) does not comprise a linear plasmid; (i) does not include a plasmid that includes a virulence factor; and/or (j) does not include a plasmid that encodes an extrachromosomal lipase and/or a tight adhesion virulence factor.

It may be thatthe method further includes administering at least one additional probiotic bacterium to the subject.

It may be thatthe at least one additional bacterium comprises, consists essentially of, or consists of a probiotic bacterium. In embodiments, the at least one bacterium includes 1, 2, 3, 4, 5, 6, 7, 8, 9, or 10 bacterial strains and/or species, less than about 10, 9, 8, 7, 6, 5, 4, 3, or 2 bacterial strains and/or species, or 1-10, 2-10, 3-10, 4-10, 5-10, 1-5, 2-5, 3-5, or 4-5 bacterial strains and/or species. In embodiments, the at least one bacterium includes a plurality of bacterial strains and/or species, *e.g.,* at least about 2, 3, 4, 5, 6, 7, 8, 9, 10 bacterial strains and/or species. In embodiments, the least one bacterium includes a *Propionibacterium* sp., *Staphylococcus* sp., and/or *Corynebacterium* sp. bacterium. In embodiments, the least one bacterium includes bacterium within the class *Betaproteobacteria.* In embodiments, the least one bacterium includes an isolated *Propionibacterium granulosum* bacterium, an isolated *Propionibacterium avidum* bacterium, an isolated *Staphylococcus epidermidis* bacterium, an isolated *Staphylococcus aureus* bacterium, and/or an isolated *Corynebacterium jeikeium* bacterium. In embodiments, the least one bacterium includes 1, 2, 3, 4, or 5 of an isolated *Propionibacterium granulosum* bacterium, an isolated *Propionibacterium avidum* bacterium, an isolated *Staphylococcus epidermidis* bacterium, an isolated *Staphylococcus aureus* bacterium, and/or an isolated *Corynebacterium jeikeium* bacterium.

It may be that the subject has been administered a bacteriophage as described herein, including embodiments thereof.

It may be that the subject has been administered an antibiotic that kills *P. acnes.* In embodiments, the antibiotic is clindamycin, doxycycline, erythromycin, or tetracycline, or a derivative of clindamycin, doxycycline, erythromycin, or tetracycline.

It may be thatthe antibiotic is clindamycin, doxycycline, erythromycin, or tetracycline, or a derivative of clindamycin, doxycycline, erythromycin, or tetracycline.

It may be thatthe method further includes administering an enzyme to the subject such as a biofilm degradation enzyme or an anti-aging enzyme. Non-limiting examples of biofilm degradation enzymes include DNAses *(e.g.,* DNAse I), restriction endonucleases, proteases *(e.g.,* papain, bromelain, Trypsin, Proteinase K, Subtilisin, or serratiopeptidase), glycosidases (e.g., dispersin, alginate lyase, amylase, or cellulase). Non-limiting examples of anti-aging enzymes include superoxide dismutase, and peroxidase.

It may be thatthe method further includes administering a topical retinoid, an antibiotic, and/or an alpha-hydroxy acid. In embodiments, the method further includes administering a topical retinoid. In embodiments, the method further includes administering an antibiotic. In embodiments, the method further includes administering an alpha-hydroxy acid. In embodiments, the method further includes administering benzoyl peroxide, salicylic acid, sulfur, resorcinol, and/or resorcinol monoacetate to the subject. In embodiments, the method further includes administering benzoyl peroxide. In embodiments, the method further includes administering salicylic acid. In embodiments, the method further includes administering sulfur. In embodiments, the method further includes administering resorcinol and/or sulfur. In embodiments, the method further includes administering resorcinol and/or resorcinol monoacetate.

It may be thatthe method further includes administering an enhancing peptide or enzyme. In embodiments, the enhancing peptide or enzyme has one or more or any combination of the following properties: biofilm degradation, improving skin penetration, antibacterial, reducing inflammation *(e.g.,* of the skin), reducing irritation *(e.g.,* of the skin), reducing redness *(e.g.,* of the skin), firming skin, removing lines, removing wrinkles, or otherwise improving appearance (e.g., of the skin).

It may be that at least about 2, 3, 4, 5, 6, 7, 8, 9, or 10 *P. acnes* bacteriophages are administered to the subject. In embodiments, the *P. acnes* bacteriophages include more than one types of *P. acnes* bacteriophage.

### EXEMPLARY METHODS AND COMPOSITIONS FOR TREATING ACNE

In an aspect of the invention, provided herein is a composition that includes at least one *Propionibacterium acnes (P. acnes)* bacteriophage, at least one anti-acne compound comprising salicylic acid, and a pharmaceutically acceptable carrier, wherein the genome of the P. acnes bacteriophage comprises a nucleotide sequence that is at least about 98%, or 99% identical to the nucleotide sequence of SEQ ID NO: 1. In embodiments, the bacteriophage is present in a composition, such as a therapeutic or cosmetic composition. In embodiments, the composition further includes a strain of probiotic bacteria. In embodiments, the composition further includes an enzyme that degrades a bacterial biofilms *(e.g.,* a component thereof) in or on human skin pores. In embodiments, the enzyme enhances penetration of the bacteriophage and/or the probiotic bacteria. In embodiments, a bacteriophage ("phage") destroys an acne-causing *(i.e.,* pathogenic) strain of *P. acnes* with a high degree of specificity and efficacy, without killing beneficial skin bacteria. In embodiments, the biofilm-degrading enzyme dissolves the biofilm to increase the susceptibility of the pathogen (e.g., by reducing pathogen adherence to host cells and/or by increasing access of the bacteriophage to pathogenic cells). In embodiments, the probiotic bacteria are immune to the bacteriophage (e.g., the bacteria lack a cellular receptor to which the bacteriophage specifically binds). In embodiments, the probiotic bacteria occupy the niche left by a killed *P. acnes* pathogenic strain. In embodiments the probiotic bacteria reduce or prevent the recolonization or growth of a subject's skin (such as a pore) by surviving pathogenic bacteria.

In an aspect of the invention a composition according to the invention for use in the therapeutic treatment of the skin disease acne is provided. In embodiments, the composition includes a lytic *P. acnes* bacteriophage, and optionally a probiotic bacterium sourced from healthy skin, and/or optionally a biofilm-degrading enzyme in the composition as an adjuvant to increase penetration of the active components.

In embodiments, a lytic *P. acnes* bacteriophage infects virulent *P. acnes* in a skin comedone. In embodiments, the bacteriophage replicates and lyses within the *P. acnes.* In embodiments, when the *P. acnes* lyses, it releases new virions. In embodiments, enzymes unclog the blocked comedones, dissolve the *P. acnes* biofilms and increase access of virions to *P. acnes.* In embodiments, the exponential proliferation of lytic *P. acnes* phages rapidly kills the *P. acnes* with high specificity, without disturbing the growth beneficial skin commensal bacteria. In embodiments, the niche vacated by the *P. acnes* is then be filled by the probiotic bacteria. In embodiments, the bacteria are sourced from healthy skin and expand to occupy the niche, thereby preventing any surviving *P. acnes* bacteria from growing back. In embodiments, this strategy helps to balance the skin microbiome in subjects and recalibrates their microbiome toward a healthy skin bacterial community. In embodiments, the biofilm-degrading enzyme is in a formulation as an adjuvant that helps unclog blocked comedones and increase access of the phage and probiotic bacteria to the pores.

In embodiments, the combination includes a probiotic bacterium, and (optionally) an enzyme that enhances the penetration of the bacteriophage. In embodiments, the pathogens are killed and the probiotic bacterium replaces the pathogen. In embodiments, a "kill and replace" approach to is used to treat acne. In embodiments, a biologic that selectively kills pathogenic bacteria that cause acne is administered to a subject. In embodiments, probiotic bacteria sourced from healthy skin are applied to occupy the niche of the killed pathogen. In embodiments, this approach avoids the problems of rampant drug resistance associated with antibiotics. In embodiments, the presence of actively dividing probiotic bacteria prevents relapses by not allowing any pathogens to grow back. In embodiments, dysbiosis on the skin of the subject is treated. In embodiments, a microbiome associated with acne is recalibrated into a healthy one.

In embodiments, the bacteriophage is a naturally occurring *P. acnes* bacteriophage.

Non-limiting examples of enzymes that may be co-administered with a bacteriophage include BL00275 from *Bacillus licheniformis;* DNase I; restriction endonucleases; deoxyribonucleases *(e.g.* from *Staphylococcus aureus* thermonuclease, *B. licheniformis* NucB, DNase 1L2); glycoside hydrolases (e.g. Dispersin B, alginate lyase, amylase, cellulase, glycanase); and proteases (e.g. subtlisin, proteinase K, trypsin, serratiopeptidase).

Non-limiting examples of probiotic bacteria that may be administered or present in a system or composition include one or more or any combination of the following bacterial species: *Propionibacterium acnes, Propionibacterium granulosum, Propionibacterium avidum, Staphylococcus epidermidis, Staphylococcus aureus,* and *Corynebacterium jeikeium.* In embodiments, a probiotic bacterial strain is be selected based on its ability to (a) colonize the skin without eliciting an adverse immune response, characterized by low lipase activity and reduced adhesion to human keratinocytes; and (b) occupy a niche similar to *Propionibacterium acnes.*

In embodiments, a biofilm degrading enzyme is present in the formulation and acts as an adjuvant, to increase the efficacy of the active ingredients (such as a bacteriophage). In embodiments, the enzyme has the capacity to degrade *P. acnes* biofilms *in vitro.*

### EXAMPLES

The following examples illustrate certain specific embodiments of the invention and are not meant to limit the scope of the invention.

Embodiments herein are further illustrated by the following examples and detailed protocols. However, the examples are merely intended to illustrate embodiments and are not to be construed to limit the scope herein.

### EXAMPLE 1. P. ACNES BACTERIOPHAGE PHIT-101 KILLS P. ACNES SELECTIVELY AND EFFICIENTLY.

*P. acnes* bacteriophages have been shown to be genetically highly similar and exhibit a broad range against multiple strains of *P. acnes.* A lead bacteriophage (PHIT-101) was used for experimentation. PHIT-101 is a single lytic phage that killed all the strain types of *P. acnes* tested (data not shown). PHIT-101 has the sequence of SEQ ID NO: 1. In order to showcase the efficacy and specificity of this phage, a plate assay was performed as follows. *P. acnes* KPA171202 and *P. granulosum* (a closely related but benign skin bacterium) were plated on separate BHI-agar plates. Sterile cotton pads were placed on each plate. The sterile cotton pads were soaked in either minocycline, an antibiotic commonly used to treat acne, or a phage solution with a titer of 2 x 10⁷ pfu/mL. After incubating the plates anaerobically for 72 hours at 37°C, the minocycline pads killed bacteria indiscriminately, showing a zone of killing on both the acne-causing *P. acnes* and the commensal *P. granulosum* (FIG. 1). In contrast, the PHIT-101 pads killed only the *P. acnes,* without disturbing the growth of beneficial *P. granulosum.*

Further evidence of the ability of PHIT-101 to kill selectively was obtained in a synthetic skin microbiome assay. A synthetic skin microbiome was formulated comprising P. *acnes, P. granulosum,* and *P. avidum,* three skin bacteria that comprise 60-80% of microbiota in the skin pore [Science (2009) 324:1190-1192]. This synthetic skin microbiome was grown anaerobically in the presence or absence of PHIT-101 (final concentration 5 x 10⁵ pfu/mL). After 48 hours of incubation at 37°C, the cells were pelleted and washed, and the relative proportions of the three species was determined using 16S amplicon next-generation sequencing (NGS) on Illumina MiSeq. The results in FIG. 2 show that PHIT-101 is able to kill *P. acnes* almost completely, without negatively affecting the growth of the commensal *P. granulosum* and *P. avidum.*

### Screening biofilm degrading enzymes (BDEs) to disrupt P. acnes biofilms.

Several recent reports (Exp Dermatol (2014) 23:687, Br J Dermatol (2015) 172:13) have established that *P. acnes* produces significant amounts of biofilm in skin pores, which prevents antibiotic penetration and results in poor treatment outcomes. In order to validate this, biofilm production of several strains of *P. acnes* was quantified. FIG. 3 shows that adherent cultures of multiple strains isolated from the microbiota of a single subject produce markedly different levels of biofilm under similar conditions. Thus the previous proof-of-concept using planktonic cells did not reflect the true conditions under which *P. acnes* grows on the skin.

Without being bound by any scientific theory, we hypothesized that biofilms might present a significant barrier to phage killing of sessile *P. acnes* cells. This hypothesis was validated in a cell survival assay (FIG. 5) which showed that unlike planktonic *P. acnes (99%* killing, FIG. 2), PHIT-101 was only able to kill about 50% of the *P. acnes* cells encased in biofilms. In order to determine whether biofilm degradation would improve phage killing, a number of enzymes was screened to find a BDE specific for *P. acnes.* The screen comprised three classes of enzymes that might degrade types of materials that may be found in biofilms: DNA, polysaccharides, and proteins. FIG. 4 shows that in the screen, DNAses had moderate activity while the best rates of biofilm degradation were found in proteases and dispersin, a glycoside hydrolase from *Aggregatibacter actinomycetemcomitans.*

In selecting the BDE to pair with the phage, dispersin was selected for two reasons: firstly, as a glycoside hydrolase it was unlikely to attack the protein coat of the phage itself, thereby avoiding possible degradation of the phage. Secondly, *P. acnes* co-forms robust biofilms with *Staphylococcus aureus* [Anaerobe (2016) 40:63-67] and dispersin is active against biofilms from both organisms. Whether the addition of dispersin would increase the efficiency of phage killing in sessile *P. acnes* was determined. FIG. 5 shows that bacterial killing of PHIT-101 was enhanced in the presence of dispersin, restoring a ~99% killing efficiency to the phage.

### EXAMPLE 2. PROBIOTIC BACTERIA

### Genotypic characterization of probiotic strains.

Strains of *P. acnes* were characterized based on point mutations in the 16S rDNA sequence which leads to phylogenetic sorting into pathogenic and probiotic strain types, and the absence of a linear plasmid found in pathogenic strains, which carries virulence factors. Using 16S-specific primers the full 16S rDNA sequence of each *P. acnes* strain was amplified and Sanger-sequenced. A probiotic strain was identified as having ribosequence (RS) of ProI or ProII. ProI strains have T838C and C1322T mutations relative to the KPA171202 type strain's 16S rDNA sequence (NIH Accession No. NC_006085.1). ProII strains have C986T and T992C mutations relative to the KPA171202 sequence. Further, using specific primer pairs, the presence or absence of a linear plasmid within each strain was determined. Probiotic strains were identified as lacking this plasmid, which carries an extrachromosomal lipase as well as the *Tad* (tight adhesion) virulence factor.

In embodiments, the probiotic strains are characterized primarily by their 16S sequences, e.g., SEQ ID NO: 3 and SEQ ID NO: 4. In embodiments, they can be genotypically identified by the lack of the plasmid bearing virulence factors, such as an extrachromosomal lipase and a *Tad* locus.

The cohort of probiotic strains was further characterized for their immunogenic potential. A lead probiotic candidate based on two factors: low lipase production, and less tight adherence to epithelial cells. The phenotypic validation of these features was important in selecting the probiotic lead candidate.

### Testing the immunogenic potential of probiotic P. acnes strains: lipase activity.

Lipases play an important role in pathogenesis of acne by hydrolyzing sebum triglycerides and releasing irritating free fatty acids in the pilosebaceous follicles. Lipase is a strong chemotactic and proinflammatory antigen. Therefore, lipase is of high interest as a pharmacological target for anti-acne drugs. In embodiments, the overall strategy is to replace the pathogenic *P. acnes* that secretes high levels of lipase with a low-secreting probiotic *P. acnes.* In order to quantify the lipase expression phenotype for each strain in our panel, lipase production of the probiotic *P. acnes* strains was compared against pathogenic *P. acnes* strains with a fluorescent lipase activity assay.

One of the most interesting findings was that each strain secreted different amounts of lipases when grown in planktonic vs adherent culture. This has been previously reported in *P. acnes* strains [Res Microbiol, (2007) 158:386-392]. Further, the data showed that when these strains were grown in liquid culture, there was no significant difference between the lipase output of the pathogenic and probiotic strains. However, when these strains were grown under biofilm conditions, an interesting change was seen. While variability in production between strains could still be observed, several probiotic strains had significantly less lipase activity than pathogenic strains (FIG. 11). Interestingly, not all strains within the probiotic cohort had low lipase activity. For example, the lipase production of strains Pr-1 and Pr-5 was over the threshold for a probiotic strain, and was not developed further. Thus by quantifying lipase production in sessile *P. acnes* cells, it was possible to screen amongst probiotic strains and select those lead candidates with the most consistent low levels of lipase activity.

Thus, while pathogenic and probiotic strains secreted similar amounts of lipase in planktonic culture, the probiotic strains secreted far less lipase in adherent culture than pathogenic strains. FIG. 8 shows that the top probiotic candidates had a low lipase profile compared to the pathogenic strain.

### Testing the immunogenic potential of probiotic P. acnes strains: cell adherence.

Available pathogenic strains were confirmed to possess a tight adhesion *(tad)* locus that plays a role in the virulence of other mammalian pathogens [J Bacteriol (2000) 182:6169-6176; Nat Rev Microbiol (2007) 5:363-375; PNAS (2003) 100:7295-7300]. Greater adherence to host cells may increase virulence or induce an inflammatory host response. The probiotic strains were previously genotypically verified to not contain the *tad* locus, and thus predicted to adhere less tightly to epithelial cells. The adhesion of pathogenic and probiotic strains to A-431 dermal epithelial cells was compared, in order to assess whether there was an appreciable difference in adherence. FIG. 9 shows that the top three probiotic candidates adhered less tightly to epithelial cells than the pathogenic strain. Interestingly, once again a subtle but persistent difference in cell adhesion was found between different strain families of *P. acnes.* Thus the strains of *P. acnes* with ProI ribosequence exhibited a slightly higher cell adherence (Pr-2 in FIG. 9) while the ProII strains adhered to cells less tightly (Pr-B, Pr-C in FIG. 9).

### Comparison of pathogenic and probiotic P. acnes in mouse ear inflammation model.

Upon validating the low immunogenic potential of the probiotic strains showing that they produced less lipase and adhered less tightly to epithelial cells, the inflammatory response of these strains was tested in a mouse ear inflammation model, which is well established and has been used previously to evaluate the inflammatory potential of *P. acnes* in the context of acne. The inflammatory potential of pathogenic and probiotic strains was compared in the following study: 10¹⁰ cfu of a strain was injected into the ears of CBA/J mice. A cohort of 5 mice was assigned to each strain. After 5 days the ears were excised and examined for inflammation. The levels of several inflammatory cytokines (IL-1 β, IL-6, IL-17, TNFα) were measured and the sections of the tissue were examined by histology. FIG. 10 shows that the pathogenic strain had significantly higher levels of IL-1β, IL-6, IL-17, and TNFα compared to the probiotic strain.

### Acute dermal safety and toxicity of probiotic strains in miniswine skin model.

A miniswine model was used to test the probiotic strain for skin irritation. Swine are one of the major animals used in translational research, and pig skin is physiologically, anatomically, biochemically and immunologically similar to human skin. Miniswine are particularly commonly used to model human dermal diseases and conditions like acne [Vet Pathol (2012) 49:344-356]. The probiotic strain was applied to the skin of three separate miniswine in two doses - 10⁸ cfu and 10⁹ cfu - in delimited skin areas. The animals were observed daily for clinical signs and the dosing site skin was scored using the Draize Scoring System at pre-dose, 0.5, 1, 4, 8, and 24 hours post dose administration. There was no erythema or edema associated with the lead probiotic strain during the entire period (Table 1), and a Draize score of 0 was observed throughout. This demonstrates the safety to acute exposure of our probiotic strain in an animal skin model.

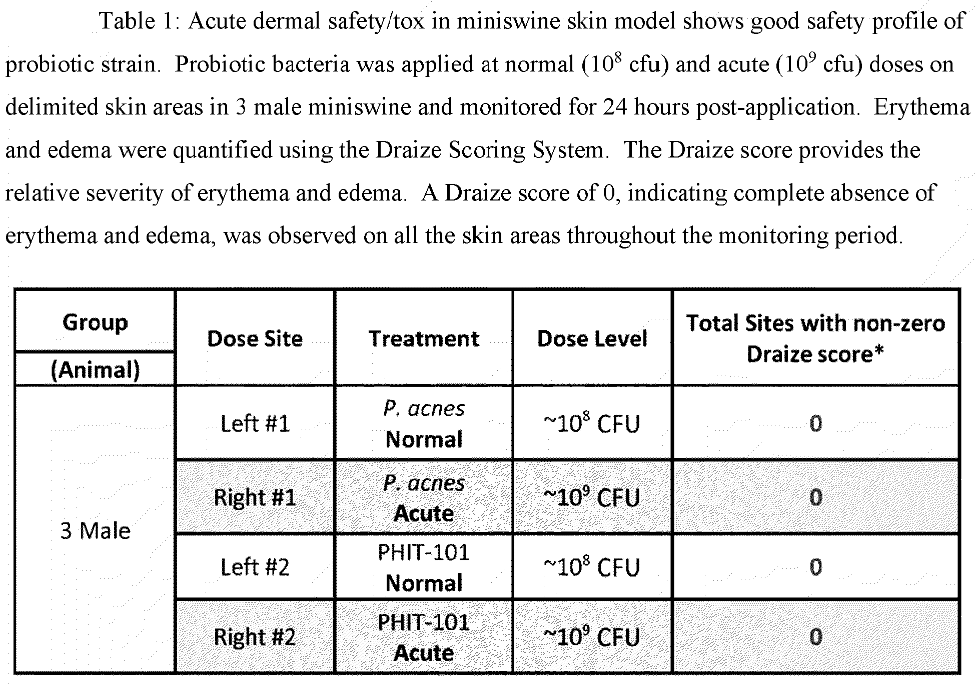

### EXAMPLE 3. BACTERIOPHAGE STABILITY IN COMPOSITIONS WITH ANTI-ACNE COMPOUNDS

In order to determine whether the phage was stable in co-formulation with either salicylic acid or benzoyl peroxide (BPO), the phage was co-incubated with these agents at a low and high concentration. The range of concentrations was determined by the permitted concentrations of these agents specified in the United States Food and Drug Administration (FDA) acne monograph for over-the-counter use. For salicylic acid, this is 0.5% to 2% (w/v), while for BPO the range is 2.5% to 10% (w/v). Buffered solutions of phage were added to these agents, and its stability at 4°C was tested over 60-90 days. FIG. 15 shows that the phages are stable in the presence of both low and high doses of salicylic acid. In contrast, FIG. 16 shows that benzoyl peroxide destabilizes the phages, and the observed rate of decrease in phage viability is steeper at a higher concentration of BPO.

### EXAMPLE 4 (PROPHETIC). TREATMENT WITH A COMBNIATION OF BACTERIOPHAGE WITH SALICYLIC ACID

A double-blind, placebo-controlled study of a composition comprising *Propionibacterium acnes* bacteriophage and salicylic acid is conducted determine the comparative efficacy of this treatment with placebo, *Propionibacterium acnes* bacteriophage alone, and salicylic acid alone. Concentrations of 0.5% and 2% (w/v) salicylic acid are administered with and without *Propionibacterium acnes* bacteriophage. In all conditions that include the *Propionibacterium acnes* bacteriophage, the phage is present in a dose of 10⁹ pfu (plaque forming units) per dose. Ten subjects who have comparably severe acne are treated for each of the following groups:
(i) Placebo (no active agent)
(ii) 0.5% salicylic acid as the sole active agent
(iii) 2% salicylic acid as the sole active agent
(iv) *Propionibacterium acnes* bacteriophage as the sole active agent
(v) the combination of 0.5% salicylic acid and *Propionibacterium acnes* bacteriophage (in a single composition)
(vi) the combination of 2% salicylic acid and *Propionibacterium acnes* bacteriophage (in a single composition)

The combination of the *Propionibacterium acne* bacteriophage with salicylic acid achieves more than an additive effect, *i.e.,* a synergistic effect (the combined effect of the bacteriophage and the salicylic acid is greater than the sum of the effects of the bacteriophage and the salicylic acid when each agent is used separately) in treating acne. The effectiveness of treatment is measured using lesion counts and an IGA (investigator global assessment) score.

### EXAMPLE 5 (PROPHETIC). TREATMENT WITH A COMBNIATION OF BACTERIOPHAGE WITH SULFUR

A double-blind, placebo-controlled study of a composition comprising *Propionibacterium acnes* bacteriophage and sulfur is conducted determine the comparative efficacy of this treatment with placebo, *Propionibacterium acnes* bacteriophage alone, and sulfur alone. Concentrations of 3% and 10% (w/v) sulfur are administered with and without *Propionibacterium acnes* bacteriophage. In all conditions that include the *Propionibacterium acnes* bacteriophage, the phage is present in a dose of 10⁹ pfu per dose. Ten subjects who have comparably severe acne are treated for each of the following groups:
(i) Placebo (no active agent)
(ii) 3% sulfur as the sole active agent
(iii) 10% sulfur as the sole active agent
(iv) *Propionibacterium acnes* bacteriophage as the sole active agent
(v) the combination of 3% sulfur and *Propionibacterium acnes* bacteriophage (in a single composition)
(vi) the combination of 10% sulfur and *Propionibacterium acnes* bacteriophage (in a single composition)

The combination of the *Propionibacterium acne* bacteriophage with sulfur achieves more than an additive effect, *i.e.,* a synergistic effect (the combined effect of the bacteriophage and the sulfur is greater than the sum of the effects of the bacteriophage and the sulfur when each agent is used separately) in treating acne. The effectiveness of treatment is measured using lesion counts and an IGA (investigator global assessment) score.

### EXAMPLE 6 (PROPHETIC). TREATMENT WITH A COMBNIATION OF BACTERIOPHAGE WITH BENZOYL PEROXIDE

A double-blind, placebo-controlled study of a composition comprising *Propionibacterium acnes* bacteriophage and BPO is conducted determine the comparative efficacy of this treatment with placebo, *Propionibacterium acnes* bacteriophage alone, and BPO alone. Concentrations of 2.5% and 10% (w/v) BPO are administered with and without *Propionibacterium acnes* bacteriophage. In all conditions that include the *Propionibacterium acnes* bacteriophage, the phage is present in a dose of 10⁹ pfu per dose. Ten subjects who have comparably severe acne are treated for each of the following groups:
(i) Placebo (no active agent)
(ii) 2.5% BPO as the sole active agent
(iii) 10% BPO as the sole active agent
(iv) *Propionibacterium acnes* bacteriophage as the sole active agent
(v) the combination of 2.5% BPO and *Propionibacterium acnes* bacteriophage (in separate compositions)
(vi) the combination of 10% BPO and *Propionibacterium acnes* bacteriophage (in a single compositions)

The combination of the *Propionibacterium acne* bacteriophage with BPO achieves more than an additive effect, *i.e.,* a synergistic effect (the combined effect of the bacteriophage and the BPO is greater than the sum of the effects of the bacteriophage and the BPO when each agent is used separately) in treating acne. The effectiveness of treatment is measured using lesion counts and an IGA (investigator global assessment) score.

### EXAMPLE 7 (PROPHETIC). ASSAY WITH A COMBNIATION OF BACTERIOPHAGE WITH BENZOYL PEROXIDE

An *in vitro* study is performed to determine the efficacy of (i) BPO; (ii) *Propionibacterium acne* bacteriophage; or (iii) *Propionibacterium acne* bacteriophage + BPO in killing planktonic and sessile pathogenic *P. acnes* bacteria.

The combination of the *Propionibacterium acne* bacteriophage with BPO achieves more than an additive effect, *i.e.,* a synergistic effect (the combined effect of the bacteriophage and the BPO is greater than the sum of the effects of the bacteriophage and the BPO when each agent is used separately) in killing sessile pathogenic *P. acnes* bacteria. The keratolytic action of BPO (similar to salicylic acid and retinoids) assists the phage in penetrating skin pores to access the P. acnes deep within the pores.

## Claims

1. A composition comprising at least one *Propionibacterium acnes (P. acnes)* bacteriophage, at least one anti-acne compound comprising salicylic acid, and a pharmaceutically acceptable carrier, wherein the genome of the *P. acnes* bacteriophage comprises a nucleotide sequence that is at least about 98%, or 99% identical to the nucleotide sequence of SEQ ID NO: 1.

2. The composition of claim 1, wherein
(a) the composition does not comprise a probiotic bacterium;
(b) the composition further comprises an anti-aging enzyme, or one or more probiotic bacteria;
(c) the pharmaceutically acceptable carrier comprises an emulsion; or
(d) the composition is in the form of a cream, lotion, suspension, or aqueous solution.

3. The composition of claim 1, wherein
(a) the salicylic acid is present at a concentration of (i) 0.5% to 2% (weight/volume), or (ii) less than 0.5% but greater than about 0.1% (weight/volume);
(b) the composition further comprises an anti-aging enzyme, and the anti- aging enzyme is a superoxide dismutase or a peroxidase; or
(c) the pharmaceutically acceptable carrier comprises an emulsion, and the emulsion is an oil-in-water emulsion or a water-in-oil emulsion.

4. The composition of claim 1, wherein the composition further comprises a *P. acnes* biofilm degrading enzyme.

5. The composition of claim 4, wherein
(a) the *P. acnes* biofilm degrading enzyme is a glycosidase, a protease, a DNAse, a glycoside hydrolase, a β-hexosaminidase, or a restriction endonuclease;
(b) the *P. acnes* biofilm degrading enzyme catalyzes the hydrolysis of linear polymers of N-acetyl-D-glucosamines;
(c) the *P. acnes* biofilm degrading enzyme hydrolyzes β-1,6-glycosidic linkages of acetylglucosamine polymers;
(d) the *P. acnes* biofilm degrading enzyme is a DNAse I, papain, bromelain, Trypsin, Proteinase K,
Subtilisin, serratiopeptidase, dispersin, alginate lyase, amylase, or cellulose; or
(e) the *P. acnes* biofilm degrading enzyme is Dispersin B.

6. The composition of claim 1, wherein the composition further comprises a probiotic bacterium, wherein the probiotic bacterium is (a) *a P. sp., Staphylococcus* sp., and/or *Corynebacterium* sp. bacterium, or (b) a bacterium within the class *Betaproteobacteria.*

7. The composition of claim 6, wherein the probiotic bacterium is a probiotic *P. acnes* bacterium.

8. The composition of claim 7, wherein the *P. acnes* bacterium
(a) comprises a 16S rDNA sequence with a mutation selected from T992C, T838C, C1322T, or C986T, compared to the KPA171202 type strain 16S rDNA sequence set forth as SEQ ID NO: 2;
(b) comprises a 16S rDNA sequence that is identical to the sequence of SEQ ID NO: 3 or SEQ ID NO: 4; and/or
(c) does not comprise a linear plasmid, a plasmid that comprises a virulence factor, or a plasmid that encodes an extrachromosomal lipase;

9. The composition of claim 7, wherein the *P. acnes* bacterium:
(a) produces less than about 20% of the level of lipase that is produced by a pathogenic *P. acnes* strain when grown in a planktonic culture;
(b) produces less than about 10% of the level of lipase that is produced by a pathogenic *P. acnes* strain when grown in an adherent culture;
(c) adheres to epithelial cells at least 50% less than a pathogenic *P. acnes* strain; and/or
(d) is less inflammatory than a pathogenic *P. acnes* strain.

10. The composition of claim 7, further comprising at least one additional probiotic bacterium, wherein said at least one additional probiotic bacterium comprises *Propionibacterium granulosum* and/or *Propionibacterium avidum.*

11. The composition of claim 9, wherein said pathogenic *P. acnes* strain
(a) comprises a 16S rDNA sequence with one or more mutations compared to the KPA171202 type strain 16S rDNA sequence set forth as SEQ ID NO: 2, wherein the one or more mutations are selected from (i) G1058C, (ii) G1058C and A1201C, (iii) G529A, (iv) G1004A and a T1007C, (v) G1268A, or (vi) T554C and G1058C; or
(b) comprises a 16S rDNA sequence that is identical to the sequence of any one of SEQ ID NOs: 5-10.

12. A combination comprising:
(a) at least one *Propionibacterium acnes* bacteriophage;
(b) at least one anti-acne compound comprising salicylic acid;
(c) a pharmaceutically acceptable carrier,
wherein (i) each of the at least one *Propionibacterium acnes* bacteriophage, the at least one anti-acne compound, and the pharmaceutically acceptable carrier are in a single composition in a container, and (ii) the genome of the *P. acnes* bacteriophage comprises a nucleotide sequence that is at least about 98%, or 99% identical to the nucleotide sequence of SEQ ID NO: 1 and;
(d) a separate container comprising an additional anti-acne compound comprising benzoyl peroxide.

13. The combination of claim 12, wherein:
(a) the benzoyl peroxide is present at a concentration of 2.5% to 10% (weight/volume); and/or
(b) the benzoyl peroxide is present at a concentration of less than 2.5% but greater than about 0.1%, 0.5%, 1%, 1.5%, or 2% (weight/volume).

14. The composition of claim 1 or the combination of claim 12 for use in a method of treating acne in a subject in need thereof.

15. The composition for use of claim 14, wherein the composition is formulated for topical administration.

## Patentansprüche

1. Zusammensetzung, umfassend zumindest einen *Propionibacterium-acnes*(*P.-acnes-*) Bakteriophagen, zumindest eine Anti-Akne-Verbindung, die Salicylsäure umfasst, und einen pharmazeutisch annehmbaren Träger, wobei das Genom des *P.-acnes*-Bakteriophagen eine Nukleotidsequenz umfasst, die zumindest etwa 98 % oder 99 % identisch mit der Nukleotidsequenz von SEQ ID NO: 1 ist.

2. Zusammensetzung nach Anspruch 1, wobei
(a) die Zusammensetzung kein probiotisches Bakterium umfasst;
(b) die Zusammensetzung ferner ein Anti-Aging-Enzym oder ein oder mehrere probiotische Bakterien umfasst;
(c) der pharmazeutisch annehmbare Träger eine Emulsion umfasst; oder
(d) die Zusammensetzung in der Form einer Creme, Lotion, Suspension oder wässrigen Lösung ist.

3. Zusammensetzung nach Anspruch 1, wobei
(a) die Salicylsäure in einer Konzentration von (i) 0,5 % bis 2 % (Gewicht/Volumen) oder (ii) weniger als 0,5 %, aber größer als etwa 0,1 % (Gewicht/Volumen) vorliegt;
(b) die Zusammensetzung ferner ein Anti-Aging-Enzym umfasst und das Anti-Aging-Enzym eine Superoxid-Dismutase oder eine Peroxidase ist; oder
(c) der pharmazeutisch annehmbare Träger eine Emulsion umfasst und die Emulsion eine Öl-in-Wasser-Emulsion oder eine Wasser-in-Öl-Emulsion ist.

4. Zusammensetzung nach Anspruch 1, wobei die Zusammensetzung ferner ein *P.-acnes-*Biofilm abbauendes Enzym umfasst.

5. Zusammensetzung nach Anspruch 4, wobei
(a) das *P.-acnes-*Biofilm abbauende Enzym eine Glycosidase, eine Protease, eine DNAse, eine Glycosidhydrolase, eine β-Hexosaminidase oder eine Restriktionsendonuklease ist;
(b) das *P.-acnes-*Biofilm abbauende Enzym die Hydrolyse von linearen Polymeren von N-Acetyl-D-glucosaminen katalysiert;
(c) das *P*.-*acnes-*Biofilm abbauende Enzym β-1,6-glykosidische Bindungen von Acetylglucosamin-Polymeren hydrolysiert;
(d) das *P.-acnes-*Biofilm abbauende Enzym eine DNAse I, Papain, Bromelain, Trypsin, Proteinase K, Subtilisin, Serratiopeptidase, Dispersin, Alginatlyase, Amylase oder Cellulose ist; oder
(e) das *P.-acnes-*Biofilm abbauende Enzym Dispersin B ist.

6. Zusammensetzung nach Anspruch 1, wobei die Zusammensetzung ferner ein probiotisches Bakterium umfasst, wobei das probiotische Bakterium (a) ein Bakterium von *P* sp., *Staphylococcus* sp. und/oder *Corynebacterium* sp., oder (b) ein Bakterium innerhalb der Klasse *Betaproteobacteria* ist.

7. Zusammensetzung nach Anspruch 6, wobei das probiotische Bakterium ein probiotisches *P.-acnes*-Bakterium ist.

8. Zusammensetzung nach Anspruch 7, wobei das *P.-acnes*-Bakterium
(a) eine 16S-rDNA-Sequenz mit einer Mutation ausgewählt aus T992C, T838C, C1322T oder C986T verglichen mit der Stamm-16S-rDNA-Sequenz vom KPA171202-Typ dargelegt als SEQ ID NO: 2 umfasst;
(b) eine 16S rDNA-Sequenz umfasst, die identisch ist mit der Sequenz von SEQ ID NO: 3 oder SEQ ID NO: 4; und/oder
(c) kein lineares Plasmid, ein Plasmid, das einen Virulenzfaktor umfasst, oder ein Plasmid, das für eine extrachromosomale Lipase kodiert, umfasst.

9. Zusammensetzung nach Anspruch 7, wobei das *P.-acnes*-Bakterium:
(a) weniger als etwa 20 % des Niveaus an Lipase produziert, der durch einen pathogenen *P.-acnes-*Stamm produziert wird, wenn in einer planktonischen Kultur gezüchtet;
(b) weniger als etwa 10 % des Niveaus an Lipase produziert, die durch einen pathogenen *P.-acnes-*Stamm produziert wird, wenn in einer adhärenten Kultur gezüchtet;
(c) an Epithelzellen zu zumindest 50 % weniger als ein pathogener *P.-acnes*-Stamm haftet; und/oder
(d) weniger entzündlich ist als ein pathogener *P.-acnes*-Stamm.

10. Zusammensetzung nach Anspruch 7, ferner umfassend zumindest ein zusätzliches probiotisches Bakterium, wobei das zumindest eine zusätzliche probiotische Bakterium *Propionibacterium granulosum* und/oder *Propionibacterium avidum* umfasst.

11. Zusammensetzung nach Anspruch 9, wobei der pathogene *P.-acnes*-Stamm
(a) eine 16S rDNA-Sequenz mit einer oder mehreren Mutationen verglichen mit der Stamm-16S-rDNA-Sequenz vom KPA171202-Typ dargelegt als SEQ ID NO: 2 umfasst, wobei die eine oder mehreren Mutationen ausgewählt sind aus (i) G1058C, (ii) G1058C und A1201C, (iii) G529A, (iv) G1004A und T1007C, (v) G1268A, oder (vi) T554C und G1058C; oder
(b) eine 16S-rDNA-Sequenz umfasst, die identisch ist mit der Sequenz einer beliebigen von SEQ ID NOs: 5-10.

12. Kombination umfassend:
(a) zumindest einen *Propionibacterium-acnes*-Bakteriophagen;
(b) zumindest eine Anti-Akne-Verbindung, die Salicylsäure umfasst;
(c) einen pharmazeutisch annehmbaren Träger,
wobei (i) jedes von dem zumindest einen *Propionibacterium-acnes-*Bakteriophagen, der zumindest einen Anti-Akne-Verbindung und dem pharmazeutisch annehmbaren Träger in einer einzigen Zusammensetzung in einem Behälter sind, und (ii) das Genom des *P.-acnes-*Bakteriophagen eine Nukleotidsequenz umfasst, die zumindest etwa 98 % oder 99 % identisch ist mit der Nukleotidsequenz von SEQ ID NO: 1; und
(d) einen separaten Behälter, der eine zusätzliche Anti-Akne-Verbindung umfasst, die Benzoylperoxid umfasst.

13. Kombination nach Anspruch 12, wobei:
(a) das Benzoylperoxid in einer Konzentration von 2,5 % bis 10 % (Gewicht/Volumen) vorliegt; und/oder
(b) das Benzoylperoxid in einer Konzentration von weniger als 2,5 %, aber größer als etwa 0,1 %, 0,5 %, 1 %, 1,5 % oder 2 % (Gewicht/Volumen) vorliegt.

14. Zusammensetzung nach Anspruch 1 oder Kombination nach Anspruch 12 zur Verwendung in einem Verfahren zur Behandlung von Akne bei einem Subjekt, das dessen bedarf.

15. Zusammensetzung zur Verwendung nach Anspruch 14, wobei die Zusammensetzung zur topischen Verabreichung formuliert ist.

## Revendications

1. Composition comprenant au moins un bactériophage de *Propionibacterium acnes (P. acnes)*, au moins un composé anti-acnéique comprenant de l'acide salicylique et un support pharmaceutiquement acceptable, dans laquelle le génome du bactériophage de *P. acnes* comprend une séquence nucléotidique identique à au moins environ 98 % ou 99 % à la séquence nucléotidique de SEQ ID N° : 1.

2. Composition de la revendication 1, dans laquelle
(a) la composition ne comprend pas de bactérie probiotique ;
(b) la composition comprend en outre une enzyme anti-âge, ou une ou plusieurs bactéries probiotiques ;
(c) le support pharmaceutiquement acceptable comprend une émulsion ; ou
(d) la composition se présente sous la forme d'une crème, d'une lotion, d'une suspension ou d'une solution aqueuse.

3. Composition de la revendication 1, dans laquelle
(a) l'acide salicylique est présent à une concentration de (i) 0,5 % à 2 % (poids/volume), ou (ii) inférieure à 0,5 % mais supérieure à environ 0,1 % (poids/volume) ;
(b) la composition comprend en outre une enzyme anti-âge, et l'enzyme anti-âge est une superoxyde dismutase ou une peroxydase ; ou
(c) le support pharmaceutiquement acceptable comprend une émulsion, et l'émulsion est une émulsion huile-dans-eau ou une émulsion eau-dans-huile.

4. Composition de la revendication 1, dans laquelle la composition comprend en outre une enzyme dégradant le biofilm de *P. acnes*.

5. Composition de la revendication 4, dans laquelle
(a) l'enzyme dégradant le biofilm de *P. acnes* est une glycosidase, une protéase, une DNAse, une glycoside hydrolase, une β-hexosaminidase ou une endonucléase de restriction ;
(b) l'enzyme dégradant le biofilm de *P. acnes* catalyse l'hydrolyse de polymères linéaires de N-acétyl-D-glucosamines ;
(c) l'enzyme dégradant le biofilm de *P. acnes* hydrolyse les liaisons β-1,6-glycosidiques de polymères d'acétylglucosamine ;
(d) l'enzyme dégradant le biofilm de *P. acnes* est une DNAse I, la papaïne, la bromélaïne, la trypsine, la protéinase K, la subtilisine, la serratiopeptidase, la dispersine, une alginate lyase, une amylase ou une cellulose ; ou
(e) l'enzyme dégradant le biofilm de *P. acnes* est la Dispersine B.

6. Composition de la revendication 1, dans laquelle la composition comprend en outre une bactérie probiotique, dans laquelle la bactérie probiotique est (a) une bactérie de *P.* sp., de *Staphylococcus* sp. et/ou de *Corynebacterium* sp., ou (b) une bactérie appartenant à la classe des *Bêtaprotéobactéries.*

7. Composition de la revendication 6, dans laquelle la bactérie probiotique est une bactérie probiotique de *P. acnes*.

8. Composition de la revendication 7, dans laquelle la bactérie de *P. acnes*
(a) comprend une séquence d'ADNr 16S présentant une mutation choisie parmi T992C, T838C, C1322T ou C986T, par rapport à la séquence d'ADNr 16S de la souche type KPA171202 définie comme SEQ ID N° : 2 ;
(b) comprend une séquence d'ADNr 16S identique à la séquence de SEQ ID N° : 3 ou SEQ ID N° : 4 ; et/ou
(c) ne comprend pas de plasmide linéaire, de plasmide comprenant un facteur de virulence ou de plasmide codant pour une lipase extrachromosomique.

9. Composition de la revendication 7, dans laquelle la bactérie de *P. acnes* :
(a) produit moins d'environ 20 % du niveau de lipase produit par une souche pathogène de *P. acnes* lorsqu'elle est cultivée en culture planctonique ;
(b) produit moins d'environ 10 % du niveau de lipase produit par une souche pathogène de *P. acnes* lorsqu'elle est cultivée en culture adhérente ;
(c) adhère aux cellules épithéliales à un niveau inférieur d'au moins 50 % à celui d'une souche pathogène de *P. acnes* ; et/ou
(d) est moins inflammatoire qu'une souche pathogène de *P. acnes*.

10. Composition de la revendication 7, comprenant en outre au moins une bactérie probiotique supplémentaire, dans laquelle ladite au moins une bactérie probiotique supplémentaire comprend *Propionibacterium granulosum et*/*ou Propionibacterium avidum.*

11. Composition de la revendication 9, dans laquelle ladite souche pathogène de *P. acnes*
(a) comprend une séquence d'ADNr 16S présentant une ou plusieurs mutations par rapport à la séquence d'ADNr 16S de la souche type KPA171202 définie comme SEQ ID N° : 2,
dans laquelle la ou les mutations sont choisies parmi (i) G1058C, (ii) G1058C et A1201C, (iii) G529A, (iv) G1004A et T1007C, (v) G1268A, ou (vi) T554C et G1058C ; ou
(b) comprend une séquence d'ADNr 16S qui est identique à la séquence de l'une quelconque des SEQ ID N° : 5-10.

12. Combinaison comprenant :
(a) au moins un bactériophage de *Propionibacterium acnes* ;
(b) au moins un composé anti-acnéique comprenant de l'acide salicylique ;
(c) un support pharmaceutiquement acceptable,
dans laquelle (i) chacun de l'au moins un bactériophage de *Propionibacterium acnes*, de l'au moins un composé anti-acnéique et du support pharmaceutiquement acceptable est présent dans une composition unique dans un récipient, et (ii) le génome du bactériophage de *P. acnes* comprend une séquence nucléotidique qui est d'au moins environ 98 % ou 99 % identique à la séquence nucléotidique de SEQ ID N° : 1 et ;
(d) un récipient séparé comprenant un composé anti-acnéique supplémentaire comprenant du peroxyde de benzoyle.

13. Combinaison de la revendication 12, dans laquelle :
(a) le peroxyde de benzoyle est présent à une concentration de 2,5 % à 10 % (poids/volume) ; et/ou
(b) le peroxyde de benzoyle est présent à une concentration inférieure à 2,5 % mais supérieure à environ 0,1 %, 0,5 %, 1 %, 1,5 % ou 2 % (poids/volume).

14. Composition de la revendication 1 ou combinaison de la revendication 12, destinée à être utilisée dans un procédé de traitement de l'acné chez un sujet en ayant besoin.

15. Composition destinée à être utilisée selon la revendication 14, dans laquelle la composition est formulée pour une administration topique.
